# EUROPÄISCHE PATENTANMELDUNG

(11) **EP 0 711 804 A2**
(43) Veröffentlichungstag der Anmeldung: **15.05.1996**
(21) Anmeldenummer: 95810693.2
(22) Anmeldetag: 06.11.1995
(51) Int. Cl.: C08K 5/3492, C08K 5/3462, G03C 1/34

(54) **Kryptolichtschutzmittel**

(30) Priorität: 14.11.1994 CH 3410/94
(71) Anmelder: CIBA-GEIGY AG, CH-4002 Basel (CH)
(72) Erfinder: Birbaum, Jean-Luc, Dr., Kobe 658 (JP); Rytz, Gerhard, Dr., CH-3007 Bern (CH); Toan, Vien Van, Dr., CH-1745 Lentigny (CH); Valet, Andreas, Dr., D-79589 Binzen (DE); Würms, Norbert, CH-1717 St. Ursen (CH)

(57) **Zusammenfassung**

Es werden Verbindungen der Formel I beschrieben 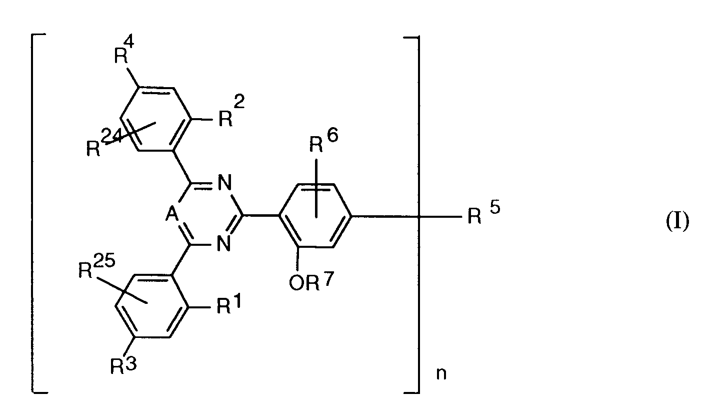
worin n 1 oder 2 ist, A für CH oder ein Stickstoffatom steht und die übrigen Reste R¹ bis R⁵ die in Anspruch 1 angegebenen Bedeutungen haben.

Die Verbindungen der Formel I lassen sich vorteilhaft zum Stabilisieren von organischem Material gegen den schädigenden Einfluß von Licht, Sauerstoff und/oder Wärme einsetzen.

## Beschreibung

Die Erfindung betrifft gegen Schädigung durch Licht, Wärme und Sauerstoff stabilisierte Zusammensetzungen, welche als Stabilisator ein Kryptolichtschutzmittel vom Typ 2,4,6-Tris-aryl-1,3,5-triazin oder 2,4,6-Tris-aryl-1,3-pyrimidin enthalten, die Verwendung dieser Kryptolichtschutzmittel zur Stabilisierung organischen Materials, sowie neue Verbindungen des genannten Typs.

Kryptolichtschutzmittel setzen unter Einwirkung von Hitze oder UV-Strahlung durch Rückspaltung die wirksamen Stabilisatoren frei. Die Anwendung von Verbindungen dieser Art, die durch Abspaltung des Substituenten am phenolischen Sauerstoffatom in Lichtschutzmittel vom Typ des 2(2-Hydroxyphenyl)benztriazols oder des 2-Hydroxybenzophenones umgewandelt werden können, ist beispielsweise in den Publikationen US-A-4 775 707, US-A-5 030 731, J. Appl. Pol. Science 22, 2165-2172 (1978) und J. Appl. Pol. Science 28, 1159-1165 (1983) beschrieben. Weitere Benztriazole mit blockierter phenolischer Hydroxylgruppe sind z.B. in US-A-3 936 418, US-A-4 247 628, Chem. Abstr. 103: 124474x und US-A-4 344 830 als Stabilisatoren genannt.

Eine neuere und strukturell völlig andersartige Klasse von Lichtschutzmitteln sind freie OH-Gruppen enthaltende Verbindungen vom Typ 2,4,6-Tris-aryl-1,3,5-triazin und 2,4,6-Tris-aryl-1,3-pyrimidin; aus der Vielzahl der einschlägigen Publikationen seien hier die folgenden genannt: US-A-3 118 887, US-A-3 242 175, US-A-3 244 708, US-A-3 249 608, CH-A-480090, CH-A-480091, CH-A-484695, US-A-3 423 360, US-A-3 843 371, WO 86/3528, US-A-4 619 956, US-A-4 831 068, US-A-4 826 978, EP-A-434608, EP-A-444323, US-A-5 369 140, EP-A-483 488, US-A-5 288 778, US-A-5 298 067, US-A-3 442 898, US-A-4 895 981.

In EP-A-604 980 wird die Verwendung von phenolischen UV-Absorbern, darunter auch solchen mit blockierter Hydroxylgruppe, zur Herstellung von verankerten Lichtschutzmitteln vorgeschlagen.

US-A-3 249 608 beschreibt einige Tris-Aryltriazine, deren Hydroxylgruppen acyliert sind, und die als Nebenprodukte bei der Herstellung von Lichtschutzmitteln anfallen.

In US-A-3 113 941 und US-A-3 113 942 werden einige Ester bzw. Ether von Tris-Hydroxyphenyl-triazinen als Kunststoffzusätze vorgeschlagen.

Es wurde nun gefunden, daß überraschenderweise auf Basis von Tris-Aryltriazin- und -pyrimidinverbindungen Kryptolichtschutzmittel erhalten werden können, die über gute Substratkompatibilität und Farbeigenschaften verfügen sowie eine hohe Rückspaltungsrate und Lagerstabilität aufweisen.

Gegenstand der Erfindung ist daher eine Zusammensetzung enthaltend
A) ein gegen Schädigung durch Licht, Sauerstoff und/oder Hitze empfindliches organisches Material und
B) als Stabilisator eine Verbindung der Formel I 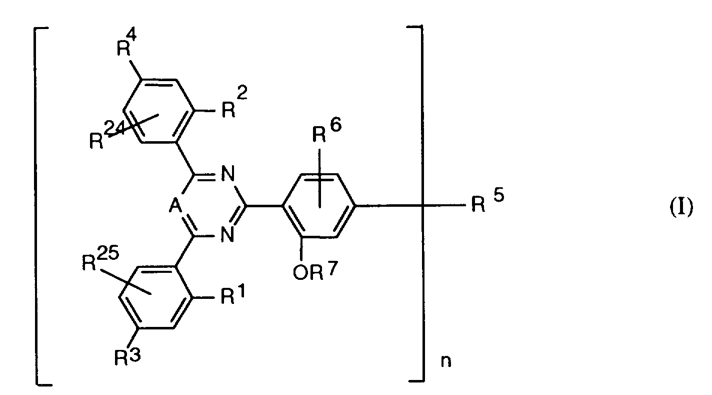 worin n 1 oder 2 ist;

A für CH oder ein Stickstoffatom steht;
R¹ und R, unabhängig voneinander, H; C₁-C₁₂-Alkyl; C₅-C₁₂-Cycloalkyl, Trifluormethyl oder OR⁷ bedeuten;
R³ und R⁴, unabhängig voneinander, H; C₁-C₁₂-Alkyl; C₅-C₁₂-Cycloalkyl, OR¹⁷, Phenyl, CN, Halogen oder OR⁷ bedeuten;
R⁵ im Fall n = 1 als monovalenter Rest C₆-C₁₈-Alkyl; C₁-C₁₈-Alkoxy; Halogen; -O-CO-R¹; -O-SO₂-R¹³; 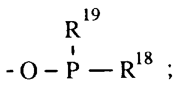 oder 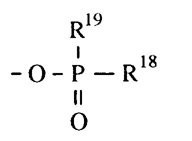 bedeutet; oder R⁵ im Fall n = 1 eine der für R¹⁷ angegebenen Bedeutungen hat oder -O-R¹⁷ bedeutet; und R⁵ als monovalenter Rest im Fall, daß keiner der Reste R¹ und R OR⁷ ist, zusätzlich H und C₁-C₅-Alkyl umfaßt;
R⁵ im Fall n = 2 als divalenter Rest -O-G-O- bedeutet, wobei
G C₂-C₁₆-Alkylen; C₄-C₁₂-Alkenylene; Xylylen; oder C₃-C₂₀-Alkylen, das durch -O- unterbrochen und/oder durch OR⁷ substituiert ist, bedeutet; oder G für eine der Gruppen -CH₂CH(OR⁷)CH₂O-R⁰-OCH₂CH(OR⁷)CH₂-;-CO-R¹-CO-;-CO-NH-R-NH-CO-;
-(CH₂)ₘ-COO-R³-OOC-(CH₂)ₘ-, worin m eine ganze Zahl aus dem Bereich 1 bis 3 ist;
   oder 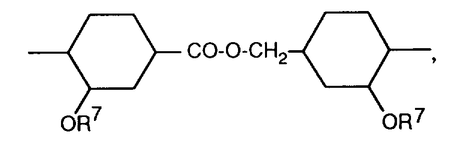 steht;
R⁶ H; C₂-C₁₈-Alenyl; -X-Z³; unsubstituiertes oder am Phenylkern durch Methyl, Halogen, -CN oder Methoxy substituiertes Benzoyl; Halogen; -SR¹⁴; -SOR¹³; -SO₂R¹³; -C(Z³)=N-Z³; -CH(Z³)-NH-Z³; ein Rest der Formel 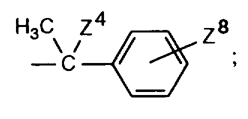 oder ein Rest der Formel 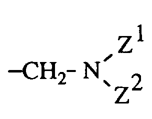 ist;
R⁷ eine der Bedeutungen -CO-R¹¹, -SO₂-R¹³, 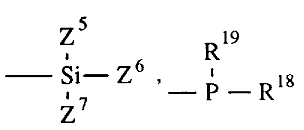 oder 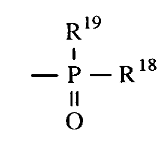 hat oder Allyl oder eine Gruppe der Formel 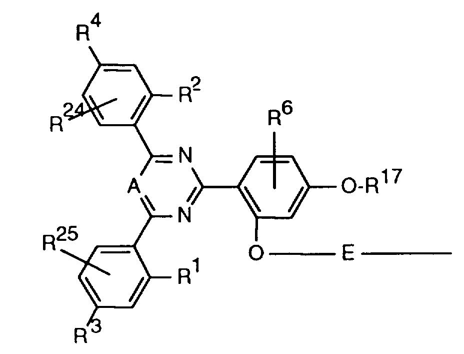 darstellt, worin E C₃-C₁₈-Alkylen oder C₄-C₁₈-Alkenylen ist;
R⁸ C₁-C₁₈-Alkyl; C₃-C₁₈-Alkenyl; durch O, N oder S unterbrochenes und/oder durch OR⁷ substituiertes C₃-C₂₀-Alkyl; durch -P(O)(OR¹⁴)₂, -N(R⁹)(R¹⁰) oder -OCOR¹¹ und/oder OR⁷ substituiertes C₁-C₄-Alkyl; Glycidyl; C₅-C₁₂-Cycloalkyl oder C₇-C₁₁-Phenylalkyl bedeutet; oder eine Gruppe der Formel 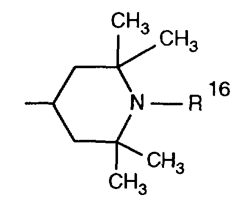 darstellt;
R⁹ und R¹⁰, unabhängig voneinander, C₁-C₁₂-Alkyl; C₃-C₁₂-Alkoxyalkyl;
C₄-C₁₆-Dialkylaminoalkyl oder C₅-C₁₂-Cycloalkyl bedeuten oder R⁹ und R¹⁰ zusammen C₃-C₉-Alkylen oder -Oxaalkylen oder -Azaalkylen bedeuten;
R¹¹ C₁-C₁₈-Alkyl; durch Halogen substituiertes C₁-C₁₈-Alkyl; C₅-C₁₂-Cycloalkyl;
C₂-C₁₈-Alkenyl; -CH₂-CO-CH₃; C₇-C₁₂-Aralkyl; C₁-C₁₂-Alkoxy; oder Phenyl bedeutet, welches unsubstituiert oder durch C₁-C₁₂-Alkyl, C₁-C₄-Alkoxy, Halogen und/oder Benzyl substituiert ist;
R¹ C₁-C₁₈-Alkyl; durch Halogen substituiertes C₁-C₁₈-Alkyl; C₅-C₁₂-Cycloalkyl;
C₂-C₁₈-Alkenyl; Phenyl; oder -R¹⁵-O-CO-R¹¹ bedeutet; oder eine Gruppe der Formel 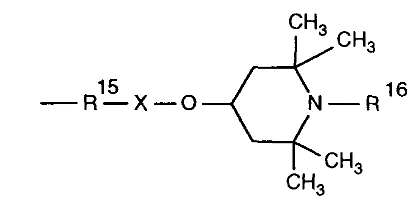 darstellt;
R¹³ C₁-C₁₂-Alkyl; C₆-C₁₀-Aryl; oder C₇-C₁₈-Alkylaryl bedeutet; und
R¹⁴ C₁-C₁₂-Alkyl oder Phenyl oder C₇-C₁₅-Phenylalkyl;
R¹⁵ C₁-C₁₈-Alkylen oder C₂-C₁₈-Alkenylen;
R¹⁶ Wasserstoff; Oxyl; Formyl; C₂-C₈-Alkanoyl; C₁-C₁₈-Alkyl; C₁-C₁₈-Alkoxy;
C₅-C₁₂-Cycloalkyl; C₅-C₁₂-Cycloalkoxy; C₇-C₁₁-Phenylalkyl; C₇-C₁₁-Phenylalkyl, welches am Phenylring durch 1 bis 3 Reste C₁-C₄-Alkyl oder C₁-C₈-Alkanoyl substituiert ist; oder C₇-C₁₁-Phenylalkoxy;
R¹⁷ C₅-C₁₈-Alkyloxycarbonyl; oder C₂-C₁₈-Alkenyl bedeutet; oder R¹⁷ C₁-C₁₈-Alkyl bedeutet, das substituiert ist durch OR⁷, C₁-C₁₈-Alkoxy, C₅-C₁₂-Cycloalkoxy,
C₂-C₁₈-Alkanoyl, C₂-C₈-Alkenyloxy, Halogen, -COOR⁸, -CONH₂, -CONHR⁹, -CON(R⁹)(R¹⁰), -NHR⁹, -N(R⁹)(R¹⁰), -NHCOR¹¹, -CN, -OCOR¹¹, eine Gruppe der Formel 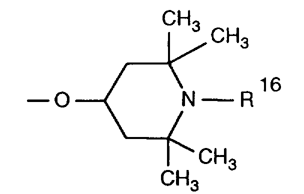 und/oder Phenoxy, welches unsubstituiert ist oder durch C₁-C₁₈-Alkyl, C₁-C₁₈-Alkoxy oder Halogen substituiert ist; oder R¹⁷ C₃-C₅₀-Alkyl bedeutet, welches durch O unterbrochen ist und durch OR⁷ substituiert sein kann; oder R¹⁷ Glycidyl;
C₅-C₁₂-Cycloalkyl; durch OR⁷, C₁-C₄-Alkyl oder -OCOR¹¹ substituiertes C₅-C₁₂-Cycloalkyl; oder unsubstituiertes oder durch OR⁷, Cl oder CH₃ substituiertes C₇-C₁₁-Phenylalkyl bedeutet;
R¹⁸ und R¹⁹, unabhängig voneinander, C₁-C₁₂-Alkoxy, Phenoxy, C₁-C₁₂-Alkyl, C₅-C₁₂-Cycloalkyl, Benzyl, Tolyl oder Phenyl darstellen;
R⁰ C₂-C₁₀-Alkylen; durch -O- unterbrochenes C₄-C₅₀-Alkylen; Phenylen; oder eine Gruppe -Phenylen-D-Phenylen- ist, worin D für -O-, -S-, -SO₂-, -CH₂- oder -C(CH₃)₂- steht;
R¹ C₂-C₁₀-Alkylen; durch O oder S unterbrochenes C₂-C₁₀-Alkylen; C₆-C₁₂-Arylen; oder C₂-C₆-Alkenylen;
R C₂-C₁₀-Alkylen; Phenylen; Tolylen; Diphenylenmethan; oder 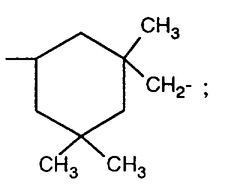 und
R³ C₂-C₁₀-Alkylen oder durch -O- unterbrochenes C₄-C₂₀-Alkylen ist;
R⁴ und R⁵, unabhängig voneinander, H, C₁-C₁₂-Alkyl; C₂-C₆-Alkenyl;
C₁-C₁₂-Alkoxy; C₃-C₁₈-Alkenyloxy; Halogen; Trifluormethyl; C₇-C₁₁-Phenylalkyl; Phenyl; durch C₁-C₁₈-Alkyl, C₁-C₁₈-Alkoxy oder Halogen substituiertes Phenyl; Phenyloxy; oder durch C₁-C₁₈-Alkyl, C₁-C₁₈-Alkoxy oder Halogen substituiertes Phenyloxy darstellen;
X eine direkte Bindung oder -CO- bedeutet;
Z¹ und Z, unabhängig voneinander, C₁-C₁₂-Alkyl sind oder gemeinsam C₄-C₁₀-Alkylen darstellen, das durch ein Sauerstoffatom unterbrochen sein kann;
Z³ C₁-C₂₀-Alkyl oder C₇-C₁₅-Phenylalkyl und
Z⁴ und Z⁸, unabhängig voneinander, Wasserstoff oder Methyl sind; und
Z⁵, Z⁶ und Z⁷, unabhängig voneinander, C₁-C₁₈-Alkyl, Cyclohexyl, Phenyl oder C₁-C₁₈-Alkoxy bedeuten.

Ein Substituent Halogen bedeutet -F, -Cl, -Br oder -I; bevorzugt ist -Cl oder -F, vor allem -Cl.

Aryl steht allgemein für einen aromatischen Kohlenwasserstoffrest, beispielsweise für Phenyl, Biphenyl oder Naphthyl. Aralkyl bezeichnet allgemein durch Aryl substituiertes Alkyl; so beinhaltet C₇-C₁₂-Aralkyl z.B. Benzyl, Phenylethyl, Phenylpropyl, Phenylbutyl, Phenylpentyl und Phenylhexyl; bevorzugt sind Benzyl und α-Methylbenzyl. Alkylaryl steht für durch Alkyl substituiertes Aryl; C₇-C₁₈-Alkylaryl beinhaltet unter anderem Methylphenyl (Tolyl), Dimethylphenyl (Xylyl), Trimethylphenyl, Tetramethylphenyl, Pentamethylphenyl, Ethylphenyl, Propylphenyl (z.B.Cumyl), Butylphenyl (z.B. tert.Butylphenyl), Methylbutylphenyl, Dibutylphenyl, Pentylphenyl, Hexylphenyl, Dihexylphenyl, Heptylphenyl, Octylphenyl, Nonylphenyl, Decylphenyl, Undecylphenyl, Dodecylphenyl, Methylnaphthyl, Dimethylnaphthyl, Ethylnaphthyl, Propylnaphthyl, Butylnaphthyl, Pentylnaphthyl, Hexylnaphthyl, Heptylnaphthyl, Octylnaphthyl; von besonderer Bedeutung sind davon beispielsweise Tolyl, Xylyl, Propylphenyl und Butylphenyl.

Cycloalkyl bezeichnet allgemein einen gesättigten carbocyclischen Rest der Summenformel CᵢH₂ᵢ, wobei i eine ganze Zahl ist. C₅-C₁₂-Cycloalkyl beinhaltet beispielsweise Cyclopentyl, Cyclohexyl, Cycloheptyl, Cyclooctyl, Cyclododecyl; bevorzugt ist Cyclohexyl.

In Formel I stellt der in den Phenylkern hineinragende Strich mit dem Symbol R⁶ einen Substituenten dar, der sich an einer der drei noch freien Positionen in o-, m- oder p-Position zur phenolischen OH-Gruppe befindet.

Der Substituent R⁶ befindet sich vorzugsweise in o- oder p-Position zur phenolischen OH-Gruppe, besonders in p-Position.
R⁶ stellt beispielsweise Wasserstoff; C₁-C₁₂-Alkyl; C₆-C₁₈-Alkanoyl; Benzoyl; Methylbenzoyl; Dimethylbenzoyl; durch -Cl, -Br, -CN oder -OCH₃ substituiertes Benzoyl; α-Methylbenzyl; α,α-Dimethylbenzyl; N,N-Dialkylaminomethyl;
1-Piperidylmethyl; 1-(4-Oxapiperidyl)methyl; ein Imid eines Acylrestes; oder α-(N-Alkylamino)alkyl dar. Vorzugsweise ist R⁶ Wasserstoff, C₁-C₆-Alkyl, Benzoyl, α-Methylbenzyl, Allyl oder ein Rest der Formeln 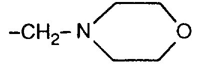 oder 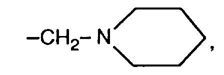 beispielsweise Wasserstoff oder C₁-C₆-Alkyl oder Allyl, besonders Wasserstoff oder Methyl, vor allem Wasserstoff.

Die Reste R¹, R, R³, R⁴, R⁵, R⁶, R⁸, R⁹, R¹⁰, R¹¹, R¹, R¹³, R¹⁴, R¹⁶, R¹⁷, R¹⁸, R¹⁹, Z¹, Z, Z³, Z⁵, Z⁶ und Z⁷ als Alkyl stellen, im Rahmen der angegebenen Definitionen, verzweigtes oder unverzweigtes Alkyl dar wie Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl. Bevorzugt sind R¹, R, R³, R⁴, R⁶, R⁸, R¹¹, R¹, R¹⁴ und R¹⁶ als Alkyl C₁-C₈-Alkyl, vor allem C₁-C₄-Alkyl wie Methyl, n- oder tert.Butyl, besonders Methyl. R⁹, R¹⁰ sind vorzugsweise C₁-C₈-Alkyl, vor allem Butyl.

R⁶ als Alkanoyl ist beispielsweise Acetyl, Propionyl, Butyryl, Valeryl, Capronyl, Capryl, Caprinyl, Lauryl, Myristyl, Palmityl, oder Stearyl; bevorzugt ist C₆-C₁₈-Alkanoyl.

R³, R⁴, R⁵ und R¹⁶ bedeuten als C₁-C₁₈-Alkoxy z.B. Methoxy, Ethoxy, Propoxy, Isopropoxy, Butoxy, Isobutoxy, tert-Butoxy, Pentoxy, Hexoxy, Heptoxy, Octyloxy, Nonyloxy, Decyloxy, Undecyloxy, Dodecyloxy, Tridecyloxy, Tetradecyloxy, Pentadecyloxy, Hexadecyloxy, Heptadecyloxy oder Octadecyloxy; bevorzugt sind C₄-C₁₂-Alkoxy, beispielsweise n-Butoxy, n-Pentoxy, n-Hexoxy, n-Heptoxy, n-Octyloxy, 1-Ethyl-hexyloxy, n-Nonyloxy, n-Decyloxy.

Besonders bevorzugt sind Verbindungen der Formel I, worin R⁵ die Bedeutung OR¹⁷ hat.

R⁵ als substituiertes C₁-C₁₈-Alkoxy stellt vorzugsweise Alkoxyalkyloxy, durch 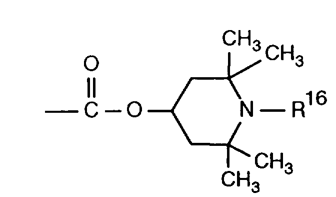 oder 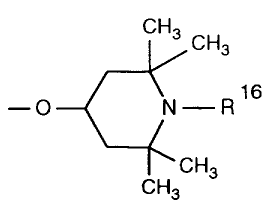 substituiertes Alkyloxy, oder Alkyloxy dar, welches durch Alkenyloyloxy und/oder OR⁷ substituiert ist; von besonderem Interesse ist beispielsweise R⁵ als -OCH₂CH₂OCOCH=CH₂, -OCH₂CH(OR⁷)C₈H₁₇, -OCH₂CH(OR⁷)C₁₂H₂₅, -OCH₂CH(OR⁷)CH₂OC₈H₁₇, -OCH₂CH(OR⁷)CH₂O-(CH₂)₁₂₋₁₄-CH₃ , -OCH₂CH(OR⁷)CH₂OCOC(CH₃)=CH₂, -OCH₂CH(OR⁷)CH₂OCOCH=CH₂,

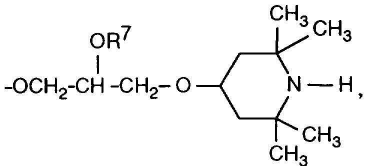

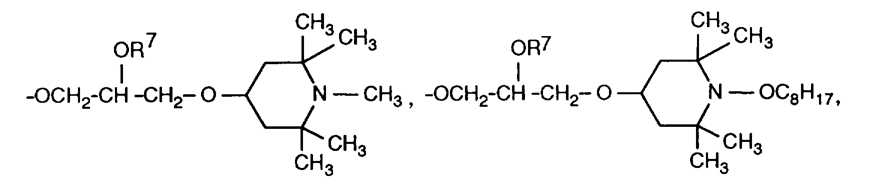

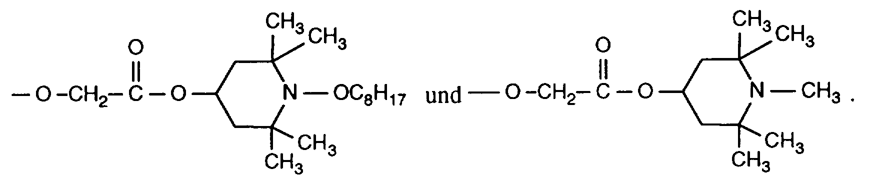

R¹, R, R³ und R⁴ sind besonders bevorzugt Wasserstoff oder Methyl. Von besonderer Bedeutung sind Verbindungen, worin die Reste R¹, R, R³ und R⁴ gleich sind.
R⁷ umfaßt als -CO-R¹¹ beispielsweise die Bedeutungen Acetyl, Propionyl, Butanoyl (Butyryl), Pentanoyl (Valeryl), Hexanoyl (Caproyl), Heptanoyl, Octanoyl, Nonanoyl, Decanoyl, Undecanoyl, Dodecanoyl, Acryloyl, Methacryloyl, Pentenoyl, 9-Undecenoyl, Benzoyl;
als -SO₂-R¹³ beispielsweise Phenylsulfonyl, Heptylsulfonyl, Octylsulfonyl, Nonylsulfonyl, Decylsulfonyl, Undecylsulfonyl, Dodecylsulfonyl, Tridecylsulfonyl, Tetradecylsulfonyl, Pentadecylsulfonyl, Hexadecylsulfonyl, Heptadecylsulfonyl, Octadecylsulfonyl;
als Silylrest beispielsweise Trimethylsilyl, Trimethoxysilyl, Triethylsilyl, Triethoxysilyl, Tripropylsilyl, Tripropoxysilyl, Tributylsilyl, Tributoxysilyl, Dimethylmethoxysilyl, Diethylethoxysilyl, Dimethylethoxysilyl, Dimethoxymethylsilyl, Diethoxyethylsilyl, Diethoxymethylsilyl und ähnliche Niederalkyl- bzw. Niederalkoxysilylreste; als Phosphor-haltiger Rest beispielsweise Dimethoxyphosphoryl, Diethoxyphosphoryl, Dipropoxyphosphoryl, Dibutoxyphosphoryl, Methoxymethylphosphoryl, Ethoxymethylphosphoryl, Propoxymethylphosphoryl, Butoxymethylphosphoryl, Dimethylphosphoryl, Phenylmethylphosphoryl, Phenylmethoxyphosphoryl, Phenylethoxyphosphoryl, sowie entsprechende Reste des 3-wertigen Phosphors. Hervorzuheben sind Verbindungen der Formel I, worin R⁷ eine der Bedeutungen -CO-R¹¹, -SO₂-R¹³ oder 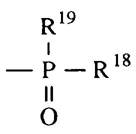 hat.

R⁸, R¹¹, R¹ und R¹⁷ in der Bedeutung C₃-C₁₈Alkenyl umfassen u.a. Allyl, Isopropenyl, 2-Butenyl, 3-Butenyl, Isobutenyl, n-Penta-2,4-dienyl, 3-Methyl-but-2-enyl, n-Oct-2-enyl, n-Dodec-2-enyl, iso-Dodecenyl, n-Octadec-2-enyl, n-Octadec-4-enyl. Bei R¹⁷, R¹¹ und R¹ ist auch die Bedeutung Vinyl möglich. R¹¹ und R¹ als Alkenyl stellen besonders bevorzugt -CH=CH₂ oder -C(CH₃)=CH₂ dar.

R¹⁷ als unsubstituiertes oder substituiertes C₅-C₈-Cycloalkyl ist beispielsweise Cyclopentyl, Cyclohexyl, Cycloheptyl, Cyclooctyl, Methylcyclohexyl oder Acetyloxycyclohexyl; bevorzugt ist Cyclohexyl.

R¹⁶ stellt bevorzugt Wasserstoff; Oxyl; C₂-C₈-Alkanoyl; C₁-C₁₂-Alkyl; C₁-C₁₈-Alkoxy; Cyclohexyl; C₅-C₈-Cycloalkoxy; C₇-C₁₁-Phenylalkyl; oder C₇-C₁₁-Phenylalkyl dar. Besonders bevorzugte Reste R¹⁶ sind Wasserstoff, C₁-C₈-Alkyl, C₁-C₁₈-Alkoxy, C₅-C₈-Cycloalkoxy und C₇-C₉-Phenylalkyl wie z.B. Benzyl α-Methylbenzyl; vor allem Wasserstoff, Methyl, C₆-C₁₂-Alkoxy und Cyclohexyloxy.

R¹⁶ als Cycloalkyloxy ist bevorzugt Cyclohexyloxy; R¹⁶ als substituiertes oder unsubstituiertes C₇-C₁₁-Phenylalkyl ist bevorzugt Benzyl, α-Methylbenzyl oder Methylphenyl-methyl.

Tragen Alkylreste weitere Substituenten oder stellen einzelne Reste Alkylen dar, können freie Valenzen sowie Bindungen an Substituenten von demselben oder von verschiedenen Kohlenstoffatomen ausgehen. Vorzugsweise gehen Bindungen von Alkylresten mit 2 oder mehr Kohlenstoffatomen von verschiedenen Kohlenstoffatomen aus, insbesondere dann, wenn sie an Heteroatome binden.

R¹⁵ und die Reste R⁰ bis R³ als Alkylen bedeuten im Rahmen der angegebenen Definitionen beispielsweise Methylen, Ethylen, Propylen, Butylen, Pentylen, Hexylen, Heptylen, Octylen, Nonylen, Decylen, Undecylen, Dodecylen, Tridecylen, Tetradecylen, Pentadecylen, Hexadecylen, Heptadecylen, Octadecylen; bevorzugt ist, wo möglich, C₂-C₁₈-Alkylen oder C₄-C₁₂-Alkenylen. Besonders bevorzugt sind endständige Reste, d.h. die freien Valenzen befinden sich an den Enden der längsten Kohlenstoftkette.

R⁴ und R⁵ sind, unabhängig voneinander, bevorzugt H oder C₁-C₁₂-Alkyl, beispielsweise H oder CH₃, vor allem Wasserstoff.

Von gesondertem technischem Interesse sind solche Verbindungen der Formel I oder Ia, worin R⁴ und R⁵ in ortho-Position zum Triazinring stehen; insbesondere solche, worin R⁴ H ist, R⁵ Methyl ist, R OR⁷ bedeutet und R¹ C₁-C₁₂-Alkyl ist.

Für A gleich CH, leiten sich die Verbindungen der Formel I von 1,3-Pyrimidin ab, für A gleich N von 1,3,5-Triazin. Für A gleich CH sind R¹ und R, unabhängig voneinander, vorzugsweise Wasserstoff oder Alkyl, besonders H oder C₁-C₄-Alkyl, vor allem H oder Methyl. A steht bevorzugt für ein Stickstoffatom.

Bevorzugt sind Zusammensetzungen enthaltend Verbindungen der Formel I, worin n 1 ist.

Hervorzuheben sind solche Zusammensetzungen, worin in der Verbindung der Formel I
R¹ und R, unabhängig voneinander, H; C₁-C₁₂-Alkyl; Cyclohexyl, Trifluormethyl oder OR⁷ bedeuten;
R³ und R⁴, unabhängig voneinander, H; C₁-C₁₂-Alkyl; Cyclohexyl, C₁-C₁₈-Alkoxy, durch O unterbrochenes und/oder durch OR⁷ substituiertes C₃-C₁₈-Alkoxy, Halogen oder OR⁷ bedeuten;
R⁵ im Fall n = 1 als monovalenter Rest eine der für R¹⁷ angegebenen Bedeutungen hat oder C₆-C₁₈-Alkyl; C₁-C₁₈-Alkoxy; Halogen; -O-CO-R¹; -O-SO₂-R¹³ oder -O-R¹⁷ bedeutet; und im Fall, daß R¹ und R nicht OR⁷ sind, zusätzlich H und C₁-C₅-Alkyl umfallt;
R⁵ im Fall n = 2 als divalenter Rest -O-G-O- bedeutet, wobei G C₂-C₁₆-Alkylen; C₄-C₁₂-Alkenylene; Xylylen; C₃-C₂₀-Alkylen, das durch -O- unterbrochen ist, bedeutet; oder G für eine der Gruppen -CH₂CH(OR⁷)CH₂O-R⁰-OCH₂CH(OR⁷)CH₂-; -CO-R¹-CO-; -CO-NH-R-NH-CO-;
-(CH₂)ₘ-COO-R³-OOC-(CH₂)ₘ-, worin m eine ganze Zahl aus dem Bereich 1 bis 3 ist; oder 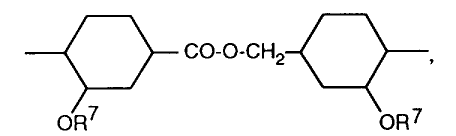 steht;
R⁶ in o-Position zu R⁵ und in p-Position zu OR⁷ steht;
R⁹ und R¹⁰, unabhängig voneinander, C₁-C₁₂-Alkyl; C₃-C₁₂-Alkoxyalkyl;
C₄-C₁₆-Dialkylaminoalkyl oder Cyclohexyl bedeuten oder
R⁹ und R¹⁰ zusammen C₃-C₉-Alkylen oder -Oxaalkylen oder -Azaalkylen bedeuten;
R¹¹ C₁-C₁₈-Alkyl; Trihalogenmethyl; C₂-C₁₈-Alkenyl; -CH₂-CO-CH₃; C₇-C₁₂-Aralkyl;
C₁-C₁₂-Alkoxy; oder Phenyl bedeutet, welches unsubstituiert oder durch C₁-C₁₂-Alkyl,
C₁-C₄-Alkoxy, Halogen und/oder Benzyl substituiert ist;
R¹⁷ C₅-C₁₈-Alkyloxycarbonyl; oder C₂-C₁₈-Alkenyl bedeutet; oder R¹⁷ C₁-C₁₈-Alkyl bedeutet, das substituiert ist durch OR⁷, C₁-C₁₈-Alkoxy, C₂-C₁₈-Alkanoyl,
C₂-C₈-Alkenyloxy, Halogen, -COOR⁸, -CONH₂, -CONHR⁹, -CON(R⁹)(R¹⁰), -NHR⁹, -N(R⁹)(R¹⁰), -NHCOR¹¹, -CN, -OCOR¹¹, eine Gruppe der Formel 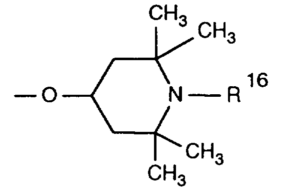 und/oder Phenoxy, welches unsubstituiert ist oder durch C₁-C₁₈-Alkyl, C₁-C₁₈-Alkoxy, oder Halogen substituiert ist; oder R¹⁷ C₄-C₂₀-Alkyl darstellt, welches durch O unterbrochen ist und durch OR⁷ substituiert sein kann; oder R¹⁷ Glycidyl; Cyclohexyl;
durch OR⁷, C₁-C₄-Alkyl oder -OCOR¹¹ substituiertes Cyclohexyl; oder unsubstituiertes oder durch Cl oder CH₃ substituiertes C₇-C₁₁-Phenylalkyl bedeutet; und
R¹⁸ und R¹⁹, unabhängig voneinander, C₁-C₆-Alkoxy, C₁-C₄-Alkyl, Cyclohexyl, Tolyl oder Phenyl darstellen;
R⁴ und R⁵, unabhängig voneinander, H oder C₁-C₁₂-Alkyl sind; und
Z⁵, Z⁶ und Z⁷, unabhängig voneinander, C₁-C₄-Alkyl, oder C₁-C₄-Alkoxy bedeuten.

Von besonderer Bedeutung sind Zusammensetzungen, worin in der Verbindung der Formel I
R¹ und R, unabhängig voneinander, H; C₁-C₄-Alkyl oder OR⁷ bedeuten;
R³ und R⁴, unabhängig voneinander, H; C₁-C₄-Alkyl; C₁-C₈-Alkoxy, durch O unterbrochenes und/oder durch OR⁷ substituiertes C₃-C₁₂-Alkoxy, Halogen oder OR⁷ bedeuten;
R⁵ im Fall n = 1 als monovalenter Rest eine der für R¹⁷ angegebenen Bedeutungen hat oder Halogen; C₆-C₁₈-Alkyl; C₁-C₁₈-Alkoxy; -O-CO-R¹; -O-SO₂-R¹³ oder -O-R¹⁷ bedeutet; und im Fall, daß R¹ und R nicht OR⁷ sind, zusätzlich H und C₁-C₅-Alkyl umfaßt;
R⁵ im Fall n = 2 als divalenter Rest -O-G-O- bedeutet, wobei
G C₂-C₁₆-Alkylen; C₄-C₁₂-Alkenylene; oder Xylylen bedeutet; oder G für eine der Gruppen -CH₂CH(OR⁷)CH₂O-R⁰-OCH₂CH(OR⁷)CH₂-; -CO-R¹-CO-;
-CO-NH-R-NH-CO-; -(CH₂)ₘ-COO-R³-OOC-(CH₂)ₘ- steht, worin m eine ganze Zahl aus dem Bereich 1 bis 3 ist;
R⁶ H; Allyl; C₁-C₁₀-Alkyl; Acetyl oder Benzoyl ist;
R⁷ eine der Bedeutungen -CO-R¹¹, -SO₂-R¹³, 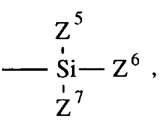 Allyl oder 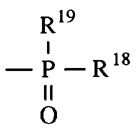 hat oder eine Gruppe der Formel 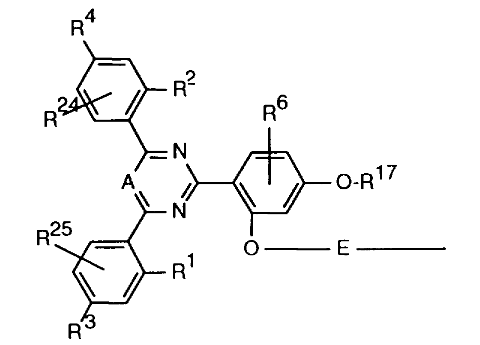 darstellt, worin E C₃-C₁₈-Alkylen oder C₄-C₁₈-Alkenylen ist;
R⁸ C₁-C₁₈-Alkyl; C₃-C₁₈-Alkenyl; durch O, N oder S unterbrochenes und/oder durch OR⁷ substituiertes C₃-C₂₀-Alkyl; Glycidyl; oder eine Gruppe der Formel 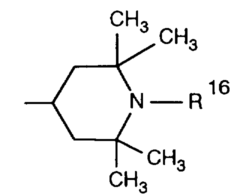 darstellt;
R⁹ und R¹⁰, unabhängig voneinander, C₁-C₁₂-Alkyl bedeuten oder zusammen C₄-C₉-Alkylen oder -Oxaalkylen oder -Azaalkylen bedeuten;
R¹¹ C₁-C₁₂-Alkyl; Trihalogenmethyl; C₂-C₄-Alkenyl; C₁-C₁₂-Alkoxy; Phenyl; Tolyl oder Xylyl bedeutet;
R¹ C₁-C₁₈-Alkyl; C₂-C₁₈-Alkenyl; Phenyl; -R¹⁵-O-CO-C(CH₃)=CH₂; oder
-R¹⁵-O-CO-CH=CH₂ bedeutet; oder eine Gruppe der Formel 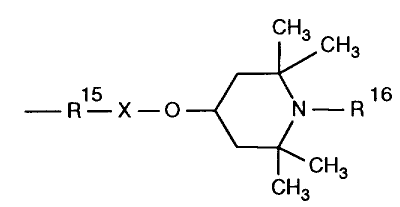 darstellt;
R¹³ Phenyl oder C₇-C₁₈-Alkylphenyl bedeutet; und
R¹⁵ C₂-C₁₂-Alkylen;
R¹⁶ Wasserstoff; Oxyl; C₂-C₈-Alkanoyl; C₁-C₁₂-Alkyl; C₁-C₁₈-Alkoxy; Cyclohexyl;
C₅-C₁₂-Cycloalkoxy; C₇-C₁₁-Phenylalkyl; oder C₇-C₁₁-Phenylalkoxy darstellt;
R¹⁷ C₃-C₁₈-Alkenyl bedeutet; oder R¹⁷ C₁-C₁₂-Alkyl bedeutet, das substituiert ist durch OR⁷, C₁-C₁₈-Alkoxy, -COOR⁸, -CONHR⁹, -CON(R⁹)(R¹⁰), -OCOR¹¹, und/oder eine Gruppe der Formel 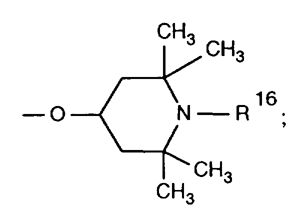 oder R¹⁷ durch 1 bis 6 O unterbrochenes C₄-C₂₀-Alkyl, welches durch OR⁷ substituiert sein kann; oder Glycidyl; C₅-C₁₂-Cycloalkyl; oder C₇-C₁₁-Phenylalkyl bedeutet;
R¹⁸ und R¹⁹, unabhängig voneinander, C₁-C₄-Alkoxy, Methyl oder Phenyl darstellen;
R⁰ C₂-C₁₀-Alkylen; Phenylen; oder eine Gruppe -Phenylen-D-Phenylen- ist, worin D für -O-, -SO₂- oder -C(CH₃)₂- steht;
R¹ C₂-C₁₀-Alkylen; Phenylen; oder C₂-C₆-Alkenylen;
R C₂-C₁₀-Alkylen; oder 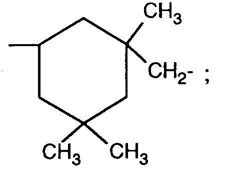
R³ C₂-C₁₀-Alkylen ist; und
R⁴ und R⁵, unabhängig voneinander, H oder Methyl sind.

Bevorzugt sind darunter solche Zusammensetzungen, worin in der Verbindung der Formel I
A für ein Stickstoffatom steht;
R⁶ in o-Position zu R⁵ und in p-Position zu OR⁷ steht; und
R⁷ C₂-C₈-Alkanoyl, Benzoyl, Phenylsulfonyl, -CO-CF₃, Allyl, 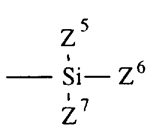 oder C₇-C₉-Alkylphenylsulfonyl bedeutet oder eine Gruppe der Formel 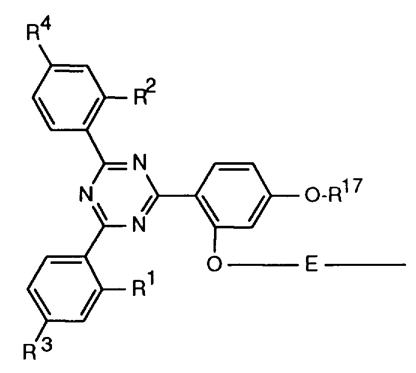 darstellt, worin E C₃-C₆-Alkylen oder C₄-C₆-Alkenylen ist; und
Z⁵, Z⁶ und Z⁷, unabhängig voneinander, C₁-C₄-Alkyl bedeuten;
insbesondere solche, worin in der Verbindung der Formel I
R¹ und R, unabhängig voneinander, H; C₁-C₄-Alkyl oder OR⁷ bedeuten;
R³ und R⁴, unabhängig voneinander, H; C₁-C₄-Alkyl; C₁-C₄-Alkoxy, durch O unterbrochenes und durch OR⁷ substituiertes C₃-C₁₂-Alkoxy, Cl oder OR⁷ bedeuten;
R⁵ im Fall n = 1 -O-CO-R¹ oder -O-R¹⁷ bedeutet; und im Fall, daß R¹ und R nicht OR⁷ sind, zusätzlich H umfaßt;
R⁵ im Fall n = 2 -O-G-O- bedeutet, wobei G C₂-C₁₆-Alkylen ist;
R⁶ H oder C₁-C₁₀-Alkyl ist;
R⁸ C₁-C₈-Alkyl darstellt;
R¹¹ C₁-C₃-Alkyl; Trifluormethyl; Trichlormethyl; C₁-C₄-Alkoxy; C₂-C₃-Alkenyl; Phenyl; Tolyl oder Xylyl bedeutet;
R¹ C₁-C₈-Alkyl; oder Phenyl bedeutet;
R¹³ Phenyl oder C₇-C₉-Alkylphenyl bedeutet;
R¹⁶ Wasserstoff; Oxyl; C₁-C₁₂-Alkyl; C₁-C₁₈-Alkoxy; Cyclohexyloxy; oder
C₇-C₁₁-Phenylalkyl darstellt;
R¹⁷ C₁-C₁₈-Alkyl; oder Allyl bedeutet; oder R¹⁷ C₁-C₁₂-Alkyl bedeutet, das substituiert ist durch OR⁷, C₁-C₁₈-Alkoxy, -COOR⁸ und/oder -OCOR¹¹.

Einige der oben als Komponente (B) beschriebenen Verbindungen der Formel I sind neue Verbindungen. Gegenstand der Erfindung sind daher auch Verbindungen der Formel Ia 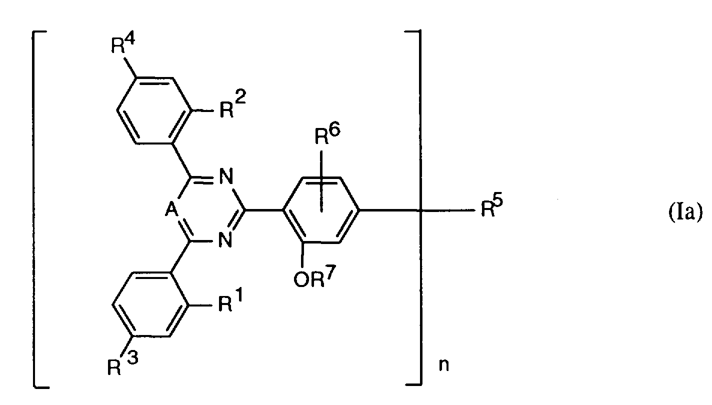
worin n 1 oder 2 ist;
A für CH oder ein Stickstoffatom steht;
R¹ und R, unabhängig voneinander, H; C₁-C₁₂-Alkyl; C₅-C₁₂-Cycloalkyl, Trifluormethyl oder OR⁷ bedeuten;
R³ und R⁴, unabhängig voneinander, H; C₁-C₁₂-Alkyl; C₅-C₁₂-Cycloalkyl, OR¹⁷, Halogen oder OR⁷ bedeuten;
R⁵ im Fall n = 1 als monovalenter Rest C₆-C₁₈-Alkyl; C₁-C₁₈-Alkoxy; Halogen; -O-SO₂-R¹³; 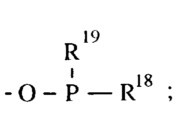 oder 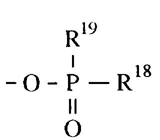 bedeutet; oder R⁵ im Fall n = 1 eine der für R¹⁷ angegebenen Bedeutungen hat oder -O-R¹⁷ bedeutet; und R⁵ als monovalenter Rest im Fall, daß R¹ und R nicht OR⁷ sind, zusätzlich H und C₁-C₅-Alkyl umfaßt;
R⁵ im Fall n = 2 als divalenter Rest -O-G-O- bedeutet, wobei
G C₂-C₁₆-Alkylen; C₄-C₁₂-Alkenylene; Xylylen; oder C₃-C₂₀-Alkylen, das durch -O- unterbrochen und/oder durch OR⁷ substituiert ist, bedeutet; oder G für eine der Gruppen -CH₂CH(OR⁷)CH₂O-R⁰-OCH₂CH(OR⁷)CH₂-; -CO-R¹-CO-; -CO-NH-R-NH-CO-; -(CH₂)ₘ-COO-R³-OOC-(CH₂)ₘ-, worin m eine ganze Zahl aus dem Bereich 1 bis 3 ist; oder 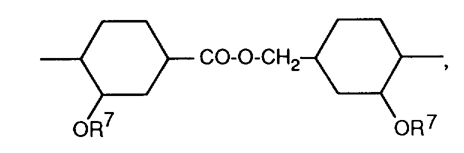 steht;
R⁶ H; C₂-C₁₈-Alkenyl; -X-Z³; unsubstituiertes oder am Phenylkern durch Methyl, Halogen, -CN oder Methoxy substituiertes Benzoyl; Halogen; -SR¹⁴; -SOR¹³; -SO₂R¹³; -C(Z³)=N-Z³; -CH(Z³)-NH-Z³; ein Rest der Formel 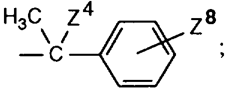 oder ein Rest der Formel 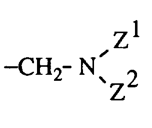 ist;
R⁷ eine der Bedeutungen -CO-R¹¹, -SO₂-R¹³, 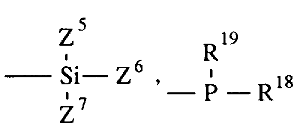 oder 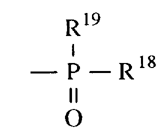 hat oder Allyl oder eine Gruppe der Formel 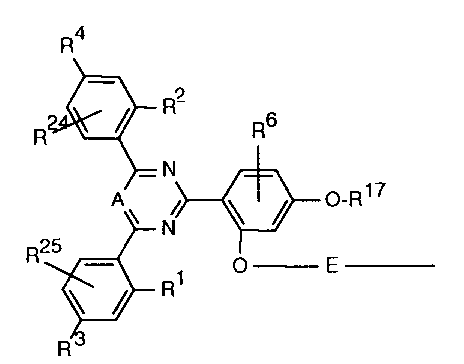 darstellt, worin E C₃-C₁₈-Alkylen oder C₄-C₁₈-Alkenylen ist;
R⁸ C₁-C₁₈-Alkyl; C₃-C₁₈-Alkenyl; durch O, N oder S unterbrochenes und/oder durch OR⁷ substituiertes C₃-C₂₀-Alkyl; durch -P(O)(OR¹⁴)₂, -N(R⁹)(R¹⁰) oder -OCOR¹¹ und/oder OR⁷ substituiertes C₁-C₄-Alkyl; Glycidyl; C₅-C₁₂-Cycloalkyl oder C₇-C₁₁-Phenylalkyl bedeutet; oder eine Gruppe der Formel 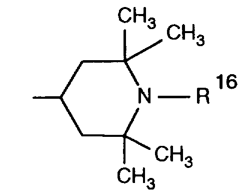 darstellt;
R⁹ und R¹⁰, unabhängig voneinander, C₁-C₁₂-Alkyl; C₃-C₁₂-Alkoxyalkyl;
C₄-C₁₆-Dialkylaminoalkyl oder C₅-C₁₂-Cycloalkyl bedeuten oder
R⁹ und R¹⁰ zusammen C₃-C₉-Alkylen oder -Oxaalkylen oder -Azaalkylen bedeuten;
R¹¹ C₁-C₁₈-Alkyl; C₅-C₁₂-Cycloakyl; durch Halogen substituiertes C₁-C₁₈-Alkyl;
C₂-C₁₈-Alkenyl; -CH₂-CO-CH₃; C₇-C₁₂-Aralkyl; C₁-C₁₂-Alkoxy; oder Phenyl bedeutet, welches unsubstituiert oder durch C₁-C₁₂-Alkyl, C₁-C₄-Alkoxy, Halogen und/oder Benzyl substituiert ist;
R¹³ C₁-C₁₂-Alkyl; C₆-C₁₀-Aryl; oder C₇-C₁₈-Alkylaryl bedeutet; und
R¹⁴ C₁-C₁₂-Alkyl oder Phenyl oder C₇-C₁₅-Phenylalkyl;
R¹⁵ C₁-C₁₈-Alkylen oder C₂-C₁₈-Alkenylen;
R¹⁶ Wasserstoff; Oxyl; Formyl; C₂-C₈-Alkanoyl; C₁-C₁₈-Alkyl; C₁-C₁₈-Alkoxy;
C₅-C₁₂-Cycloalkyl; C₅-C₁₂-Cycloalkoxy; C₇-C₁₁-Phenylalkyl; C₇-C₁₁-Phenylalkyl, welches am Phenylring durch 1 bis 3 Reste C₁-C₄-Alkyl oder C₁-C₈-Alkanoyl substituiert ist; oder C₇-C₁₁-Phenylalkoxy;
R¹⁷ C₅-C₁₈-Alkyloxycarbonyl; oder C₂-C₁₈-Alkenyl bedeutet; oder R¹⁷ C₁-C₁₈-Alkyl bedeutet, das substituiert ist durch OR⁷, C₁-C₁₈-Alkoxy, C₅-C₁₂-Cycloalkoxy, C₂-C₁₈-Alkanoyl, C₂-C₈-Alkenyloxy, Halogen, -COOR⁸, -CONH₂, -CONHR⁹, -CON(R⁹)(R¹⁰), -NHR⁹, -N(R⁹)(R¹⁰), -NHCOR¹¹, -CN, -OCOR¹¹, eine Gruppe der Formel 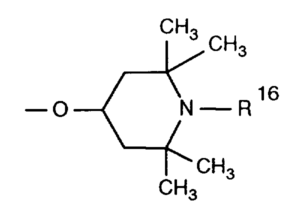 und/oder Phenoxy, welches unsubstituiert ist oder durch C₁-C₁₈-Alkyl, C₁-C₁₈-Alkoxy oder Halogen substituiert ist; oder R¹⁷ C₃-C₅₀-Alkyl bedeutet, welches durch O unterbrochen ist und durch OR⁷ substituiert sein kann; oder R¹⁷ Glycidyl;
C₅-C₁₂-Cycloalkyl; durch OR⁷, C₁-C₄-Alkyl oder -OCOR¹¹ substituiertes
C₅-C₁₂-Cycloalkyl; oder unsubstituiertes oder durch OR⁷, Cl oder CH₃ substituiertes
C₇-C₁₁-Phenylalkyl bedeutet;
R¹⁸ und R¹⁹, unabhängig voneinander, C₁-C₁₂-Alkoxy, Phenoxy, C₁-C₁₂-Alkyl, C₅-C₁₂-Cycloalkyl, Benzyl, Tolyl oder Phenyl darstellen;
R⁰ C₂-C₁₀-Alkylen; durch -O- unterbrochenes C₄-C₅₀-Alkylen; Phenylen; oder eine Gruppe -Phenylen-D-Phenylen- ist, worin D für -O-, -S-, -SO₂-, -CH₂- oder -C(CH₃)₂- steht;
R¹ C₂-C₁₀-Alkylen; durch O oder S unterbrochenes C₂-C₁₀-Alkylen; C₆-C₁₂-Arylen; oder C₂-C₆-Alkenylen;
R C₂-C₁₀-Alkylen; Phenylen; Tolylen; Diphenylenmethan; oder 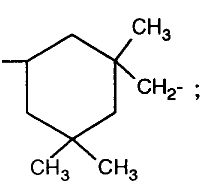 und
R³ C₂-C₁₀-Alkylen oder durch -O- unterbrochenes C₄-C₂₀-Alkylen ist;
R⁴ und R⁵, unabhängig voneinander, H, C₁-C₁₂-Alkyl; C₂-C₆-Alkenyl;
C₁-C₁₂-Alkoxy; C₃-C₁₈-Alkenyloxy; Halogen; Trifluormethyl; C₇-C₁₁-Phenylalkyl;
Phenyl; durch C₁-C₁₈-Alkyl, C₁-C₁₈-Alkoxy oder Halogen substituiertes Phenyl;
Phenyloxy; oder durch C₁-C₁₈-Alkyl, C₁-C₁₈-Alkoxy oder Halogen substituiertes Phenyloxy darstellen;
X eine direkte Bindung oder -CO- bedeutet;
Z¹ und Z, unabhängig voneinander, C₁-C₁₂-Alkyl sind oder gemeinsam C₄-C₁₀-Alkylen darstellen, das durch ein Sauerstoffatom unterbrochen sein kann;
Z³ C₁-C₂₀-Alkyl oder C₇-C₁₅-Phenylalkyl und
Z⁴ und Z⁸, unabhängig voneinander, Wasserstoff oder Methyl sind; und
Z⁵, Z⁶ und Z⁷, unabhängig voneinander, C₁-C₁₈-Alkyl, Cyclohexyl, Phenyl oder C₁-C₁₈-Alkoxy bedeuten.

Für Verbindungen der Formel Ia gelten im wesentlichen die oben für Verbindungen der Formel I beschriebenen Bevorzugungen. Von besonderem Interesse sind weiterhin Verbindungen der Formel Ia, worin
R¹ und R, unabhängig voneinander, H; C₁-C₁₂-Alkyl; Cyclohexyl, Trifluormethyl oder OR⁷ bedeuten;
R³ und R⁴, unabhängig voneinander, H; C₁-C₁₂-Alkyl; Cyclohexyl, C₁-C₁₈-Alkoxy, durch O unterbrochenes und/oder durch OR⁷ substituiertes C₃-C₁₈-Alkoxy, Halogen oder OR⁷ bedeuten;
R⁵ im Fall n = 1 als monovalenter Rest eine der für R¹⁷ angegebenen Bedeutungen hat oder C₆-C₁₈-Alkyl; C₁-C₁₈-Alkoxy; Halogen; -O-SO₂-R¹³ oder -O-R¹⁷ bedeutet; und im Fall, daß R¹ und R nicht OR⁷ sind, zusätzlich H und C₁-C₅-Alkyl umfaßt;
R⁵ im Fall n = 2 als divalenter Rest -O-G-O- bedeutet, wobei
G C₂-C₁₆-Alkylen; C₄-C₁₂-Alkenylene; Xylylen; C₃-C₂₀-Alkylen, das durch -O- unterbrochen ist, bedeutet; oder G für eine der Gruppen
-CH₂CH(OR⁷)CH₂O-R⁰-OCH₂CH(OR⁷)CH₂-; -CO-R¹-CO-; -CO-NH-R-NH-CO-;
-(CH₂)ₘ-COO-R³-OOC-(CH₂)ₘ-, worin m eine ganze Zahl aus dem Bereich 1 bis 3 ist; oder 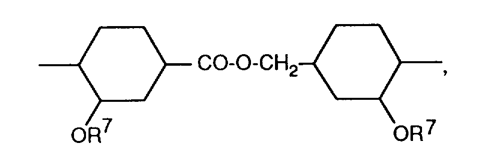 steht;
R⁶ in o-Position zu R⁵ und in p-Position zu OR⁷ steht;
R⁹ und R¹⁰, unabhängig voneinander, C₁-C₁₂-Alkyl; C₃-C₁₂-Alkoxyalkyl;
C₄-C₁₆-Dialkylaminoalkyl oder Cyclohexyl bedeuten oder
R⁹ und R¹⁰ zusammen C₃-C₉-Alkylen oder -Oxaalkylen oder -Azaalkylen bedeuten;
R¹¹ C₁-C₁₈-Alkyl; Trihalogenmethyl; C₂-C₁₈-Alkenyl; -CH₂-CO-CH₃; C₇-C₁₂-Aralkyl;
C₁-C₁₂-Alkoxy; oder Phenyl bedeutet, welches unsubstituiert oder durch C₁-C₁₂-Alkyl,
C₁-C₄-Alkoxy, Halogen und/oder Benzyl substituiert ist;
R¹⁷ C₅-C₁₈-Alkyloxycarbonyl; oder C₂-C₁₈-Alkenyl bedeutet; oder R¹⁷ C₁-C₁₈-Alkyl bedeutet, das substituiert ist durch OR⁷, C₁-C₁₈-Alkoxy, C₂-C₁₈-Alkanoyl,
C₂-C₈-Alkenyloxy, Halogen, -COOR⁸, -CONH₂, -CONHR⁹, -CON(R⁹)(R¹⁰), -NHR⁹, -N(R⁹)(R¹⁰), -NHCOR¹¹, -CN, -OCOR¹¹, eine Gruppe der Formel 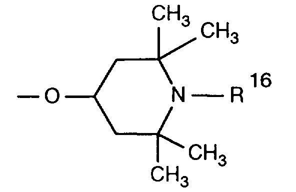 und/oder Phenoxy, welches unsubstituiert ist oder durch C₁-C₁₈-Alkyl, C₁-C₁₈-Alkoxy, oder Halogen substituiert ist; oder R¹⁷ C₄-C₂₀-Alkyl darstellt, welches durch O unterbrochen ist und durch OR⁷ substituiert sein kann; oder R¹⁷ Glycidyl; Cyclohexyl;
durch OR⁷, C₁-C₄-Alkyl oder -OCOR¹¹ substituiertes Cyclohexyl; oder unsubstituiertes oder durch Cl oder CH₃ substituiertes C₇-C₁₁-Phenylalkyl bedeutet; und
R¹⁸ und R¹⁹, unabhängig voneinander, C₁-C₆-Alkoxy, C₁-C₄-Alkyl, Cyclohexyl, Tolyl oder Phenyl darstellen;
R⁴ und R⁵, unabhängig voneinander, H oder C₁-C₁₂-Alkyl sind; und
Z⁵, Z⁶ und Z⁷, unabhängig voneinander, C₁-C₄-Alkyl, oder C₁-C₄-Alkoxy bedeuten.

Insbesondere von Interesse sind Verbindungen der Formel Ia, worin
R¹ und R, unabhängig voneinander, H; C₁-C₄-Alkyl oder OR⁷ bedeuten;
R³ und R⁴, unabhängig voneinander, H; C₁-C₄-Alkyl; C₁-C₈-Alkoxy, durch O unterbrochenes und/oder durch OR⁷ substituiertes C₃-C₁₂-Alkoxy, Halogen oder OR⁷ bedeuten;
R⁵ im Fall n = 1 als monovalenter Rest eine der für R¹⁷ angegebenen Bedeutungen hat oder Halogen; C₆-C₁₈-Alkyl; C₁-C₁₈-Alkoxy; -O-SO₂-R¹³ oder -O-R¹⁷ bedeutet; und im Fall, daß R¹ und R nicht OR⁷ sind, zusätzlich H und C₁-C₅-Alkyl umfaßt;
R⁵ im Fall n = 2 als divalenter Rest -O-G-O- bedeutet, wobei
G C₂-C₁₆-Alkylen; C₄-C₁₂-Alkenylene; oder Xylylen bedeutet; oder G für eine der Gruppen -CH₂CH(OR⁷)CH₂O-R⁰-OCH₂CH(OR⁷)CH₂-; -CO-R¹-CO-;
-CO-NH-R-NH-CO-; -(CH₂)ₘ-COO-R³-OOC-(CH₂)ₘ- steht, worin m eine ganze Zahl aus dem Bereich 1 bis 3 ist;
R⁶ H; Allyl; C₁-C₁₀-Alkyl; Acetyl oder Benzoyl ist;
R⁷ eine der Bedeutungen -CO-R¹¹, -SO₂-R¹³, 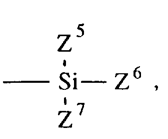 Allyl oder 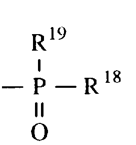 hat oder eine Gruppe der Formel 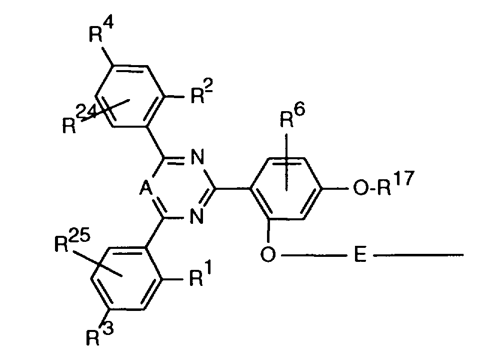 darstellt, worin E C₃-C₁₈-Alkylen oder C₄-C₁₈-Alkenylen ist;
R⁸ C₁-C₁₈-Alkyl; C₃-C₁₈-Alkenyl; durch O, N oder S unterbrochenes und/oder durch OR⁷ substituiertes C₃-C₂₀-Alkyl; Glycidyl; oder eine Gruppe der Formel 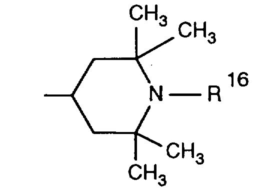 darstellt;
R⁹ und R¹⁰, unabhängig voneinander, C₁-C₁₂-Alkyl bedeuten oder zusammen
C₄-C₉-Alkylen oder -Oxaalkylen oder -Azaalkylen bedeuten;
R¹¹ C₁-C₁₂-Alkyl; Trihalogenmethyl; C₂-C₄-Alkenyl; C₁-C₁₂-Alkoxy; Phenyl; Tolyl oder Xylyl bedeutet;
R¹³ Phenyl oder C₇-C₁₈-Alkylphenyl bedeutet; und
R¹⁵ C₂-C₁₂-Alkylen;
R¹⁶ Wasserstoff; Oxyl; C₂-C₈-Alkanoyl; C₁-C₁₂-Alkyl; C₁-C₁₈-Alkoxy; Cyclohexyl;
C₅-C₁₂-Cycloalkoxy; C₇-C₁₁-Phenylalkyl; oder C₇-C₁₁-Phenylalkxy darstellt;
R¹⁷ C₃-C₁₈-Alkenyl bedeutet; oder R¹⁷ C₁-C₁₂-Alkyl bedeutet, das substituiert ist durch OR⁷, C₁-C₁₈-Alkoxy, -COOR⁸, -CONHR⁹, -CON(R⁹)(R¹⁰), -OCOR¹¹, und/oder eine Gruppe der Formel 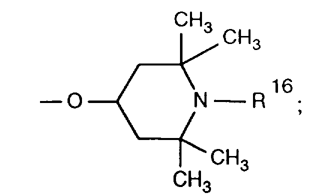 oder R¹⁷ durch 1 bis 6 O unterbrochenes C₄-C₂₀-Alkyl, welches durch OR⁷ substituiert sein kann; oder Glycidyl; C₅-C₁₂-Cycloalkyl; oder C₇-C₁₁-Phenylalkyl bedeutet;
R¹⁸ und R¹⁹, unabhängig voneinander, C₁-C₄-Alkoxy, Methyl oder Phenyl darstellen;
R⁰ C₂-C₁₀-Alkylen; Phenylen; oder eine Gruppe -Phenylen-D-Phenylen- ist, worin D für -O-, -SO₂- oder -C(CH₃)₂- steht;
R¹ C₂-C₁₀-Alkylen; Phenylen; oder C₂-C₆-Alkenylen;
R C₂-C₁₀-Alkylen; oder 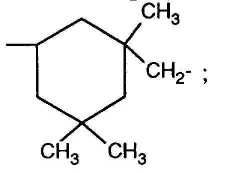
R³ C₂-C₁₀-Alkylen ist; und
R⁴ und R⁵, unabhängig voneinander, H oder Methyl sind.

Besonders hervorzuheben sind darunter solche Verbindungen, worin
A für ein Stickstoffatom steht;
R⁶ in o-Position zu R⁵ und in p-Position zu OR⁷ steht; und
R⁷ C₂-C₈-Alkanoyl, Benzoyl, Phenylsulfonyl, Allyl, -CO-CF₃, 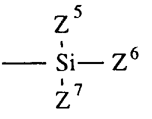 oder C₇-C₉-Alkylphenylsulfonyl bedeutet oder eine Gruppe der Formel 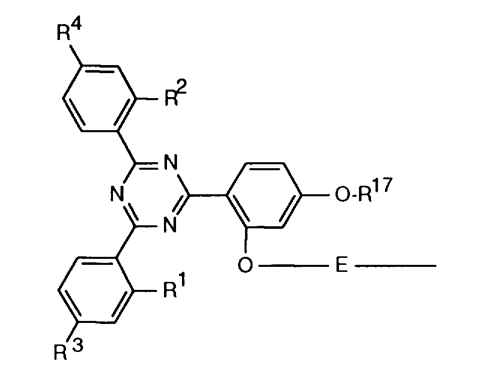 darstellt, worin E C₃-C₆-Alkylen oder C₄-C₆-Alkenylen ist; und
Z⁵, Z⁶ und Z⁷, unabhängig voneinander, C₁-C₄-Alkyl bedeuten;
vor allem diejenigen Verbindungen der Formel Ia, worin
R¹ und R, unabhängig voneinander, H; C₁-C₄-Alkyl oder OR⁷ bedeuten;
R³ und R⁴, unabhängig voneinander, H; C₁-C₄-Alkyl; C₁-C₄-Alkoxy, durch O unterbrochenes und durch OR⁷ substituiertes C₃-C₁₂-Alkoxy, Cl oder OR⁷ bedeuten;
R⁵ im Fall n = 1 -O-R¹⁷ bedeutet und im Fall, daß R¹ und R nicht OR⁷ sind, zusätzlich H umfaßt; und im Fall n = 2 -O-G-O- bedeutet, wobei G C₂-C₁₆-Alkylen ist;
R⁶ H oder C₁-C₁₀-Alkyl ist;
R⁸ C₁-C₈-Alkyl darstellt;
R¹¹ C₁-C₃-Alkyl; Trifluormethyl; Trichlormethyl; C₁-C₄-Alkoxy; C₂-C₃-Alkenyl; Phenyl; Tolyl oder Xylyl bedeutet;
R¹³ Phenyl oder C₇-C₉-Alkylphenyl bedeutet;
R¹⁶ Wasserstoff; Oxyl; C₁-C₁₂-Alkyl; C₁-C₁₈-Alkoxy; Cyclohexyloxy; oder C₇-C₁₁-Phenylalkyl darstellt;
R¹⁷ C₁-C₁₈-Alkyl; oder Allyl bedeutet; oder R¹⁷ C₁-C₁₂-Alkyl bedeutet, das substituiert ist durch OR⁷, C₁-C₁₈-Alkoxy, -COOR⁸ und/oder -OCOR¹¹; und
R⁴ und R⁵, unabhängig voneinander, H oder Methyl sind.

Zur Herstellung von Verbindungen der Formel I und Ia kann von freie OH-Gruppen enthaltenden 2,4,6-Tris-aryl-1,3,5-triazinen oder 2,4,6-Tris-aryl-1,3-pyrimidinen ausgegangen werden. Diese Ausgangsverbindungen können gemäß oder in Analogie zu einer der in EP-A-434 608, US-A-3 442 898, US-A-4 895 981 oder in der Publikation von H. Brunetti und C.E. Lüthi, Helv. Chim. Acta 55, 1566 (1972), angegebenen Methoden durch Friedel-Crafts-Addition von Halogentriazinen oder -pyrimidinen an entsprechende Phenole erhalten werden. Daran kann sich eine weitere Umsetzung nach bekannten Methoden anschließen zu Verbindungen der Formel I oder Ia, worin R⁵ ungleich Wasserstoff oder OR⁷ ist; solche Umsetzungen und Verfahren sind beispielsweise beschrieben in EP-A-434 608, Seite 15, Zeile 11, bis Seite 17, Zeile 1; vergl. auch US-A-3 442 898. Auch Umsetzungen mit Verbindungsgemischen, beispielsweise technischen Isomerengemischen, sind möglich, die zu entsprechenden Produktgemischen führen; solche Reaktionen können entsprechend oder in Analogie zu Verfahren durchgeführt werden, wie sie z.B. in der EP-A-444 323 beschrieben sind.

Diese Ausgangsverbindungen, die noch freie OH-Gruppen enthalten, können anschließend einer weiteren Reaktion unter Bildung der erfindungsgemäßen Kryptolichtschutzmittel unterworfen werden. Diese Reaktion kann beispielsweise nach bekannten Methoden in Analogie zu den in US-A-4 775 707, US-A-5 030 731, J. Appl. Pol. Science 22, 2165-2172 (1978) und J. Appl. Pol. Science 28, 1159-1165 (1983) beschriebenen Verfahren durchgeführt werden. Eine Acylierung zu Verbindungen, worin R⁷ -CO-R¹¹ bedeutet, kann auch in Anlehnung an das in US-A-3 249 608 beschriebene Verfahren erfolgen, wobei das Acylierungsreagens jedoch zweckmäßig im Überschuß eingesetzt wird.

So kann die Umsetzung zu Verbindungen der Formel I oder Ia, worin R⁷ -CO-R¹¹, SO₂-R¹³, 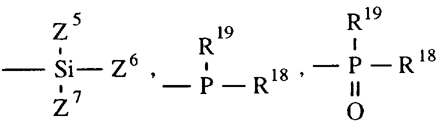 oder Allyl bedeutet, beispielsweise durch Reaktion mit den entsprechenden Chloriden Cl-CO-R¹¹, Cl-SO₂-R¹³, 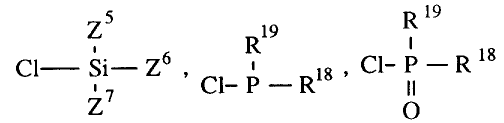 oder Allylchlorid erfolgen. Acylierte Verbindungen können weiterhin durch Umsetzung mit Anhydriden, Ketenen oder Estern, beispielsweise Niederalkylestern, erhalten werden.

Die genannten Reagentien können in etwa equimolarer Menge bezüglich der in der Ausgangsverbindung vorhandenen Hydroxylgruppen eingesetzt werden. Vorteilhaft ist jedoch ihr Einsatz im Überschuß, beispielsweise 2 bis 20 Mol pro Mol umzusetzendes OH.

Häufig werden die für die jeweilige Acylierungs-, Sulfonylierungs-, Phosphonylierungs- oder Silylierungsreaktion gebräuchlichen Katalysatoren eingesetzt. So werden für Acylierungs- und Sulfonylierungsreaktionen häufig tertiäre oder quaternäre Amine wie z.B. Triethylamin, Dimethylaminopyridin oder Tetrabutylammoniumsalze verwendet; Einzelheiten können beispielsweise der oben aufgeführten US-A-4 775 707 entnommen werden.

Die Umsetzung kann mit oder ohne Lösemittel durchgeführt werden. Gegebenenfalls können die genannten Reagentien selbst als Lösemittel eingesetzt werden, zweckmäßig geschieht dies z.B. bei Verwendung von Anhydriden, die im Bereich von ca. 20-100°C flüssig sind, beispielsweise bei Umsetzung mit Acetanhydrid oder Benzoesäureanhydrid. Häufig werden auch inerte organische Lösemittel verwendet, beispielsweise Kohlenwasserstoffe wie Toluol, Xylol, Petrolether, Ligroin; oder chlorierte Kohlenwasserstoffe wie CCl₄, CHCl₃, Monochlorbenzol; oder Ether wie Tetrahydrofuran oder Dibutylether; oder andere inerte Lösemittel wie z.B. Acetonitril.

Die Temperatur ist allgemein nicht kritisch; meist wird bei Temperaturen gearbeitet, die zwischen 20°C und dem Siedepunkt des Lösemittels liegen, beispielsweise zwischen 50°C und 150°C. Die Aufarbeitung kann nach gängigen Methoden erfolgen, z.B. durch Ausfällen des Produktes in Wasser, Filtration und Trocknen; im Bedarfsfall können weitere Reinigungsschritte vorgenommen werden wie z.B. Umkristallisation.

Die Verbindungen der Formeln I und Ia lassen sich vorteilhaft als Stabilisatoren für organische Materialien gegen Schädigung durch Licht, Sauerstoff oder Hitze einsetzen. Ganz besonders eignen sich diese Verbindungen als Lichtstabilisatoren (UV-Absorber). Solche zu stabilisierenden Materialien können z.B. Oele, Fette, Wachse, Kosmetika, Biocide oder photographische Materialien sein. Von besonderem Interesse ist die Verwendung in polymeren Materialien, wie sie in Kunststoffen, Kautschuken, Anstrichstoffen oder Klebstoffen vorliegen. Beispiele für Polymere und andere Substrate, die auf diese Weise stabilisiert werden können, sind die folgenden:
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen hoher Dichte und hoher Molmasse (HDPE-HMW), Polyethylen hoher Dichte und ultrahoher Molmasse (HDPE-UHMW), Polyethylen mittlerer Dichte (MDPE), Polyethylen niederer Dichte (LDPE), lineares Polyethylen niederer Dichte (LLDPE), verzweigtes Polyethylen niederer Dichte (VLDPE).
   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly-(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes und bromiertes Copolymer aus Isobutylen-Isopren (Halobutylkautschuk), chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide, Polyetherimide, Polyesterimide, Polyhydantoine und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z.B. von Epoxyacrylaten, Urethan-acrylaten oder Polyester-acrylaten.
25. Alkydharze, Polyesterharze und Acrylatharze, die mit Melaminharzen, Harnstoffharzen, Isocyanaten, Isocyanuraten, Polyisocyanaten oder Epoxidharzen vernetzt sind.
26. Vernetzte Epoxidharze, die sich von aliphatischen, cycloaliphatischen, heterocyclischen oder aromatischen Glycidylverbindungen ableiten, z.B. Produkte von Bisphenol-A-diglycidylethern, Bisphenol-F-diglycidylethern, die mittels üblichen Härtern wie z.B. Anhydriden oder Aminen mit oder ohne Beschleunigern vernetzt werden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO, PBT/PC/ABS oder PBT/PET/PC.

Die Erfindung betrifft auch ein Verfahren zum Stabilisieren von organischem Material gegen Schädigung durch Licht, Sauerstoff und/oder Hitze, dadurch gekennzeichnet, daß man diesem als Stabilisator eine Verbindung der Formel I zusetzt sowie die Verwendung von Verbindungen der Formel I zum Stabilisieren von organischem Material.

Die Menge des zu verwendenden Stabilisators richtet sich nach dem zu stabilisierenden organischen Material und der beabsichtigten Verwendung des stabilisierten Materials. Im allgemeinen enthält die erfindungsgemäße Zusammensetzung auf 100 Gew.-Teile der Komponente A 0,01 bis 15 Gew.-Teile, insbesondere 0,05-10 Gew.-Teile, vor allem 0,1-5 Gew.-Teile einer Verbindung der Formel I (Komponente B).

Der Stabilisator (Komponente B) kann auch ein Gemisch sein von zwei oder mehr Verbindungen der Formeln I oder Ia. Die erfindungsgemäßen Zusammensetzungen können außer dem Stabilisator der Formel I noch andere Stabilisatoren oder sonstige Zusätze enthalten, wie z.B. Antioxidantien, weitere Lichtschutzmittel, Metalldesaktivatoren, Phosphite oder Phosphonite. Beispiele hierfür sind die folgenden Stabilisatoren:

### 1. Antioxidantien

1.1. Alkylierte Monophenole, z.B. 2,6-Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole wie z.B. 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis(3,5-di-tert-butyl-4-hydroxyphenyl)adipat.

1.4. Tocopherole, z.B. α-Tocopherol, β-Tocopherol, γ-Tocopherol, δ-Tocopherol und Mischungen davon (Vitamin E).

1.5. Hydroxylierte Thiodiphenylether, z.B. 2,2'-Thio-bis(6-tert-butyl-4-methylphenol), 2,2'-Thio-bis(4-octylphenol), 4,4'-Thio-bis(6-tert-butyl-3-methylphenol),4,4'-Thio-bis-(6-tert-butyl-2-methylphenol), 4,4'-Thio-bis(3,6-di-sec.-amylphenol), 4,4'-Bis(2,6-dimethyl-4-hydroxyphenyl)-disulfid.

1.6. Alkyliden-Bisphenole, z.B. 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis-(4,6-di-tert-butylphenol), 2,2'-Ethyliden-bis(6-tert-butyl-4-isobutylphenol), 2,2'-Methylen-bis[6-(α-methylbenzyl)-4-nonylphenol], 2,2'-Methylen-bis[6-(α,α-dimethylbenzyl)-4-nonylphenol], 4,4'-Methylen-bis(2,6-di-tert-butylphenol), 4,4'-Methylen-bis(6-tert-butyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1,1,3-Tris(5-tert-butyl-4-hydroxy-2-methylphenyl)-butan, 1,1 -Bis(5-tert-butyl-4-hydroxy-2-methyl-phenyl)-3-n-dodecylmercaptobutan, Ethylenglycol-bis[3,3-bis(3'-tert-butyl-4'-hydroxyphenyl)-butyrat], Bis(3-tert-butyl-4-hydroxy-5-methyl-phenyl)-dicyclopentadien, Bis[2-(3'-tert-butyl-2'-hydroxy-5'-methyl-benzyl)-6-tert-butyl-4-methyl-phenyl]-terephthalat, 1,1-Bis(3,5-dimethyl-2-hydroxyphenyl)-butan, 2,2-Bis(3,5-di-tert-butyl-4-hydroxyphenyl)-propan, 2,2-Bis(5-tert-butyl-4-hydroxy-2-methylphenyl)-4-n-dodecylmercapto-butan, 1,1,5,5-Tetra-(5-tert-butyl-4-hydroxy-2-methylphenyl)-pentan.

1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tridecyl-4-hydroxy-3,5-di-tert-butylbenzyl-mercaptoacetat, Tris(3,5-di-tert-butyl-4-hydroxybenzyl)-amin, Bis(4-tert-butyl-3-hydroxy-2,6-dimethylbenzyl)-dithioterephthalat, Bis(3,5-di-tert-butyl-4-hydroxybenzyl)-sulfid, Isooctyl-3,5-di-tert-butyl-4-hydroxybenzyl-mercaptoacetat.

1.8. Hydroxybenzylierte Malonate, z.B. Dioctadecyl-2,2-bis(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis(3,5-di-tert-butyl-4-hydroxybenzyl)-malonat, Di-[4-(1,1,3,3-tetramethylbutyl)-phenyl]-2,2-bis(3,5-di-tert-butyl-4-hydroxybenzyl)-malonat.

1.9. Hydroxybenzyl-Aromaten, z.B. 1,3,5-Tris(3,5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol, 2,4,6-Tris(3,5-di-tert-butyl-4-hydroxybenzyl)-phenol.

1.10. Triazinverbindungen, z.B. 2,4-Bis-octylmercapto-6-(3,5-di-tert-butyl-4-hydroxyanilino)-1,3,5-triazin, 2-Octylmercapto-4,6-bis(3,5-di-tert-butyl-4-hydroxyanilino)- 1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris(3,5-di-tert-butyl-4-hydroxybenzyl)-isocyanurat, 1,3,5-Tris(4-tert-butyl-3-hydroxy-2,6-dimethylbenzyl)-isocyanurat, 2,4,6-Tris(3,5-di-tert-butyl-4-hydroxyphenylethyl)-1,3,5-triazin, 1,3,5-Tris(3,5-di-tert-butyl-4-hydroxyphenylpropionyl)-hexahydro-1,3,5-triazin, 1,3,5-Tris(3,5-dicyclohexyl-4-hydroxybenzyl)-isocyanurat.

1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.13. Ester der β-(3,5-Di-tert-butyl-4-hydroxyphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl- 1-phospha-2,6,7-trioxabicyclo-[2.2.2]-octan.

1.14. Ester der β-(5-tert-Butyl-4-hydroxy-3-methylphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der β-(3,5-Dicyclohexyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.17. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis(3,5-di-tert-butyl-4-hydroxyphenylpropionyl)-trimethylendiamin, N,N'-Bis(3,5-di-tert-butyl-4-hydroxyphenylpropionyl)-hydrazin.

1.18. Ascorbinsäure (Vitamin C).

1.19. Aminische Antioxidantien, wie z.B. N,N'-Di-isopropyl-p-phenylendiamin, N,N'-Di-sec-butyl-p-phenylendiamin, N,N'-Bis(1,4-dimethyl-pentyl )-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-(4-tert-Octylphenyl)-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylaminophenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/Isohexyl-diphenylaminen, Gemische aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.

### 2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl- 2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis(α,α-dimethylbenzyl)-2'-hydroxyphenyl)-benzotriazol, Mischung aus 2-(3'-tert-Butyl-2'-hydroxy-5'-(2-octyloxycarbonylethyl)phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; 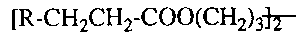 mit R = 3'-tert-Butyl-4'-hydroxy-5'-2H-benzotriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis(2,2,6,6-tetramethyl-piperidin-4-yl)-sebacat, Bis(2,2,6,6-tetramethyl-piperidin-4-yl)-succinat, Bis(1,2,2,6,6-pentamethylpiperidin-4-yl)-sebacat, Bis(1-octyloxy-2,2,6,6-tetramethylpiperidin-4-yl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethyl-4-piperidyl)-1,2,3,4-butantetraoat, 1,1'-(1,2-Ethandiyl)-bis(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis(1-octyloxy-2,2,6,6-tetramethylpiperidyl)-sebacat, Bis(1-octyloxy-2,2,6,6-tetramethylpiperidyl)-succinat, Kondensationsprodukt aus N,N'-Bis(2,2,6,6-tetramethyl-4-piperidyl)-hexamethylendiamin und 4-Morpholino-2,6-dichlor- 1,3,5-triazin, Kondensationsprodukt aus 2-Chlor-4,6-di-(4-n-butylamino-2,2,6,6-tetramethylpiperidyl)-1,3,5-triazin und 1,2-Bis(3-aminopropylamino)-ethan, Kondensationsprodukt aus 2-Chlor-4,6-di-(4-n-butylamino- 1,2,2,6,6-pentamethylpiperidyl)-1,3,5-triazin und 1,2-Bis(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, Gemisch von 4-Hexadecyloxy- und 4-Stearyloxy-2,2,6,6-tetramethylpiperidin, Kondenstionsprodukt aus N,N'-Bis(2,2,6,6-tetramethyl-4-piperidyl)-hexamethylendiamin und 4-Cyclohexylamino-2,6-dichlor-1,3,5-triazin, Kondensationsprodukt aus 1,2-Bis(3-aminopropylamino)-ethan und 2,4,6-trichlor-1,3,5-triazin sowie 4-Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxo-spiro[4,5]decan, Umsetzungsprodukt von 7,7,9,9-Tetramethyl-2-cycloundecyl-1-oxa-3,8-diaza-4-oxospiro[4,5]decan und Epichlorhydrin.

2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'-di-tert-butyl-oxanilid, 2-Ethoxy-2'-ethyl-oxanilid, N,N'-Bis(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis-(2,4-dimethylphenyl)-1,3,5-triazin, 2-[2-Hydroxy-4-(2-hydroxy-3-butyloxy-propyloxy)phenyl]-4,6-bis(2,4-dimethylphenyl)-1,3,5-triazin, 2-[2-Hydroxy-4-(2-hydroxy-3-octyloxy-propyloxy)phenyl]-4,6-bis(2,4-dimethylphenyl)-1,3,5-triazin, 2-[4-(dodecyloxy/tridecyloxy-2-hydroxypropoxy)-2-hydroxy-phenyl]-4,6-bis(2,4-dimethylphenyl)-1,3,5-triazin, 2-[2-Hydroxy-4-(2-hydroxy-3-dodecyloxy-propoxy)phenyl]-4,6-bis(2,4-dimethylphenyl)-1,3,5-triazin, 2-(2-Hydroxy-4-hexyloxy)phenyl-4'6-diphenyl- 1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy)phenyl]-1,3,5-triazin, 2-(2-Hydroxyphenyl)-4-(4-methoxyphenyl)-6-phenyl-1,3,5-triazin.

3. Metalldesaktivatoren, wie z.B. N,N'-Diphenyloxalsäurediamid, N-Salicylal-N'-salicyloylhydrazin, N,N'-Bis(salicyloyl)-hydrazin, N,N'-Bis(3,5-di-tert-butyl-4-hydroxyphenylpropionyl)-hydrazin, 3-Salicyloylamino-1,2,4-triazol, Bis(benzyliden)-oxalsäuredihydrazid, Oxanilid, Isophthalsäure-dihydrazid, Sebacinsäure-bis-phenylhydrazid, N,N'-Di-acetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris(nonylphenyl)-phosphit, Trilaurylphosphit, Trioctadecylphosphit, Distearyl-pentaerythritdiphosphit, Tris(2,4-di-tert-butylphenyl)-phosphit, Diisodecylpentaerythrit-diphosphit, Bis(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis(2,4-di-tert-butylphenyl)-4,4'-biphenylen-diphosphonit, 6-Isooctyloxy-2,4,8,10-tetra-tert-butyl- 12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis(2,4-di-tert-butyl-6-methylphenyl)-methylphosphit, Bis-(2,4-di-tert-butyl-6-methylphenyl)-ethylphosphit.

5. Hydroxylamine wie z.B. N,N-Dibenzylhydroxylamin, N,N-diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecylhydroxylamin, N-Hexadecyl-N-octadecylhydroxylamin, N-Heptadecyl-N-octadecylhydroxylamin, N,N-Dialkylhydroxylamin aus hydrierten Talgfettaminen.

6. Nitrone wie z.B. N-Benzyl-alpha-phenyl-nitron, N-Ethyl-alpha-methyl-nitron, N-Octyl-alpha-heptyl-nitron, N-Lauryl-alpha-undecyl-nitron, N-Tetradecyl-alpha-tridecylnitron, N-Hexadecyl-alpha-pentadecyl-nitron, N-Octadecyl-alpha-heptadecyl-nitron, N-Hexadecyl-alpha-heptadecyl-nitron, N-Ocatadecyl-alpha-pentadecyl-nitron, N-Heptadecyl-alpha-heptadecyl-nitron, N-Octadecyl-alpha-hexadecyl-nitron, Nitrone abgeleitet von N,N-Dialkylhydroxylaminen hergestellt aus hydrierten Talgfettaminen.

7. Thiosynergisten wie z.B. Thiodipropionsäure-di-laurylester oder Thiodipropionsäure-di-stearylester.

8. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis(β-dodecylmercapto)-propionat.

9. Polyamidstabilisatoren, wie z.B. Kupfersalze in Kombination mit Jodiden und/oder Phosphorverbindungen und Salze des zweiwertigen Mangans.

10. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearal, Mg-Behenat, Mg-Stearat, Na-Ricinoleat, K-Palmitat, Antimonbrenzcatechinat oder Zinnbrenzcatechinat.

11. Nukleierungsmittel, wie z.B. anorganische Stoffe wie z.B. Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z.B. 4-tert-Butylbenzoesäure, Adipinsäure, Diphenylessigsäure, Natriumsuccinat oder Natriumbenzoat; polymere Verbindungen wie z.B. ionische Copolymerisate ("Ionomere").

12. Füllstoffe und Verstärkungsmittel, wie z.B. Calciumcarbonat, Silikate, Glasfasern, Glaskugeln, Asbest, Talk, Kaolin, Glimmer, Bariumsulfat, Metalloxide und -hydroxide, Ruß, Graphit, Holzmehl und Mehle oder Fasern anderer Naturprodukte, synthetische Fasern.

13. Sonstige Zusätze, wie z.B. Weichmacher, Gleitmittel, Emulgatoren, Pigmente, Rheologieadditive, Katalysatoren, Verlaufshilfsmittel, Optische Aufheller, Flammschutzmittel, Antistatika, Treibmittel.

14. Benzofuranone bzw. Indolinone, wie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 oder EP-A-0 591 102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis[5,7-di-tert-butyl-3-(4-[2-hydroxyethoxy]-phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Die Art und Menge der zugesetzten weiteren Stabilisatoren wird von der Art des zu stabilisierenden Substrates und dessen Verwendungszweck bestimmt; häufig werden 0,1 bis 5 Gew.-% bezogen auf das zu stabilisierende Polymer verwendet.

Die erfindungsgemäße Zusammensetzung enthält vorzugsweise neben den Komponenten A, und B als Komponente C ein Lichtschutzmittel vom Typ der sterisch gehinderten Amine, der 2-(2-Hydroxyphenyl)-1,3,5-triazine und/oder der 2-Hydroxyphenyl-2H-benztriazole. Beispiele für solche Costabilisatoren finden sich in obiger Liste unter den Punkten 2,1,2.6 und 2.8.

Zur Erzielung maximaler Lichtbeständigkeit ist vor allem der Zusatz von sterisch gehinderten Aminen, wie sie in der genannten Liste unter 2.6 aufgeführt sind, von Interesse. Eine Zusammensetzung, welche neben den Komponenten A, und B als Komponente C ein Lichtschutzmittel vom Typ der sterisch gehinderten Amine (HALS) enthält, ist daher von besonderem Interesse.

Vorzugsweise handelt es sich dabei um ein 2,2,6,6-Tetraalkylpiperidinderivat, das mindestens eine Gruppe der Formel 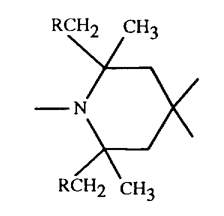 enthält, worin R Wasserstoff oder Methyl, insbesondere Wasserstoff, ist.

Besonders vorteilhaft lassen sich die erfindungsgemäßen Verbindungen der Formel I einsetzen in Zusammensetzungen, die als Komponente A ein synthetisches organisches Polymer, insbesondere ein thermoplastisches Polymer, ein Bindemittel für Überzüge wie beispielsweise Lacke, oder ein photographisches Material enthalten.

Wenn es sich bei Komponente A um ein Bindemittel für Überzüge handelt, ist der Zusatz der oben beschriebenen Komponente C zur erfindungsgemäßen Zusammensetzung besonders empfehlenswert.

Als thermoplastische Polymere kommen beispielsweise Polyolefine sowie Polymere in Frage, die Heteroatome in der Hauptkette enthalten. Bevorzugt sind auch solche Zusammensetzungen, worin Komponente A ein photographisches Material oder ein thermoplastisches Polymer ist, das Stickstoff, Sauerstoff und/oder Schwefel, insbesondere Stickstoff oder Sauerstoff, in der Hauptkette enthält.

Polymere, die Heteroatome in der Hauptkette enthalten, sind vor allem O, S und/oder N enthaltende Polymere. Beispiele für solche Polymere sind die folgenden Klassen von thermoplastischen Polymeren:
1. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
2. Polyphenylenoxide und -sulfide und deren Mischungen mit Styrolpolymeren oder Polyamiden.
3. Polyamide und Copolyamide, z.B. solche, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einen Elastomer als Modifikator, z.B.
   Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamid-systeme").
4. Polyharnstoffe, Polyimide, Polyamid-imide und Polybenzimidazole.
5. Polyester, z.B. solche, die sich von Dicarbonsäuren und Dialkoholen und/oder von Hydroxycarbonsäuren oder den entsprechenden Lactonen ableiten, wie Polyethylenterephthalat, Polybutylenterephthalat,
   Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
6. Polycarbonate und Polyestercarbonate, insbesondere aromatische Polycarbonate, wie z.B. solche basierend auf 2,2-Bis(4-hydroxyphenyl)-propan oder 1,1-Bis(4-hydroxyphenyl)-cyclohexan.
7. Polysulfone, Polyethersulfone und Polyetherketone, insbesondere aromatische Polymere dieser Klasse.
8. Mischungen (Polyblends) solcher Polymerer untereinander oder mit anderen Polymeren, z.B. mit Polyolefinen, Polyacrylaten, Polydienen oder anderen Elastomeren als Schlagzähmodifikatoren.

Bevorzugt sind darunter die Polycarbonate, Polyester, Polyamide, Polyacetale, Polyphenylenoxide und Polyphenylensulfide, insbesondere aber die Polycarbonate.

Von Interesse sind auch Zusammensetzungen, worin Komponente (A) ein Polyolefin ist, beispielsweise Polyethylen oder Polypropylen.

Die Einarbeitung in das zu stabilisierende organische Material, beispielsweise in die synthetischen organischen, insbesondere thermoplastischen Polymere, kann durch Zusatz der erfindungsgemäßen Verbindungen und gegebenenfalls weiterer Additive nach den in der Technik üblichen Methoden erfolgen. Die Einarbeitung kann zweckmäßig vor oder während der Formgebung, beispielsweise durch Mischen der pulverförmigen Komponenten oder durch Zusatz des Stabilisators zur Schmelze oder Lösung des Polymeren, oder durch Aufbringen der gelösten oder dispergierten Verbindungen auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der erfindungsgemäßen Verbindungen in Polymere besteht in deren Zugabe vor oder während der Polymerisation der entsprechenden Monomeren bzw. vor der Vernetzung.

Die erfindungsgemäßen Verbindungen oder Mischungen davon können auch in Form eines Masterbatches, der diese Verbindungen beispielsweise in einer Konzentration von 2,5 bis 25 Gew.% enthält, den zu stabilisierenden Kunststoffen zugesetzt werden.

Zweckmäßig kann die Einarbeitung der erfindungsgemäßen Verbindungen nach folgenden Methoden erfolgen:
- als Emulsion oder Dispersion (z.B. zu Latices oder Emulsionspolymeren)
- als Trockenmischung während des Vermischens von Zusatzkomponenten oder Polymermischungen
- durch direktes Zugeben in die Verarbeitungsapparatur (z.B. Extruder, Innenmischer usw.)
- als Lösung oder Schmelze.

Die so erhaltenen stabilisierten Polymerzusammensetzungen können nach den üblichen Methoden, wie z.B. durch Heißpressen, Spinnen, Extrudieren oder Spritzgießen, in geformte Gegenstände überführt werden, wie z.B. in Fasern, Folien, Bändchen, Platten, Stegplatten, Gefäße, Rohre und sonstige Profile.

Die Erfindung betrifft daher weiterhin die Verwendung der erfindungsgemäßen Polymerzusammensetzung zur Herstellung eines geformten Gegenstandes.

Von Interesse ist auch die Verwendung in Mehrschichtsystemen. Hierbei wird eine erfindungsgemäße Polymerenzusammensetzung mit einem relativ hohen Gehalt an Stabilisator der Formel I, beispielsweise 5 - 15 Gew.-%, in dünner Schicht (10-100 µm) auf einen geformten Gegenstand aus einem Polymer, das wenig oder keinen Stabilisator der Formel I enthält, aufgebracht. Das Aufbringen kann zugleich mit der Formgebung des Grundkörpers geschehen, z.B. durch sogenannte Coextrusion. Das Aufbringen kann aber auch auf den fertig geformten Grundkörper geschehen, z.B. durch Lamination mit einem Film oder durch Beschichtung mit einer Lösung. Die äußere Schicht bzw. die äußeren Schichten des fertigen Gegenstandes haben die Funktion eines UV-Filters, der das Innere des Gegenstandes gegen UV-Licht schützt. Die äußere Schicht enthält vorzugsweise 5-15 Gew.%, insbesondere 5-10 Gew.%, mindestens eines Stabilisators der Formel I.

Ebenfalls von besonderem Interesse ist die Verwendung der erfindungsgemäßen Verbindungen der Formel I als Stabilisatoren für Überzüge, beispielsweise für Lacke. Gegenstand der Erfindung sind daher auch solche Zusammensetzungen, deren Komponente A ein filmbildendes Bindemittel ist.

Die Verwendung in Mehrschichtsystemen ist möglich, wobei die Konzentration der Verbindung der Formel I (Komponente B) in der Deckschicht höher sein kann, beispielsweise 1 bis 15 Gew.-Teile B, vor allem 3-10 Gew.-Teile B auf 100 Gew.-Teile festes Bindemittel A.

Als Bindemittel (Komponente A) kommen prinzipiell alle in der Technik gebräuchlichen in Betracht, beispielsweise solche, wie sie beschrieben sind in Ullmann's Encyclopedia of Industrial Chemistry, 5. Ed., Vol. A18, pp. 368-426, VCH, Weinheim 1991. Allgemein handelt es sich um ein filmbildendes Bindemittel basierend auf einem thermoplastischen oder thermohärtbaren Harz, vorwiegend auf einem thermohärtbaren Harz. Beispiele hierfür sind Alkyd-, Acryl-, Polyester-, Phenol-, Melamin-, Epoxid-, Polyurethanharze und deren Gemische.

Komponente A kann ein kalt aushärtbares oder ein heiß aushärtbares Bindemittel sein, wobei die Zugabe eines Härtungskatalysators vorteilhaft sein kann. Geeignete Katalysatoren, die die Aushärtung des Bindemittels beschleunigen, sind beispielsweise beschrieben in Ullmann's Encyclopedia of Industrial Chemistry, Vol. A 18, S. 469, VCH Verlagsgesellschaft, Weinheim 1991.

Bevorzugt sind Überzugsmittel, worin die Komponente A ein Bindemittel aus einem funktionellen Acrylatharz und einem Vernetzer ist.

Beispiele von Überzugsmitteln mit speziellen Bindemitteln sind:
1. Lacke auf Basis von kalt- oder heiß-vernetzbaren Alkyd-, Acrylat-, Polyester-, Epoxid- oder Melaminharzen oder Mischungen solcher Harze, gegebenenfalls mit Zusatz eines Härtungskatalysators;
2. Zweikomponenten-Polyurethanlacke auf Basis von hydroxylgruppenhaltigen Acrylat-, Polyester- oder Polyetherharzen und aliphatischen oder aromatischen Isocyanaten, Isocyanuraten oder Polyisocyanaten;
3. Einkomponenten-Polyurethanlacke auf Basis von blockierten Isocyanaten, Isocyanuraten oder Polyisocyanaten, die während des Einbrennens deblockiert werden;
4. Zweikomponentenlacke auf Basis von (Poly)ketiminen und aliphatischen oder aromatischen Isocyanaten, Isocyanuraten oder Polyisocyanaten;
5. Zweikomponentenlacke auf Basis von (Poly)ketiminen und einem ungesättigten Acrylatharz oder einem Polyacetoacetatharz oder einem Methacrylamidoglykolatmethylester;
6. Zweikomponentenlacke auf Basis von carboxyl- oder aminogruppenhaltigen Polyacrylaten und Polyepoxiden;
7. Zweikomponentenlacke auf Basis von anhydridgruppenhaltigen Acrylatharzen und einer Polyhydroxy- oder Polyaminokomponente;
8. Zweikomponentenlacke auf Basis von (Poly)oxazolinen und anhydridgruppenhaltigen Acrylatharzen oder ungesättigten Acrylatharzen oder aliphatischen oder aromatischen Isocyanaten, Isocyanuraten oder Polyisocyanaten;
9. Zweikomponentenlacke auf Basis von ungesättigten Polyacrylaten und Polymalonaten;
10. thermoplastische Polyacrylatlacke auf Basis von thermoplastischen Acrylatharzen oder fremdvernetzenden Acrylatharzen in Kombination mit veretherten Melaminharzen;
11. Lacksysteme auf Basis von siloxanmodifizierten oder fluormodifizierten Acrylatharzen.

Bei den erfindungsgemäßen Überzugsmitteln kann es sich auch um strahlenhärtbare Überzugsmittel handeln. In diesem Fall besteht das Bindemittel im wesentlichen aus monomeren oder oligomeren Verbindungen mit ethylenisch ungesättigten Bindungen, die nach der Applikation durch UV- oder Elektronenstrahlung gehärtet, d.h. in eine vernetzte, hochmolekulare Form überführt werden. Entsprechende Systeme sind in der oben genannten Publikation, Ullmann's Encyclopedia of Industrial Chemistry, 5. Ed., Vol. A18, Seiten 451-453, beschrieben. In strahlenhärtbaren Überzugsmitteln können die Verbindungen der Formel I auch ohne Zusatz von sterisch gehinderten Aminen eingesetzt werden.

Das Überzugsmittel kann außer den Komponenten A, B und gegebenenfalls C weitere Komponenten enthalten, z.B. Lösungsmittel, Pigmente, Farbstoffe, Weichmacher, Stabilisatoren, Thixotropiemittel, Trocknungskatalysatoren oder/und Verlaufhilfsmittel. Mögliche Komponenten sind beispielsweise solche, wie sie in Ullmann's Encyclopedia of Industrial Chemistry, 5. Ed., Vol. A18, pp. 429-471, VCH, Weinheim 1991 beschrieben sind.

Mögliche Trocknungskatalysatoren beziehungsweise Härtungskatalysatoren sind beispielsweise organische Metallverbindungen, Amine, aminogruppenhaltige Harze oder/und Phosphine. Organische Metallverbindungen sind z.B. Metallcarboxylate, insbesondere solche der Metalle Pb, Mn, Co, Zn, Zr oder Cu, oder Metallchelate, insbesondere solche der Metalle Al, Ti oder Zr oder Organometallverbindungen wie z.B. Organozinnverbindungen.

Beispiele für Metallcarboxylate sind die Stearate von Pb, Mn oder Zn, die Octoate von Co, Zn oder Cu, die Naphthenate von Mn und Co oder die entsprechenden Linoleate, Resinate oder Tallate.

Beispiele für Metallchelate sind die Aluminium-, Titan- oder Zirkonium-Chelate von Acetylaceton, Ethylacetylacetat, Salicylaldehyd, Salicylaldoxim, o-Hydroxyacetophenon oder Ethyl-trifluoracetylacetat und die Alkoxide dieser Metalle.

Beispiele für Organozinnverbindungen sind Dibutylzinnoxid, Dibutylzinn-dilaurat oder Dibutylzinn-dioctoat.

Beispiele für Amine sind vor allem tertiäre Amine, wie z.B. Tributylamin, Triethanolamin, N-Methyl-diethanolamin, N-Dimethylethanolamin, N-Ethylmorpholin, N-Methylmorpholin oder Diazabicyclooctan (Triethylendiamin) sowie deren Salze. Weitere Beispiele sind quaternäre Ammoniumsalze, wie z.B. Trimethylbenzylammoniumchlorid.

Aminogruppenhaltige Harze sind gleichzeitig Bindemittel und Härtungskatalysator. Beispiel hierfür sind aminogruppenhaltige Acrylat-copolymere.

Als Härtungskatalysator können auch Phosphine verwendet werden, wie z.B. Triphenylphosphin.

Die erfindungsgemäßen Überzugsmittel können auf beliebige Substrate aufgebracht werden, beispielsweise auf Metall, Holz, Kunststoff oder keramische Materialien. Vorzugsweise werden sie beim Lackieren von Automobilen als Decklack verwendet. Besteht der Decklack aus zwei Schichten, wovon die untere Schicht pigmentiert ist und die obere Schicht nicht pigmentiert ist, so kann das erfindungsgemäße Überzugsmittel für die obere oder die untere Schicht oder für beide Schichten verwendet werden, vorzugsweise jedoch für die obere Schicht

Die erfindungsgemäßen Überzugsmittel können auf die Substrate nach den üblichen Verfahren aufgebracht werden, beispielsweise durch Streichen, Besprühen, Gießen, Tauchen oder Elektrophorese; s.a. Ullmann's Encyclopedia of Industrial Chemistry, 5. Ed., Vol. A18, pp. 491-500.

Die Härtung der Überzüge kann - je nach dem Bindemittelsystem - bei Raumtemperatur oder durch Erwärmen erfolgen. Vorzugsweise härtet man die Überzüge bei 50-150°C, Pulverlacke auch bei höheren Temperaturen.

Die erfindungsgemäß erhaltenen Überzüge weisen eine hervorragende Beständigkeit gegen schädigende Einflüsse von Licht, Sauerstoff und Wärme auf; insbesondere ist auf die gute Licht- und Witterungsbeständigkeit der so erhaltenen Überzüge, beispielsweise Lacke, hinzuweisen.

Die Erfindung betrifft daher auch einen Überzug, besonders ein Lack, der durch einen Anteil der erfindungsgemäßen Verbindung der Formel I gegen schädigende Einflüsse von Licht, Sauerstoff und Wärme stabilisiert ist. Der Lack ist vorzugsweise ein Decklack für Automobile.

Von Interesse sind auch solche Bindemittel, in die eine Verbindung der Formel I durch Copolymerisation oder Copolykondensation eingebaut ist. Hierzu eignen sich solche Verbindungen der Formel I, in denen der Rest R⁷ eine copolymerisierbare ethylenisch ungesättigte Gruppe oder zur Copolykondensation geeignete funktionelle Gruppe enthält. In diesem Fall kann das erfindungsgemäße Überzugsmittel auch nur aus einer Komponente, nämlich dem Bindemittel mit eingebautem Stabilisator bestehen.

Meistens enthalten die Überzugsmittel ein organisches Lösungsmittel oder Lösungsmittelgemisch, in dem das Bindemittel löslich ist. Das Überzugsmittel kann aber auch eine wässrige Lösung oder Dispersion sein. Das Vehikel kann auch ein Gemisch eines organischen Lösungmittels und Wasser sein. Das Überzugsmittel kann auch ein feststoffreicher Lack (high solids Lack) sein oder kann lösungsmittelfrei (Pulverlack) sein.

Die Pigmente können anorganische, organische oder metallische Pigmente sein. Vorzugsweise enthalten die erfindungsgemäßen Überzugsmittel keine Pigmente und werden als Klarlack verwendet.

Ebenfalls bevorzugt ist der Einsatz des Überzugsmittels als Decklack für Anwendungen in der Automobilindustrie, besonders als pigmentierte oder unpigmentierte Deckschicht des Lackes. Die Verwendung für darunter liegende Schichten ist jedoch auch möglich.

Ein weiterer Gegenstand der vorliegenden Erfindung ist die Verwendung einer Verbindung der Formel I in photographischen Materialien als Stabilisator gegen Lichtschädigung, insbesondere UV-Lichtschädigung, und das photographische Material enthaltend eine Verbindung der Formel I, wobei auch Mischungen von Verbindungen der Formel I in Frage kommen.

Die erfindungsgemäßen Verbindungen können für alle Arten photosensitiven Materials verwendet werden. Beispielsweise können sie für Farbpapier, Farbumkehrpapier, Direkt-Positiv-Farbmaterial, Farbnegativfilm, Farbpositivfilm, Farbumkehrfilm und weitere eingesetzt werden. Unter anderem werden sie bevorzugt für photosensitives Farbmaterial, welches ein Umkehrsubstrat enthält oder welches Positive bildet, verwendet.

Ferner können die erfindungsgemäßen Verbindungen mit weiteren UV-Absorbern, insbesondere mit in wässriger Gelatine dispergierbaren, kombiniert werden, beispielsweise mit Hydroxyphenylbenztriazolen (vgl. beispielsweise US-A-4,853,471, US-A-4,973,702, US-A-4,921,966 und US-A-4,973,701), Benzophenonen, Oxaniliden, Cyanoacrylaten, Salicylsäureestern, Acrylnitrilen oder Thiazolinen. Dabei ist es vorteilhaft, diese weiteren in Öl gelösten UV-Absorber in anderen Schichten im photographischen Material einzusetzen als die erfindungsgemäßen UV-Absorber. Insbesondere lassen sich photographische Materialien ähnlich solchen, wie sie in US-A-4,518,686 beschrieben sind, mit gutem Erfolg stabilisieren.

Die Erfindung betrifft daher auch ein photographisches Material enthaltend auf einem Träger eine blauempfindliche, eine grünempfindliche und/oder eine rotempfindliche Silberhalogenidemulsionsschicht sowie gegebenenfalls eine Protektionsschicht, wobei oberhalb der obersten Silberhalogenidemulsionsschicht, eine Schicht mit einem UV-Absorber angeordnet ist, dadurch gekennzeichnet, daß der UV-Absorber der Formel I entspricht.

In einer weiteren Ausführungsform enthält das erfindungsgemäße Material eine Schicht mit einem UV-Absorber der Formel I, welche zwischen der grün- und rotempfindlichen Silberhalogenidemulsionsschicht angeordnet ist, wobei oberhalb der obersten Silberhalogenidemulsionsschicht eine weitere Schicht mit einem UV-Absorber der Formel I angeordnet sein kann.

Gute Ergebnisse werden auch erzielt, wenn der UV-Absorber der Formel I zusätzlich in der rotempfindlichen Silberhalogenidemulsionsschicht enthalten ist.

Bevorzugt sind weiterhin photographische Materialien, welche eine Schicht enhaltend eine Verbindung der Formel I oberhalb der obersten Silberhalogenidemulsionsschicht und/oder zwischen der grün- und rotempfindlichen Silberhalogenidemulsionsschicht aufweisen, wobei zusätzlich ein öllöslicher UV-Absorber in einer Schicht enthalten ist, welche keinen UV-Absorber der Formel I enthält.

Des weiteren kann es vorteilhaft sein, wenn alle oder ein Teil der besagten Schichten, welche einen UV-Absorber enthalten können, einen UV-Absorber der Formel I und/oder einen weiteren in wässriger Gelatine dispergierbaren UV-Absorber aufweisen, wobei jedoch mindestens in einer Schicht ein UV-Absorber der Formel I enthalten sein muß.

Vorzugsweise enthält das erfindungsgemäße Material zwischen den Silberhalogenidemulsionsschichten Gelatinezwischenschichten.

Bevorzugt sind solche photographischen Materialien, welche als Silberhalogenid in der blauempfindlichen, grünempfindlichen und/oder rotempfindlichen Schicht Silberchloridbromid, welches mindestens zu 90 mol % aus Silberchlorid besteht, enthalten.

Desweiteren sind photographische Materialien bevorzugt, welche die Silberhalogenidemulsionsschichten in der Reihenfolge blauempfindliche, grünempfindliche und rotempfindliche Silberhalogenidemulsionsschicht enthalten.

Die erfindungsgemäßen photographischen Materialien bieten gegenüber Materialien mit Benztriazol-UV-Absorbern den Vorteil, daß die UV-Absorber der Formel I in einer vergleichsweise geringen Menge benötigt werden, um einen ausreichenden Schutz gegen UV-Strahlung zu gewährleisten. Dies bedeutet, daß die Dicke der Schichten, in die die UV-Absorber der Formel I eingelagert werden, sehr dünn sein können, was sich z.B. auf die Schärfe der mit diesem Material hergestellten Abbildungen positiv auswirkt.

Im erfindungsgemäßen Material verwendbare Gelbkuppler sind vorzugsweise Verbindungen der Formel A 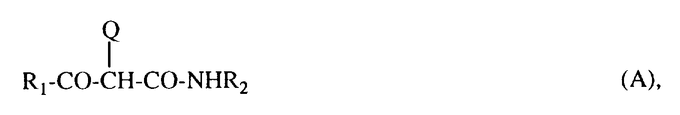 worin R₁ Alkyl oder Aryl ist, R₂ Aryl ist und Q Wasserstoff oder eine Gruppe ist, die durch Reaktion mit dem oxidierten Entwickler abgespaltet werden kann.

Eine Gruppe von Gelbkupplern sind solche Verbindungen der Formel A, in denen R₁ t-Butyl ist und R₂ eine Gruppe der Formel 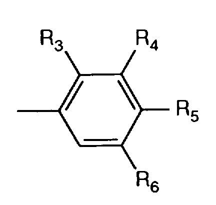 ist, worin R₃ Wasserstoff, Halogen, Alkyl oder Alkoxy bedeutet und R₄, R₅ und R₆ Wasserstoff, Halogen, Alkyl, Alkenyl, Alkoxy, Aryl, Carboxy, Alkoxycarbonyl, eine Carbamoylgruppe, eine Sulfon- oder Sulfamoylgruppe, eine Alkylsulfonaminogruppe, Acylaminogruppe, Ureidogruppe oder Aminogruppe bedeuten.

Vorzugsweise sind R₃ Chlor, R₄ und R₅ Wasserstoff und R₆ eine Acylaminogruppe. Hierzu gehören auch die Verbindungen der Formel 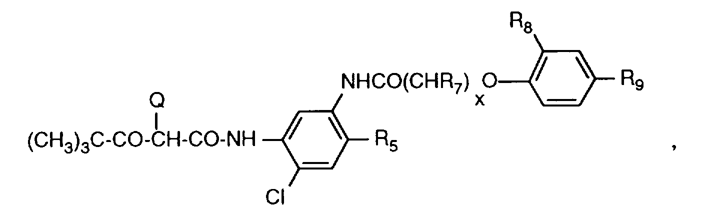 worin x O-4 ist, R₇ Wasserstoff oder Alkyl bedeutet und R₈ und R₉ Alkyl sind.

Eine andere Gruppe von Gelbkupplern entspricht der Formel B 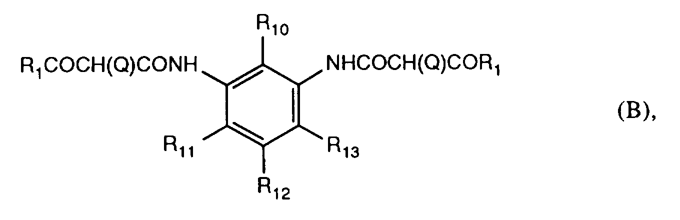 worin R₁₀ Wasserstoff, Halogen oder Alkoxy ist,
R₁₁, R₁₂ und R₁₃ Wasserstoff, Halogen, Alkyl, Alkenyl, Alkoxy, Aryl, Carboxyl, Alkoxycarbonyl, eine Carbamoylgruppe, eine Sulfongruppe, Sulfamoylgruppe, Sulfonamidogruppe, Acylaminogruppe, Ureidogruppe oder Aminogruppe bedeuten und R₁ und Q die oben angegebene Bedeutung haben.

Dazu gehören Verbindungen der Formel B, in denen R₁ t-Butyl ist, R₁₀ Chlor ist, R₁₁ und R₁₃ Wasserstoff sind und R₁₂ Alkoxycarbonyl ist.

In den Verbindungen der Formel A und B kann die Abgangsgruppe Q Wasserstoff sein oder sie ist eine heterocyclische Gruppe 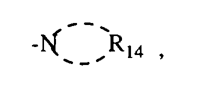 worin R₁₄ eine organische zweiwertige Gruppe ist, die den Ring zu einem 4-7-gliedrigen Ring ergänzt, oder Q ist eine Gruppe -OR₁₅, worin R₁₅ Alkyl, Aryl, Acyl oder ein heterocyclischer Rest ist.

Die Gelbkuppler werden üblicherweise in einer Menge von 0,05-2 Mol und vorzugsweise 0,1-1 Mol pro Mol Silberhalogenid verwendet.

Magentakuppler können z.B. einfache 1-Aryl-5-pyrazolone sein oder mit 5-gliedrigen Heteroringen kondensierte Pyrazolderivate wie z.B. Imidazopyrazole, Pyrazolopyrazole, Pyrazolotriazole oder Pyrazolotetrazole.

Eine Gruppe von Magentakupplern sind 5-Pyrazolone der Formel C, 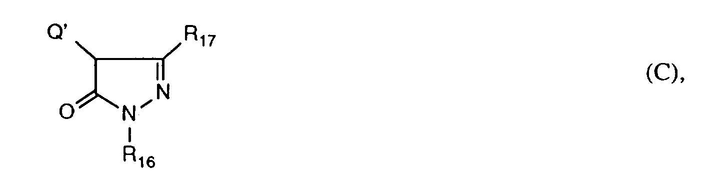 wie sie in der Britischen Patentschrift 2,003,473 beschrieben sind. Darin ist R₁₆ Wasserstoff, Alkyl, Aryl, Alkenyl oder eine heterocyclische Gruppe. R₁₇ ist Wasserstoff, Alkyl, Aryl, eine heterocyclische Gruppe, eine Estergruppe, Alkoxygruppe, Alkylthiogruppe, Carboxylgruppe, Arylaminogruppe, Acylaminogruppe, (Thio)-harnstoffgruppe, (Thio)-carbamoylgruppe, Guanidinogruppe oder Sulfonamidogruppe.

Bevorzugt ist R₁₇ eine Gruppe 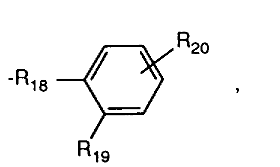 worin R₁₈ Imino, Acylamino oder Ureido ist, R₁₉ Wasserstoff, Halogen, Alkyl oder Alkoxy ist, R₂₀ Wasserstoff, Alkyl, Acylamino, Carbamoyl, Sulfamoyl, Sulfonamido, Alkoxycarbonyl, Acyloxy oder eine Urethangruppe ist.

Wenn Q' Wasserstoff ist, so ist der Magentakuppler tetraäquivalent in bezug auf das Silberhalogenid.

Typische Beispiele für diesen Typ von Magentakupplern sind Verbindungen der Formel 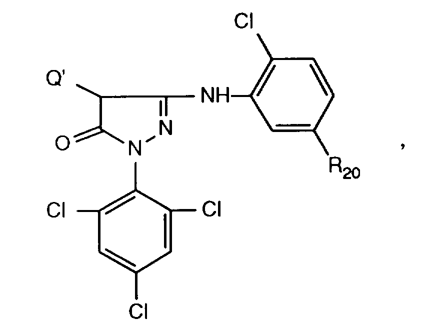 worin R₂₀ die oben genannten Bedeutungen hat, und Q', wie oben beschrieben, eine Abgangsgruppe ist. Diese Verbindungen liegen bevorzugt im erfindungsgemäßen Material vor.

Weitere Beispiele solcher tetraäquivalenter Magentakuppler sind zu finden in den US-A 2,983,608, 3,061,432, 3,062,653, 3,127,269, 3,152,896, 3,311,476, 3,419,391, 3,519,429, 3,558,319, 3,582,322, 3,615,506, 3,684,514, 3,834,908, 3,888,680, 3,891,445, 3,907,571, 3,928,044, 3,930,861, 3,930,866 und 3,933,500 und in JP-A-89/309,058.

Wenn Q' in Formel C nicht Wasserstoff ist sondern eine Gruppe, die bei der Reaktion mit dem oxidierten Entwickler eliminiert wird, so handelt es sich um einen diäquivalenten Magentakuppler. Q kann in diesem Fall z.B. Halogen oder eine über O, S oder N an den Pyrazolring gebundenen Gruppe sein. Solche diäquivalenten Kuppler ergeben eine höhere Farbdichte und sind reaktiver gegenüber dem oxidierten Entwickler als die entsprechenden tetraäquivalenten Magentakuppler.

Beispiele für diäquivalente Magentakuppler sind beschrieben in den US-A 3,006,579, 3,419,391, 3,311,476, 3,432,521, 3,214,437, 4,032,346, 3,701,783, 4,351,897, 3,227,554, in den EP-A-133,503, DE-A-2,944,601, JP-A-78/34044, 74/53435, 74/53436, 75/53372 und 75/122935.

Ueber ein zweiwertiges Q' können 2 Pyrazolonringe verknüpft werden und man erhält dann sogenannte Bis-Kuppler. Solche sind z.B. beschrieben in den US-A-2,632,702, US-A-2,618,864, GB-A-968,461, GB-A-786,859, JP-A-76/37646, 59/4086, 69/16110, 69/26589, 74/37854 und 74/29638. Bevorzugt ist Y eine O-Alkoxyarylthio-Gruppe.

Wie vorstehend erwähnt, können als Magentakuppler auch mit 5-gliedrigen Heterocyclen kondensierte Pyrazole - sogenannte Pyrazoloazole - verwendet werden. Deren Vorteile gegenüber einfachen Pyrazolen ist, daß sie Farben von größerer Formalin-Beständigkeit und reineren Absorptionsspektren aufweisen.

Magentakuppler vom Pyrazoloazoltyp, welche ebenfalls bevorzugt sind, können durch die Formeln

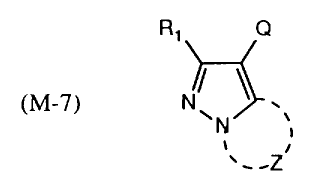

dargestellt werden, worin R₁ Wasserstoff oder ein Substituent ist, Z die zur Vervollständigung eines 5-gliedrigen Ringes mit 2 oder 3 Stickstoffatomen notwendigen nichtmetallischen Atome darstellt, wobei dieser Ring substituiert sein kann, und Q Wasserstoff oder eine Abgangsgruppe ist.

Bevorzugt hiervon sind Magentakuppler der Formeln 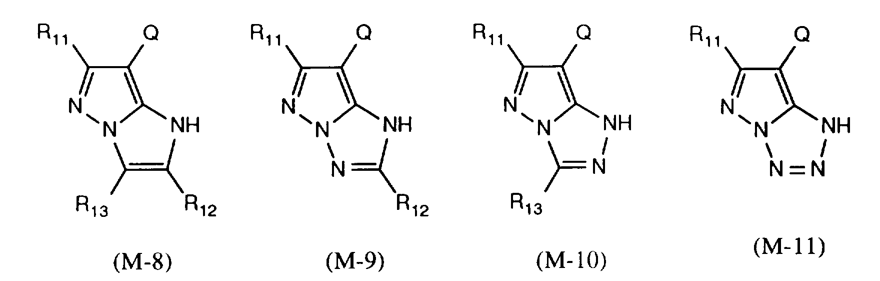 R₁₁, R₁₂ und R₁₃ bedeuten unabhängig voneinander beispielsweise Wasserstoff, Halogen, eine Gruppe der Formel -CR₃, worin die Reste R unabhängig voneinander Wasserstoff oder Alkyl sind, Aryl, Heterocyclyl, Cyano, Hydroxy, Nitro, Carboxyl, Amino, Alkoxy, Aryloxy, Acylamino, Alkylamino, Anilino, Ureido, Sulfamoylamino, Alkylthio, Arylthio, Alkoxycarbonylamino, Sulfonamido, Carbamoyl, Sulfamoyl, Sulfonyl, Alkoxycarbonyl, Heterocyclyl-oxy, Azo, Acyloxy, Carbamoyloxy, Silyloxy, Aryloxycarbonylamino, Imido, heterocyclische Ring-thio, Sulfinyl, Phosphonyl, Aryloxycarbonyl, Acyl oder Azolyl, und vorzugsweise Wasserstoff; Halogen (z.B. Chlor, Brom), eine Gruppe der Formel -CR₃, worin die Reste R unabhängig voneinander Wasserstoff oder Alkyl sind, Aralkyl, Alkenyl, Alkinyl, Cycloalkyl oder Cycloalkenyl und besonders bevorzugt Methyl, Ethyl, Propyl, Isopropyl, t-Butyl, Tridecyl, 2-Methansulfonylethyl, 3-(3-Pentadecylphenoxy)propyl, 3-(4-(2-(4-(4-Hydroxyphenylsulfonyl)phenoxy)dodecanamido)phenyl)propyl, 2-Ethoxytridecyl, Trifluoromethyl, Cyclopentyl, 3-(2,4-Di-t-Amylphenoxy)propyl); Aryl (z.B. Phenyl, 4-t-Butylphenyl, 2,4-Di-t-amylphenyl, 4-Tetradecaneamidophenyl); Heterocyclyl (z.B. 2-Furyl, 2-Thienyl, 2-Pyrimidinyl, 2-Benzothiazolyl); Cyano; Hydroxy, Nitro; Carboxy; Amino; Alkoxy (z.B. Methoxy, Ethoxy, 2-Methoxyethoxy; 2-Dodecylethoxy, 2-Methansulfonylethoxy); Aryloxy (z.B. Phenoxy, 2-Methylphenoxy, 4-t-Butylphenoxy, 3-Nitrophenoxy, 3-t-Butyloxycarbamoylphenoxy, 3-Methoxycarbamoyl); Acylamino (z.B. Acetoamido, Benzamido, Tetradecanamido, 2-(2,4-Di-t-amylphenoxy)butanamido, 4-(3-t-Butyl-4-hydroxyphenoxy)butanamido, 2-(4-(4-Hydroxyphenylsulfonyl)phenoxy)decanamido); Methylbutylamino); Anilino (z.B. Phenylamino, 2-Chloranilino, 2-Chloro-5-tetradecanaminoanilino, 2-Chloro-5-dodecyloxycarbonylanilino, N-Acetylanilino, 2-Chloro-5-(alpha-(3-t-butyl-4-hydroxyphenoxy)dodecanamidoanilino); Ureido (z.B. Phenylureido, Methylureido, N,N-Dibutylureido); Sulfamoylamino (z.B. N,N-Dipropylsulfamoylamino, N-Methyl-N-decylsulfamoylamino); Alkylthio (z.B. Methylthio, Octylthio, Tetradecylthio, 2-Phenoxyethylthio, 3-Phenoxypropylthio, 3-(4-t-Butylphenoxy)propylthio); Arylthio (z.B. Phenylthio, 2-Butoxy-5-t-octylphenylthio, 3-Pentadecylphenylthio, 2-Carboxyphenylthio, 4-Tetradecanamidophenylthio); Alkoxycarbonylamino (z. B. Methoxycarbonylamino, Tetradecyloxycarbonylamino); Sulfonamido (z.B. Methansulfonamido, Hexadecansulfonamido, Benzolsulfonamido, p-Toluolsulfonamido, Octadecansulfonamido, 2-Methyloxy-5-t-butylbenzolsulfonamido); Carbamoyl (z.B. N-Ethylcarbamoyl, N,N-Dibutylcarbamoyl, N-(2-Dodecyloxyethyl)carbamoyl, N-Methyl-N-dodecylcarbamoyl, N-(3-(2,4-Di-t-Amylphenoxy)propyl)carbamoyl); Sulfamoyl (z.B. N-Ethylsulfamoyl, N,N-Dipropylsulfamoyl, N-2(-Dodecyloxyethyl)sulfamoyl, N-Ethyl-N-dodecylsulfamoyl, N,N-Diethylsulfamoyl); Sulfonyl (z.B. Methansulfonyl, Octansulfonyl, Benzolsulfonyl, Toluolsulfonyl); Alkoxycarbonyl (z.B. Methoxycarbonyl, Butoxycarbonyl, Dodecyloxycarbonyl, Octadecyloxycarbonyl); heterocyclische Ringoxy (z.B. l-Phenyltetrazol-5-oxy, 2-Tetrahydropyranyloxy); Azo (z.B. Phenylazo, 4-Methoxyphenylazo, 4-Pivaloylaminophenylazo, 2-Hydroxy-4-propanoylphenylazo); Acyloxy (z.B. Acetoxy); Carbamoyloxy (z.B. N-Methylcarbamoyloxy, N-Phenylcarbamoyloxy); Silyloxy (z.B. Trimethylsilyloxy, Dibutylmethylsilyloxy); Aryloxycarbonylamino (z.B. Phenoxycarbonylamino); Imido (z.B. N-Succinimido, N-Phthalimido, 3-Octadecenylsuccinimido); heterocyclische Ring-thio (z.B. 2-Benzothiazolylthio, 2,4-Diphenyloxy-1,3,5-triazol-6-thio, 2-Pyridylthio); Sulfinyl (z.B. Dodecansulfinyl, 3-Pentadecylphenylsulfinyl, 3-Phenoxypropylsulfinyl); Phosphonyl (z.B. Phenoxyphosphonyl, Octyloxyphosphonyl, Phenylphosphonyl); Aryloxycarbonyl (z.B. Phenoxycarbonyl); Acyl (z.B. Acetyl, 3-Phenylpropanoyl, Benzoyl, 4-Dodecyloxybenzoyl); Azolyl (z.B. Imidazolyl, Pyrazolyl, 3-Chloro-pyrazol-1-yl).

Diese Substituenten sind gegebenenfalls weiter substituiert, beispielsweise durch Halogen oder durch einen über ein C-, O-, N- oder S-Atom gebundenen organischen Rest.

Die bevorzugten Gruppen R₁₁ sind Alkyl, Aryl, Alkoxy, Aryloxy, Alkylthio, Ureido, Urethan und Acylaminogruppen.

R₁₂ kann die Bedeutung von R₁₁ besitzen und ist vorzugsweise Wasserstoff, Alkyl, Aryl, ein heterocyclischer Ring, Alkoxycarbonyl, Carbamoyl, Sulfamoyl, Sulfinyl, Acyl oder Cyano.

R₁₃ kann die Bedeutung von R₁₁ haben und ist vorzugsweise Wasserstoff, Alkyl, Aryl, Heterocyclyl, Alkoxy, Aryloxy, Alkylthio, Arylthio, Alkoxycarbonyl, Carbamoyl oder Acyl, vorzugsweise Alkyl, Aryl, Heterocyclic, Alkylthio oder Arylthio.

Q ist Wasserstoff oder eine Abgangsgruppe wie Halogen, Alkoxy, Aryloxy, Acyloxy, Alkyl- oder Arylsulfonyloxy, Acylamino, Alkyl- oder Aryl-sulfonamido, Alkoxycarbonyloxy, Aryloxycarbonyloxy, Alkyl-, Aryl- oder Heterocyclyl-S-Carbamoylamino, ein 5- oder 6-gliedriger stickstoffhaltiger heterocyclischer Rest, Imido und Arylazo. Diese Gruppen sind gegebenenfalls wie für R₁₁ gezeigt weiter substituiert.

Vorzugsweise ist Q Halogen (z.B. Fluor, Chlor, Brom); Alkoxy (z.B. Ethoxy, Dodecyloxy, Methoxyethylcarbamoylmethoxy, Carboxypropyloxy, Methylsulfonylethoxy, Ethoxycarbonylmethoxy); Aryloxy (z.B. 4-Methylphenoxy, 4-Chlorphenoxy, 4-Methoxyphenoxy, 4-Carboxyphenoxy, 3-Ethoxycarboxyphenoxy, 3-Acetylaminophenoxy, 2-Carboxyphenoxy); Acyloxy (z.B. Acetoxy, Tetradecanoyloxy, Benzoyloxy); Alkyl- oder Aryl-sulfonyloxy (z.B. Methansulfonyloxy, Toluolsulfonyloxy); Acylamino (z.B. Dichloracetylamino, Heptafluorobutyrylamino); Alkyl- oder Arylsulfonamido (z.B. Methanesulfonamido, Trifluoromethansulfonamido, p-Toluolsulfonylamido); Alkoxycarbonyloxy (z.B. Ethoxycarbonyloxy, Benzyloxycarbonyloxy); Aryloxycarbonyloxy (z.B. Phenoxycarbonyloxy); Alkyl-, Aryl- oder Heterocyclyl-S- (z.B. Dodecylthio, 1-Carboxydodecylthio, Phenylthio, 2-Butoxy-5-t-octylphenylthio, Tetrazolylthio); Carbamoylamino (z.B. N-Methylcarbamoylamino, N-Phenylcarbamoylamino); 5- oder 6-gliedriger stickstoffhaltiger Ring (z.B. Imidazolyl, Pyrazolyl, Triazolyl, Tetrazolyl, 1,2-Dihydro-2-oxo-1-pyridyl); Imido (z.B. Succinimido, Hydantoinyl); Arylazo (z.B. Phenylazo, 4-Methoxyphenylazo).

Q kann auch entsprechende Bisverbindungen bilden durch Kondensation von 4 Äquivalenten Kuppler mit einem Aldehyd oder Keton. Des weiteren kann Q photographisch wirksame Gruppen enthalten wie Entwicklungsinhibitoren oder Entwicklungsbeschleuniger. Vorzugsweise ist Q Halogen, Alkoxy, Aryloxy, Alkyl-, Aryl-thio, oder eine 5- oder 6-gliedrige stickstoffhaltige heterocyclische Gruppe, die an den Ort der Kupplung über ein Stickstoffatom gebunden ist.

Pyrazolo-tetrazole sind beschrieben in der JP-A-85/33552; Pyrazolo-pyrazole in der JP-A-85/43,695; Pyrazolo-imidazole in den JP-A-85/35732, JP-A-86/18949 und US-A-4,500,630; Pyrazolo-triazole in den JP-A-85/186,567, JP-A-86/47957, JP-A-85/215,687, JP-A-85/197,688, JP-A-85/172,982, EP-A-119,860, EP-A-173,256, EP-A-178,789, EP-A-178,788 und in Research Disclosure 84/24,624.

Weitere Pyrazoloazol-Magentakuppler sind beschrieben in: JP-A-86/28,947, JP-A-85/140,241, JP-A-85/262,160, JP-A-85/213,937, JP-A-87/278,552, JP-A-87/279,340, JP-A-88/100,457, EP-A-177,765, EP-A-176,804, EP-A-170,164, EP-A-164,130, EP-A-178,794, DE-A-3,516,996, DE-A-3,508,766 und Research Disclosure 81/20919, 84/24531 und 85/25758.

Cyankuppler können z.B. Derivate von Phenol, von 1-Naphthol oder von Pyrazolochinazolon sein. Bevorzugt sind Strukturen der Formel E, 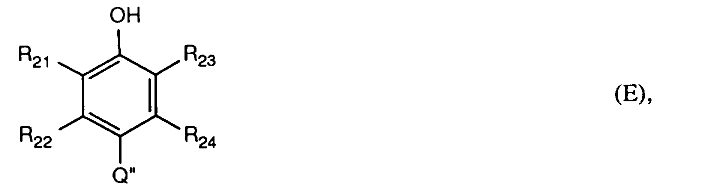 worin R₂₁, R₂₂, R₂₃ und R₂₄ Wasserstoff, Halogen, Alkyl, Carbamoyl, Amino, Sulfonamido, Phosphoramido oder Ureido sind. R₂₁ ist vorzugsweise H oder Cl, R₂₂ ist vorzugsweise eine Alkyl- oder Aminogruppe. R₂₃ ist vorzugsweise eine Aminogruppe und R₂₄ ist vorzugsweise Wasserstoff. Q" ist Wasserstoff oder eine Abgangsgruppe, die bei der Reaktion mit den oxidierten Entwickler abgespalten wird. Eine ausführliche Aufzählung von Cyankupplern ist im US-A-4,456,681 zu finden.

Weitere Beispiele von Cyankupplern sind in folgenden US-A- zu finden:
2,369,929, 2,423,730, 2,434,272, 2,474,293, 2,521,293, 2,521,908, 2,698,794, 2,706,684, 2,772,162, 2,801,171, 2,895,826, 2,908,573, 3,034,892, 3,046,129, 3,227,550, 3,253,294, 3,311,476, 3,386,301, 3,419,390, 3,458,315, 3,476,560, 3,476,563, 3,516,831, 3,560,212, 3,582,322, 3,583,971, 3,591,383, 3,619,196, 3,632,347, 3,652,286, 3,737,326, 3,758,308, 3,839,044, 3,880,661, 4,004,929, 4,124,396, 4,333,999, 4,463,086, 4,456,681, 4,873,183 und 4,923,791 und in den EP-A-354,549 und EP-A-398,664.

In der rotempfindlichen Silberhalogenidemulsionsschicht des erfindungsgemäßen Materials kommen vorzugsweise Cyankuppler der Formel

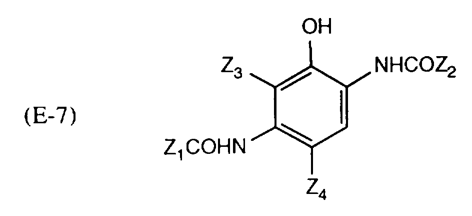

und/oder der Formel

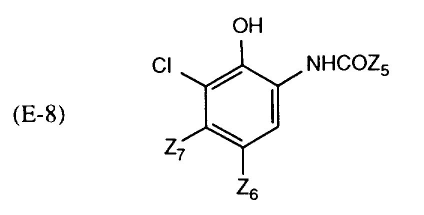

zum Einsatz, worin
Z₁ Alkyl, Aryl, Z₂ Alkyl, Cycloalkyl, Aryl, eine heterocyclische Gruppe, oder eine Ballastgruppe, Z₃ Wasserstoff oder Halogen ist, Z₁ und Z₃ zusammen einen Ring bilden können, und Z₄ Wasserstoff oder eine Abgangsgruppe ist, und Z₅ eine Ballastgruppe, Z₆ Wasserstoff oder eine Abgangsgruppe und Z₇ Alkyl ist.

Die für farbfotographische Materialien üblicherweise verwendeten Farbentwickler sind p-Dialkylaminoaniline. Beispiele hierfür sind 4-Amino-N,N-diethylanilin, 3-Methyl-4-amino-N,N-diethylanilin, 4-Amino-N-ethyl-N-α-hydroxyethylanilin, 3-Methyl-4-amino-N-ethyl-N-α-hydroxyethylanilin, 3-Methyl-4-amino-N-ethyl-N-α-hydroxyethylanilin, 3-Methyl-4-amino-N-ethyl-N-α-methansulphonamidoethylanilin, 3-Methyl-4-amino-N-ethyl-N-α-methoxyethyl-anilin, 3-α-Methansulphonamidoethyl-4-amino-N,N-diethylanilin, 3-Methoxy-4-amino-N-ethyl-N-α-hydroxyethylanilin, 3-Methoxy-4-amino-N-ethyl-N-α-methoxyethylanilin, 3-Acetamido-4-amino-N,N-diethylanilin, 4-Amino-N,N-dimethylanilin, N-Ethyl-N-α-[α'-(α"-methoxyethoxy)ethoxy]ethyl-3-methyl-4-aminoanilin, N-Ethyl-N-α-(α'-methoxyethoxy)ethyl-3-methyl-4-aminoanilin, sowie die Salze solcher Verbindungen, wie z.B. Sulfate, Hydrochloride oder Toluolsulfonate.

Die erfindungsgemäß verwendeten UV-Absorber der Formel I können allein oder zusammen mit dem Farbkuppler und gegebenenfalls weiteren Zusätzen in das farbphotographische Material eingearbeitet werden, indem man sie in hochsiedenden organischen Lösungsmitteln vorlöst. Vorzugsweise verwendet man Lösungsmittel, die höher als 160°C sieden. Typische Beispiele solcher Lösungsmittel sind die Ester von Phthalsäure, Phosphorsäure, Zitronensäure, Benzoesäure oder von Fettsäuren, sowie Alkylamide und Phenole.

Meist verwendet man zusätzlich noch ein niedrig siedendes Lösungsmittel, um das Einarbeiten der Zusätze in das farbphotographische Material zu erleichtern. Beispiele für solche Lösungsmittel sind Ester wie z.B. Ethylacetat, Alkohole wie z.B. Butanol, Ketone wie z.B. Methyl-isobutyl-keton, Chlorkohlenwasserstoffe wie z.B. Methylenchlorid, oder Amide wie z.B. Dimethylformamid. Sind die Zusätze selbst flüssig, so kann man sie auch ohne Zuhilfenahme von Lösungsmitteln in das Photomaterial einarbeiten.

Die erfindungsgemäßen UV-Absorber können gegebenenfalls ohne Öl in der Gelatineschicht dispergiert werden; Research Disclosure 88/296017 und 89/303070.

Weitere Details über verwendbare hochsiedende Lösungsmittel sind in den folgenden Veröffentlichungen zu finden:

Phosphate: GB-A-791,219, BE-A-755,248, JP-A-76/76739, 78/27449, 78/218,252, 78/97573, 79/148,133, 82/216,177, 82/93323 und 83/216,177 und EP-A265,296.
Phthalate: GB-A-791,219, JP-A-77/98050, 82/93322, 82/216,176, 82/218,251, 83/24321, 83/45699, 84/79888.
Amide: GB-A-791,129, JP-A-76/105,043, 77/13600, 77/61089, 84/189,556, 87/239,149, US-A-928,741, EP-A-270,341, WO 88/00723.
Phenole: GB-A-820,329, FR-A-1,220,657, JP-A-69/69946, 70/3818, 75/123,026, 75/82078, 78/17914, 78/21166, 82/212,114 und 83/45699.

Andere sauerstoffhaltige Verbindungen: US-A-3,748,141, 3,779,765, JP-A-73/75126, 74/101,114, 74/10115, 75/101,625, 76/76740, 77/61089, EP-A-304,810 und BE-A-826,039.

Sonstige Verbindungen: JP-A-72/115,369, 72/130,258, 73/127,521, 73/76592, 77/13193, 77/36294, 79/95233, 91/2,748, 83/105,147 und Research Disclosure 82/21918.

Die Menge an hochsiedendem Lösungsmittel liegt z.B. im Bereich von 50 mg bis 2 g pro m Träger, vorzugsweise von 200 mg bis 1 g pro m .

Weitere bevorzugte Farbkuppler zur Verwendung in den erfindungsgemäßen Zusammensetzungen, Beispiele für solche Verbindungen, weitere Zusatzstoffe wie Farbschleier-Inhibitoren, DIR-Kuppler und weitere Lichtschutzmittel wie UV-Absorber, Phenole, Phosphor-III-Verbindungen, metallorganische Komplexe, Hydrochinone und Hydrochinonether, sowie genauere Angaben zum Aufbau verschiedener photographischer Materialien können beispielsweise den Publikationen EP-A-531 258 und EP-A-520 938 und der dort zitierten Literatur entnommen werden.

Die folgenden Beispiele beschreiben die erfindungsgemäßen Überzugsmittel weiter, ohne die Erfindung auf die Beispiele zu beschränken. Sofern nichts anderes angegeben ist, bedeuten darin Teile Gewichtsteile und % Gewichts-%; bei Lösungsmittelgemischen sind allgemein Volumenteile angegeben. Sofern in einem Beispiel Raumtemperatur erwähnt ist, so bedeutet dies eine Temperatur im Bereich 20-25°C. Essigester steht für Essigsäureethylester. Alkylreste sind jeweils geradkettig (n-Alkyl), sofern nichts anderes angegeben ist.

### A) Herstellungsbeispiele

A1) 34,1 g (0,1 Mol) 2-(2,4-Dihydroxyphenyl)-4,6-diphenyl-1,3,5-triazin werden mit 1,0 g (0,008 Mol) p-Dimethylaminopyridin in 150 ml Essigsäureanhydrid während 4 Stunden bei 110°C gerührt. Das Reaktionsgemisch wird auf Raumtemperatur abgekühlt und auf 1 l Wasser gegossen. Das Produkt fällt in kristalliner Form aus. Nach Filtration und anschließendem Waschen mit Wasser wird das Rohprodukt aus Acetonitril umkristallisiert. Man erhält das Produkt vom Schmelzpunkt 167-168°C der Formel: 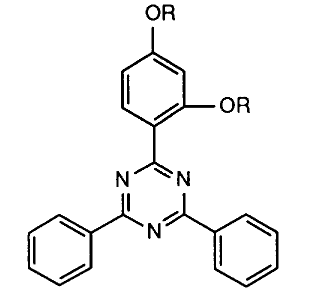 R = -CO-CH₃

| Elementaranalyse: | | berechnet | gefunden |
|---|---|---|---|
| | C: | 70,58 % | 70,47 % |
| | H: | 4,50 % | 4,59 % |
| | N: | 9,88 % | 9,99 % |

A2) 37,2 g (0,1 Mol) 2-Phenyl-4,6-(2,4-dihydroxyphenyl)-1,3,5-triazin werden mit 1,0 g (0,008 Mol) p-Dimethylaminopyridin in 150 ml Essigsäureanhydrid während 4 Stunden bei 100°C gerührt. Das Reaktionsgemisch wird auf Raumtemperatur abgekühlt und auf 1 l Wasser gegossen. Das Rohprodukt wird mit Essigester extrahiert und die organische Phase über Na₂SO₄ getrocknet und anschließend eingedampft. Der feste Rückstand wird aus Toluol umkristallisiert. Man erhält das Produkt vom Schmelzpunkt 176-179°C der Formel: 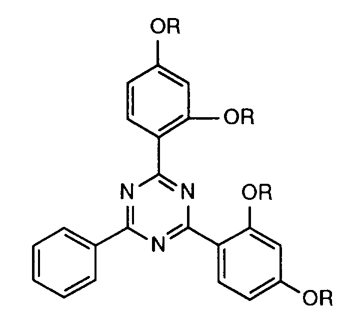 R = -CO-CH₃

| Elementaranalyse: | | berechnet | gefunden |
|---|---|---|---|
| | C: | 64,32 % | 64,35 % |
| | H: | 4,28 % | 4,37 % |
| | N: | 7,76 % | 7,84 % |

A3) 20,3 g (0,05 Mol) 2,4,6-Tris-(2,4-dihydroxyphenyl)-1,3,5-triazin werden mit 0,5 g (0,004 Mol) p-Dimethylaminopyridin in 150 ml Essigsäureanhydrid während 24 Stunden bei 110°C gerührt. Das Reaktionsgemisch wird auf Raumtemperatur abgekühlt und auf 1 l Wasser gegossen. Das Produkt fällt in kristalliner Form aus. Nach Filtration wird aus Toluol umkristallisiert. Man erhält das Produkt vom Schmelzpunkt 180-182°C der Formel: 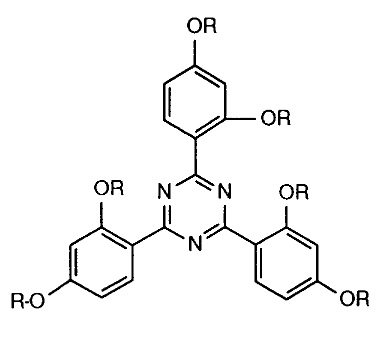 R = -CO-CH₃

A4) 20,4 g (0,05 Mol) 2-(4-Chlorphenyl)-4,6-di(2,4-dihydroxyphenyl)-1,3,5-triazin werden mit 0,5 g (0,004 Mol) p-Dimethylaminopyridin in 150 ml Essigsäureanhydrid während 4 Stunden bei 100°C gerührt. Anschließend wird das Reaktionsgemisch zur Trockne eingedampft und aus einer Mischung von Ligroin und Toluol umkristallisiert. Man erhält das Produkt vom Schmelzpunkt 104-107°C der Formel: 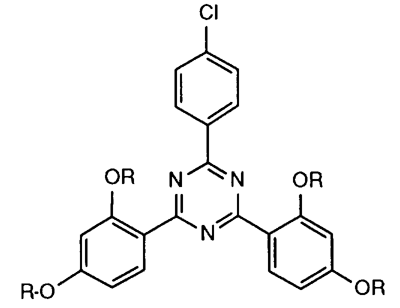 R = -CO-CH₃

| Elementaranalyse: | | berechnet | gefunden |
|---|---|---|---|
| | C: | 60,48 % | 60,37 % |
| | H: | 3,85 % | 3,90 % |
| | N: | 7,30 % | 7,40 % |
| | Cl: | 6,15 % | 6,27 % |

A5) 58,4 g (0,1 Mol) 2-(2-Hydroxy-4-[2-hydroxy-3-(2-ethylhexyloxy)-propyloxy]-phenyl)-4,6-bis-(2,4-dimethylphenyl)- 1,3,5-triazin werden mit 1,0 g (0,008 Mol) p-Dimethylaminopyridin in 250 ml Essigsäureanhydrid während 2 Stunden bei 100°C gerührt. Das Reaktionsgemisch wird auf Raumtemperatur abgekühlt und auf 2 l Wasser gegossen. Das Produkt wird mit Essigester extrahiert, die organische Phase mit Wasser neutral gewaschen, über Na₂SO₄ getrocknet und eingedampft. Man erhält das Produkt der Formel 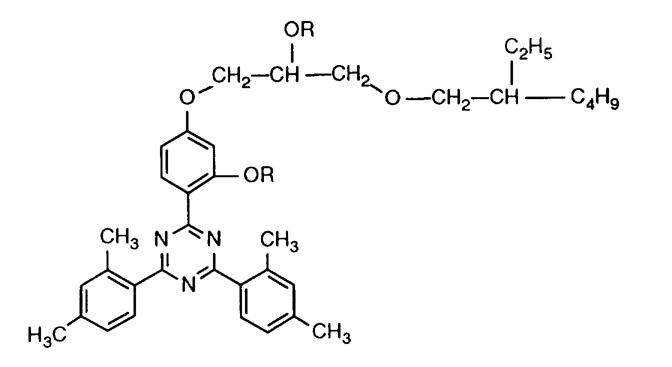 R = -CO-CH₃ als gelbes viskoses Öl mit dem Brechungsindex n_{D} ⁴⁰= 1,5751; Massenspektrum M⁺ = 667.

| Elementaranalyse: | | berechnet | gefunden |
|---|---|---|---|
| | C: | 71,94 % | 71,89 % |
| | H: | 7,40 % | 7,70 % |
| | N: | 6,29 % | 6,41 % |

A6) 53,9 g (0,1 Mol) 2-(2-Hydroxy-4-[2-hydroxy-3-methacryloyloxypropyloxy]-phenyl)-4,6-bis-(2,4-dimethylphenyl)-1,3,5-triazin werden mit 1,0 g (0,008 Mol) p-Dimethylaminopyridin in 250 ml Essigsäureanhydrid während 2 Stunden bei 100°C gerührt. Das Reaktionsgemisch wird auf Raumtemperatur abgekühlt und auf 2 l Wasser gegossen. Das Produkt wird mit Essigester extrahiert, die organische Phase mit Wasser neutral gewaschen, über Na₂SO₄ getrocknet und eingedampft. Man erhält das langsam kristallisierende Produkt, Schmelzpunkt 99-101°C, der Formel 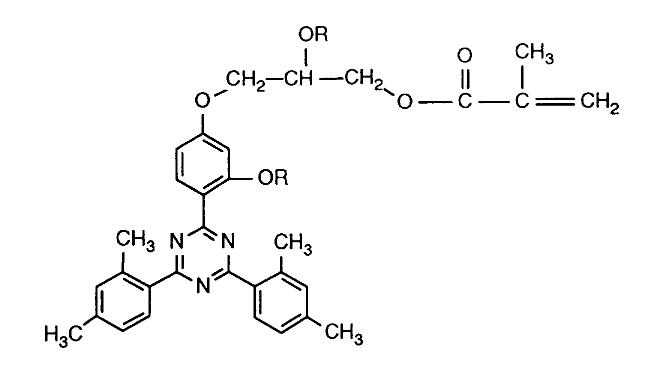 R = -CO-CH₃

| Elementaranalyse: | | berechnet | gefunden |
|---|---|---|---|
| | C: | 69,33 % | 69,36 % |
| | H: | 5,98 % | 6,02 % |
| | N: | 6,74 % | 6,78 % |

A7) 51,0 g (0,1 Mol) 2-(2-Hydroxy-4-octyloxyphenyl)-4,6-bis-(2,4-dimethylphenyl)-1,3,5-triazin werden mit 1,0 g (0,008 Mol) p-Dimethylaminopyridin in 150 ml Essigsäureanhydrid während 2 Stunden bei 100°C gerührt. Das Reaktionsgemisch wird auf Raumtemperatur abgekühlt und auf 1l Wasser gegossen. Das Produkt kristallisiert langsam aus; das kristalline Produkt wird abfiltriert, mit Wasser gewaschen und aus Acetonitril umkristallisiert. Man erhält das Produkt vom Schmelzpunkt 99-100°C der Formel 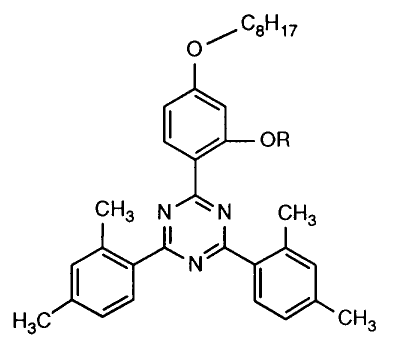 worin R Acetyl darstellt.

| Elementaranalyse: | | berechnet | gefunden |
|---|---|---|---|
| | C: | 76,19 % | 76,21 % |
| | H: | 7,49 % | 7,47 % |
| | N: | 7,62 % | 7,58 % |

A8) 50,3 g eines Gemisches aus 2-(2-Hydroxy-4-[2-hydroxy-3-dodecyloxy-propyloxy]-phenyl)-4,6-bis-(2,4-dimethylphenyl)-1,3,5-triazin und 2-(2-Hydroxy-4-[2-hydroxy-3-tredecyloxy-propyloxy]-phenyl)-4,6-bis-(2,4-dimethylphenyl)-1,3,5-triazin, wobei die Dodecyl- und Tredecylreste jeweils Isomerengemische sind, werden mit 1,0 g (0,008 Mol) p-Dimethylaminopyridin in 150 ml Essigsäureanhydrid während 1 Stunde bei 100°C gerührt. Das Reaktionsgemisch wird auf Raumtemperatur abgekühlt und auf 1l Wasser gegossen. Das Produkt wird mit Essigester extrahiert, die organische Phase mit Wasser neutral gewaschen, eingedampft und unter Hochvakuum bei 100°C getrocknet. Man erhält das Produkt der Formel 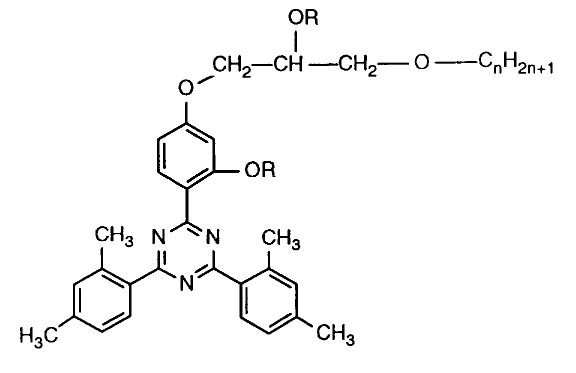 worin R für Acetyl steht und n 12 oder 13 darstellt, als gelbes Harz mit dem Brechungsindex n_{D} ⁴⁰= 1,5638; Massenspektrum M⁺ = 723 und 737.

A9) 9,8 g (0,03 Mol) 2-(2'-Hydroxyphenyl)-4,6-diphenyl-1,3,5-triazin werden mit 0,5 g (0,004 Mol) Dimethylaminopyridin in 100 ml Essigsäureanhydrid während 2 Std. bei 100°C gerührt.
Das Reaktionsgemisch wird auf Raumtemperatur abgekühlt und auf 1 lt Wasser gegossen. Das Produkt fällt in kristalliener Form aus, es wird filtriert, mit Wasser gewaschen und anschliessend aus Acetonitril umkristallisiert.

Man erhält die Verbindung 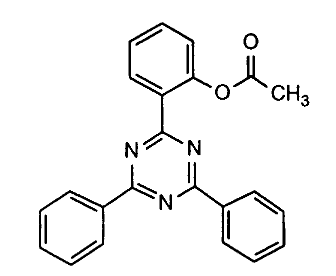 als weisses Produkt mit dem Smp. 142-143°C.

| Elementaranalyse: | | | | | | |
|---|---|---|---|---|---|---|
| berechnet: | %C | 75,19 | gefunden: | %C | 75,04 | 75,07 |
| | %H | 4,66 | | %H | 4,76 | 4,76 |
| | %N | 11,44 | | %N | 11,58 | 11,63 |

A10) 13,0 g (0,04) 2-(2'-Hydroxyphenyl)-4,6-diphenyl-1,3,5-triazin werden mit 0,5 g (0,004 Mol) Dimethylaminopyridin in 100 ml Trifluoressigsäureanhydrid während 4 Std. bei Raumtemperatur gerührt. Das Reaktionsgemisch wird mit 500 ml Acetonitril versetzt, das Produkt wird abfiltriert und getrocknet.

Man erhält die Verbindung 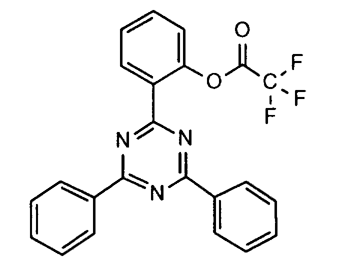 als weisses Produkt mit dem Smp. 121-123°C.

| Elementaranalyse: | | | | | | |
|---|---|---|---|---|---|---|
| berechnet: | %C | 65,56 | gefunden: | %C | 65,24 | 65,38 |
| | %H | 3,35 | | %H | 3,45 | 3,56 |
| | %N | 9,97 | | %N | 9,90 | 9,84 |

A11) 21,3 g (0,05 Mol) 2-(2-Hydroxy-4-hexyloxyphenyl)-4,6-diphenyl-1,3,5-triazin werden in 200 ml Toluol gelöst, 10,0 g (0,25 Mol) Natriumhydroxid werden in 10 ml Wasser gelöst und mit 1,7 g Tetrabutylammoniumhydrogensulfat (0,005 Mol) zur Lösung gegeben. Nun wird unter sehr gutem Rühren eine Lösung aus 7,9 g (0,055 Mol) Benzoylchlorid in 7,5 ml Toluol zugetropft. Gegen Ende des Zutropfens fällt ein weisser Niederschlag aus. Das Reaktionsgemisch wird mit Toluol verdünnt, auf 2 lt Wasser gegossen, wobei das Produkt als Niederschlag ausfällt. Es wird abfiltriert, mit Wasser gewaschen, im Vacuumschrank getrocknet und aus Methylethylketon umkristallisiert. 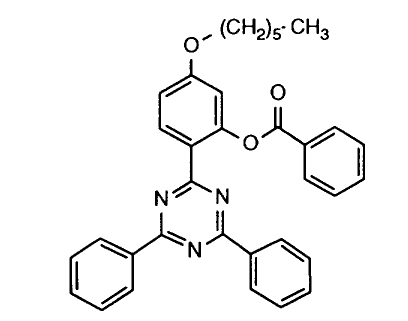 als weisses Produkt mit dem Smp. 166-168°C.

| Elementaranalyse: | | | | | | |
|---|---|---|---|---|---|---|
| berechnet: | %C | 77,10 | gefunden: | %C | 77,07 | 77,03 |
| | %H | 5,90 | | %H | 5,98 | 5,93 |
| | %N | 7,93 | | %N | 7,95 | 7,93 |

A12) 42,5 g (0,1 Mol) 2-(2-Hydroxy-4-hexyloxyphenyl-4,6-diphenyl-1,3,5-triazin werden mit 1,0 g Dimethylaminopyridin und 200 ml Essigsäureanhydrid während 2 Std. bei 100°C gerührt. Das Reaktionsgemisch wird auf 1 lt Wasser gegeben. Der weisse Niederschlag wird abfiltriert, mit Wasser gewaschen, im Vacuumschrank getrocknet und aus Acetonitril umkristallisiert.

Man erhält die Verbindung 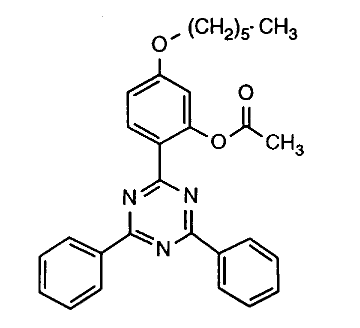 als weisses Produkt mit dem Smp. 129-131°C.

| Elementaranalyse: | | | | | | |
|---|---|---|---|---|---|---|
| berechnet: | %C | 74,50 | gefunden: | %C | 74,43 | 74,40 |
| | %H | 6,25 | | %H | 6,26 | 6,24 |
| | %N | 8,99 | | %N | 9,11 | 9,08 |

A13) 19,2 g (0,02 Mol) 1,12-bis[3-hydroxy-4-(4,6-dimethylphenyl-1,3,5-triazinyl)-phenoxy]dodecan werden mit 0,5 g (0,004 Mol) Dimethylaminopyridin und 150 ml Essigsäureanhydrid während 16 Std. bei 100°C gerührt. Das Reaktionsgemisch wird auf 1 lt Wasser gegossen, der Niederschlag wird abfiltriert, mit Wasser gewaschen, im Vacuumschrank getrocknet und aus Xylol umkristallsiert.

Man erhält die Verbindung 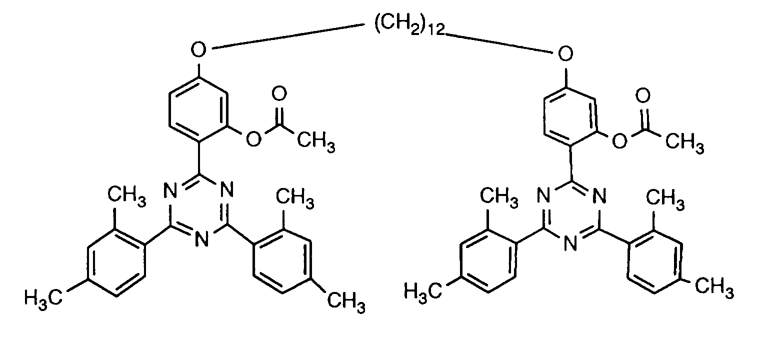 als weisses Produkt mit dem Smp. 182-184°C.

| Elementaranalyse: | | | | | | |
|---|---|---|---|---|---|---|
| berechnet: | %C | 75,83 | gefunden: | %C | 76,03 | 75,93 |
| | %H | 6,94 | | %H | 7,02 | 7,01 |
| | %N | 8,04 | | %N | 8,14 | |

A14) 32,0 g (0,05 Mol) eines Gemisches der Verbindungen 1-[2-Hydroxy-4(3-dodecyloxy-2-hydroxypropyloxy)-phenyl]-4,6-bis-(2,4-dimethylphenyl)-1,3,5-triazin und 1-[2-Hydroxy-4(3-tridecyloxy-2-hydroxypropyloxy)-phenyl]-4,6-bis-(2,4-dimethylphenyl)-1,3,5-triazin werden in 200 ml Toluol bei 40°C gelöst. 10,0 g (0,25 Mol) Natriumhydroxid werden in 10 ml Wasser gelöst und mit 1,7 g (0,005 Mol) Tetrabutylammoniumhydrogensulfat zur Lösung gegeben. Unter gutem Rühren werden 7,9 g (0,055 Mol) Benzoylchlorid in 7,5 ml Toluol zugetropft. Die Reaktion ist schwach Exotherm. Die weisse Emulsion wird auf 2 lt Wasser gegossen, die organische Phase wird mit Wasser neutral gewaschen, über Natriumsulfat getrocknet, eingedampft und am Hochvacuum getrocknet.

Man erhält ein Gemisch der Verbindungen 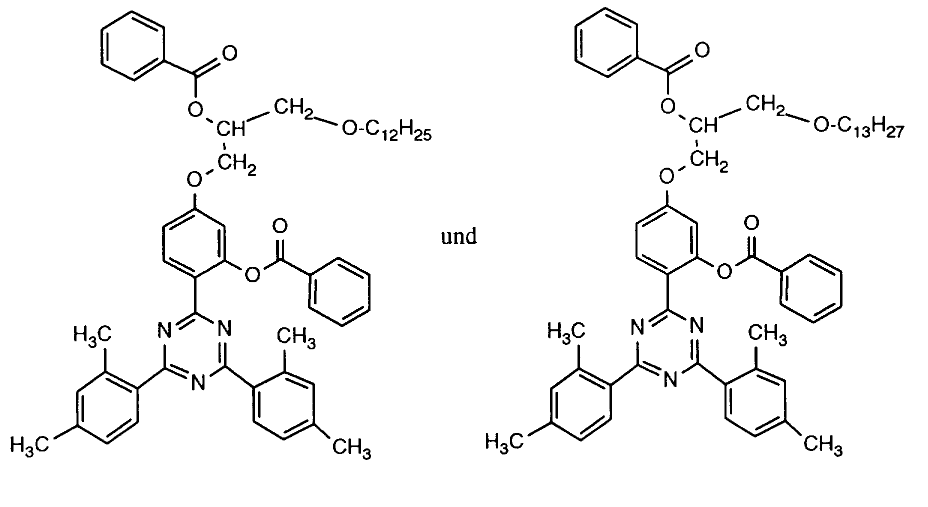 als gelbes Oel.

Massenspektrum: M₁ ⁺ = 847 M₂ ⁺ = 861

A15) 20,4 g (0,05 Mol) 2-(4-Chlorphenyl)-4,6-bis(2,4-dihydroxyphenyl)-1,3,5-triazin werden mit 30,3 g (0,3 Mol) Triethylamin in 299 ml Essigsäureethylester suspendiert. Bei Raumtemperatur werden 29,5 g (0,21 Mol) Benzoylchlorid zugetropft. Anschliessend wird 20 Std. bei 40°C gerührt.
Das Reaktionsgemisch wird mit etwas Toluol verdünnt und mit Wasser gewaschen. Die organische Phase wird über Natriumsulfat getrocknet und eingedampft. Der teilweise krist. Rückstand wird aus Acetonitril umkristallisert.

Man erhält die Verbindung 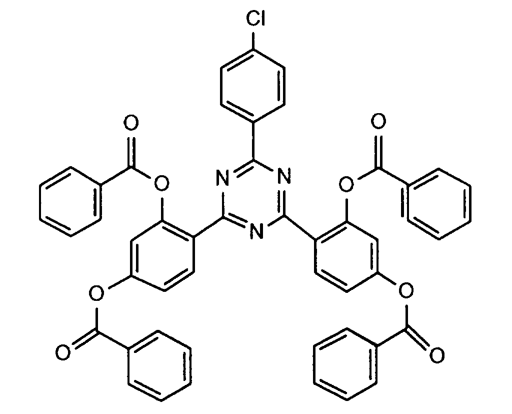 als weisses Produkt mit dem Smp. 149-152°C.

| Elementaranalyse: | | | | | |
|---|---|---|---|---|---|
| berechnet: | %C | 71,40 | gefunden: | %C | 71,17 |
| | %H | 3,66 | | %H | 3,71 |
| | %N | 5,09 | | %N | 5,17 |
| | %Cl | 4,30 | | %Cl | 4,59 |

A16) 18,6 g (0,05 Mol) 2-Phenyl-4,6-bis-(2,4-dihydroxyphenyl)-1,3,5-triazin werden mit 42 ml (0,3 Mol) Triethylamin in 250 ml Essigsäureethylester bei Raumtemperatur vorgelegt. 30,9 g (0,22 Mol) Benzoylchlorid wird bei dieser Temperatur zugetropft. Die weisse Suspension wird 20 Std. bei 50°C gerührt. Der weisse Niederschlag wird nun abfiltriert, das hellgelbe Filtrat eingedampft und der teilweise krist. Rückstand aus Acetonitril umkristallisiert.

Man erhält die Verbindung 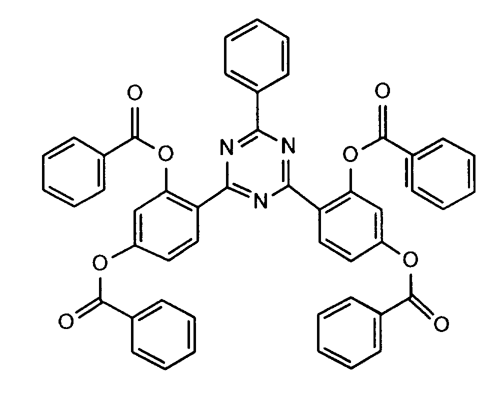 als weisses Produkt mit dem Smp. 152-155°C

| Elementaranalyse: | | | | | |
|---|---|---|---|---|---|
| berechnet: | %C | 74,51 | gefunden: | %C | 73,86 |
| | %H | 3,95 | | %H | 4,18 |
| | %N | 5,32 | | %N | 5,25 |

A17) Eine Suspension von 9,7 g (0,022 Mol) 2-(2-Hydroxy-4-methoxy-5-hexylphenyl)-4,6-bis-phenyl-1,3,5-triazin in 70 ml trockenem Toluol wird unter Stickstoff auf 50°C erhitzt. Nach 30 Min. werden 1,9 g (0,033 Mol) KOH und 1,1 g (0,0033 Mol) Tetrabutylammoniumhydrogensulfat zugesetzt und zum Rückfluß erhitzt. Anschließend werden 4 g (0,033 Mol) Allylbromid innerhalb von 30 Min. zugetropft. Nach 19 h wird filtriert, das Filtrat mit Wasser gewaschen und die organische Phase nach Trocknen über MgSO₄ eingedampft. Das Rohprodukt wird über Silicagel unter Verwendung einer Mischung aus Toluol/Petrolether im Verhältnis 1:1 chromatographiert. Man erhält das Produkt der Formel 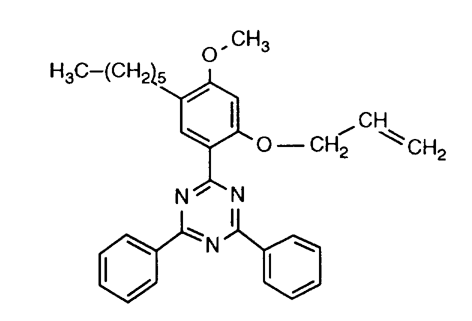 vom Schmelzpunkt 90-92°C.

A18) 28,3 g (0,060 Mol) 2-(2-Hydroxy-4-(2-hydroxy-3-butyloxy-propyloxy)-phenyl)-4,6-diphenyl-1,3,5-triazin in 100 ml Toluol werden unter Stickstoff für 30 Min. auf 50°C erhitzt. Es werden 5,0 g (0,090 Mol) KOH und 2,0 g (0,006 Mol) Tetrabutylammoniumhydrogensulfat zugesetzt und zum Rückfluß erhitzt. Anschließend werden 10,9 g (0,090 Mol) Allylbromid innerhalb von 30 Min. zugetropft. Nach 23 h werden weitere 1,7 g (0,030 Mol) KOH und 3,6 g (0,030 Mol) Allylbromid zugesetzt und die Mischung für weitere 6 h zum Rückfluß erhitzt. Anschließend wird die organische Phase mit Wasser gewaschen, über MgSO₄ getrocknet und eingedampft. Das Rohprodukt wird über Silicagel chromatographiert, eluiert wird mit Toluol, mit Toluol enthaltend 10 % Ethylacetat und schließlich mit Toluol enthaltend 20 % Ethylacetat. Man erhält das Produkt der Formel

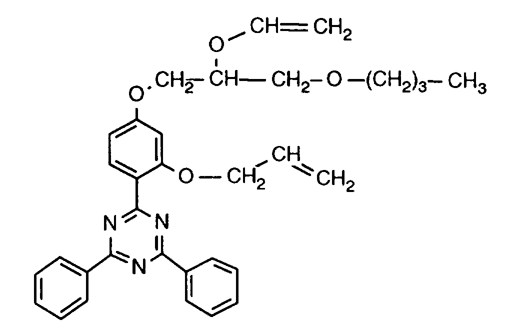

| als viskoses Öl. Elementaranalyse: | | | | | |
|---|---|---|---|---|---|
| berechnet: | %C | 74,02 | gefunden: | %C | 74,59 |
| | %H | 6,76 | | %H | 6,74 |
| | %N | 7,62 | | %N | 7,31 |

A19) Eine Suspension von 170,2 g (0,400 Mol) 2-(2-Hydroxy-4-hexyloxyphenyl)-4,6-bis-phenyl-1,3,5-triazin in 750 ml trockenem Toluol wird unter Stickstoff auf 50°C erhitzt. Nach 30 Min. werden 33,7 g (0,600 Mol) KOH und 12,9 g (0,038 Mol) Tetrabutylammoniumhydrogensulfat zugesetzt und die Mischung zum Rückfluß erhitzt. Anschließend werden 72,5 g (0,600 Mol) Allylbromid innerhalb von 60 Min. zugetropft. Nach 19 h wird filtriert, das Filtrat mit Wasser gewaschen und die organische Phase nach Trocknen über MgSO₄ eingedampft. Umkristallisieren aus Ethylcellosolve ergibt das Produkt der Formel 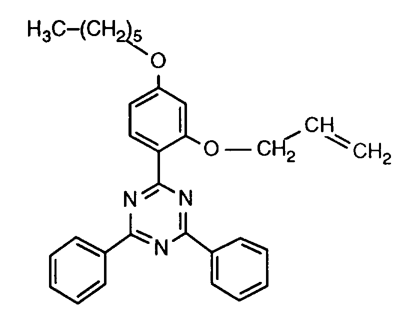 vom Schmelzpunkt 78-80°C.

A20) Eine Suspension von 85,1 g (0,200 Mol) 2-(2-Hydroxy-4-hexyloxyphenyl)-4,6-bis-phenyl-1,3,5-triazin in 500 ml trockenem Toluol wird unter Stickstoff auf 50°C erhitzt. Nach 30 Min. werden 11,2 g (0,200 Mol) KOH und 6,8 g (0,020 Mol) Tetrabutyl-ammoniumhydrogensulfat zugesetzt und die Mischung für 1 h zum Rückfluß erhitzt. Anschließend werden 10 ml (0,095 Mol) 3-Chlor-2-chlormethyl-1-propen innerhalb von 60 Min. zugetropft. Nach 53 h wird filtriert, das Filtrat mit Wasser gewaschen und die organische Phase nach Trocknen über MgSO₄ eingedampft. Umkristallisieren aus Toluol ergibt das Produkt der Formel 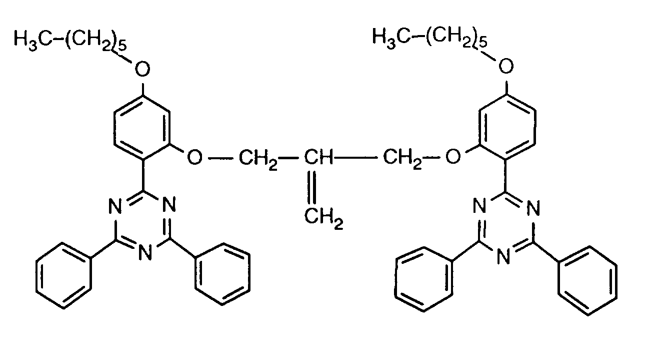 vom Schmelzpunkt 149-150°C.

A21) Eine Suspension von 34,14 g (0,100 Mol) 2-(2,4-Dihydroxyphenyl)-4,6-bis-phenyl-1,3,5-triazin in 200 ml trockenem Tetrahydrofuran (THF) wird unter Stickstoff zunächst mit 11,13 g (0,110 Mol) Triethylamin und anschließend innerhalb von 5 Min. mit 11,94 g (0,11 Mol) Chlorameisensäureethylester in 100 ml trockenem THF versetzt. Die Mischung wird unter Rühren für 3 h auf 40°C erhitzt und anschließend filtriert. Das Lösungsmittel wird vom Filtrat abgezogen und der Rückstand in Dichlormethan gelöst. Nach Waschen mit Wasser und Trocknen über MgSO₄ wird erneut eingedampft. Das Rohprodukt wird über Silicagel unter Verwendung einer Mischung aus Toluol/Petrolether im Verhältnis 1:1 chromatographiert. Man erhält das Produkt der Formel

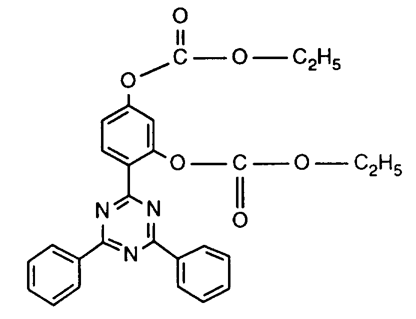

| Elementaranalyse: | | | | | |
|---|---|---|---|---|---|
| berechnet: | %C | 66,80 | gefunden: | %C | 66,77 |
| | %H | 4,78 | | %H | 4,88 |
| | %N | 8,66 | | %N | 8,70 |

A22) Eine Mischung aus 25,5 g (0,05 Mol) 2-(2-Hydroxy-4-octyloxyphenyl)-4,6-bis(2,4-dimethylphenyl)-1,3,5-triazin, 13,9 g (0,09 Mol) tert.-Butyldimethylchlorsilan und 12,3 g (0,18 Mol) Imidazol in 250 ml Dimethylacetamid werden für die Dauer von 40 h bei 70°C gerührt. Anschließend wird auf 20-25°C abgekühlt, filtriert und das Filtrat mit Petrolether enthaltend 3 % Ethylacetat chromatographiert. Nach Kristallisation erhält man die Verbindung der Formel 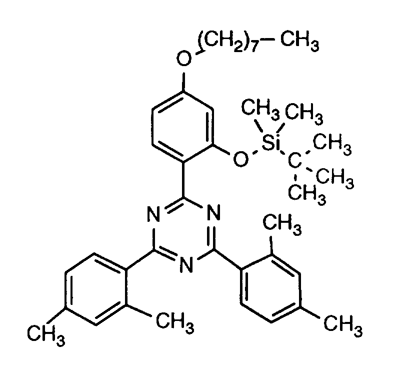 vom Schmelzpunkt 68-70°C.

A23) Zu einer Mischung aus 25,5 g (0,05 Mol) 2-(2-Hydroxy-4-octyloxyphenyl)-4,6-bis(2,4-dimethylphenyl)-1,3,5-triazin, 10,0 g (0,25 Mol) Natriumhydroxid, 10 ml Wasser und 1,7 g (0,005 Mol) Tetrabutyl-ammoniumhydrogensulfat in 200 ml Toluol wird bei 40°C tropfenweise und unter Rühren innerhalb von 15 Min. eine Mischung aus 7,9 g (0,055 Mol) Benzoylchlorid und 7,5 ml Toluol gegeben. Nach 45 Min. Erhitzen auf 40°C wird das Reaktionsgemisch auf 2 l Wasser gegossen. Extraktion mit Toluol, Neutralwaschen der organischen Phase mit Wasser, Eindampfen und Umkristallisieren aus Acetonitril liefert das Produkt der Formel 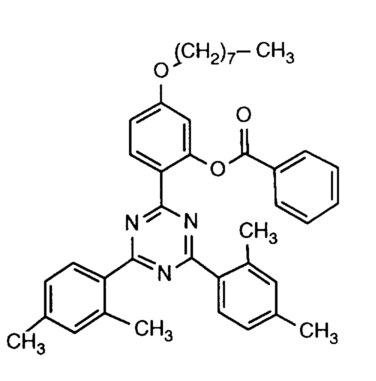 vom Schmelzpunkt 99-101°C.

Zwischenprodukte für die Beispiele A24 und A25: Verbindungen (a)-(c)
a) 2-Mesityl-4,6-dichlor-1,3,5-triazin
   Eine Lösung von 109,5 g (0,55 Mol) 2-Brom-mesitylen (Reinheit 98%) in 150 ml abs. THF (Reinheit 99,5%) wird innerhalb von 1 ¹/₂ Stunden unter Stickstoff zu einer gerührten, auf 60°C temperierten Suspension von 14,6 g (0,60 Mol) Magnesiumspänen (Reinheit 99,8%) in 100 ml abs. THF gegeben, der ein Iodkristall beigegeben ist. Anschließend wird die Mischung für 30 Minuten auf Rückflußtemperatur gehalten (68°C). Nach Abkühlen wird das erhaltene Grignard-Reagens in einen Tropftrichter überführt und tropfenweise zu einer Lösung von 96,0 g (0,52 Mol) Cyanurchlorid (98%) in 270 ml THF gegeben. Während der Zugabe, die 1 ¹/₂ Stunden dauert, wird durch Kühlen eine Temperatur zwischen 15 und 30°C gehalten. Anschließend wird die Mischung bei 25°C für 2 Stunden gerührt, dann auf 2 1 einer Eis-Wasser-Mischung enthaltend 80 ml 32 % HCl (0,81 Mol) gegossen. Nach einstündigem Rühren wird filtriert. Der Filterkuchen wird in 1000 ml Wasser suspendiert, 30 Min. gerührt und wieder filtriert. Diese Operation wird noch zweimal wiederholt. Der Filterkuchen wird für 24 h bei 25°C und einem Druck von 60 mmHg (8000 Pa) über P₂O₅ getrocknet. 171,0 g Rohprodukt werden anschließend in Toluol gelöst, heiß filtriert, und durch Zusatz von Hexan und Kühlen auf 0°C kristallisiert. Nach Abfiltrieren und Trocknen werden 82,8 g des Titelproduktes (a) erhalten 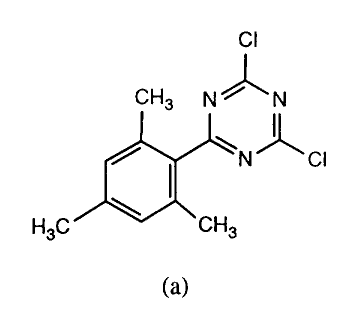 mit dem Smp. 85-91°C.
b) 2-Mesityl-4,6-bis(2,4-dihydroxyphenyl)-1,3,5-triazin
   Zu einer Suspension von 130,0 g (0,485 Mol) 2-Mesityl-4,6-dichlor-1,3,5-triazin (a) in 300 ml Benzin vom Siedebereich 110-140°C und 385 ml Sulfolan werden unter Rühren 148,7 g (1,21 Mol) wasserfreies Aluminiumtrichlorid (Reinheit 98%) gegeben. Die Mischung erwärmt sich dabei auf 45°C. Dazu wird während 45 Min. eine Lösung von 133,5 g (1,21 Mol) Resorcin (Reinheit 98%) in 155 ml Sulfolan gegeben. Die Mischung wird unter HCl-Entwicklung für 5 h 30 Min. auf 80-85°C erwärmt. Die obere Phase (Benzin) wird entfernt, die untere, dickflüssige Phase wird heiß in eine gerührte Mischung aus 2,1 l Methanol und 2,1 1 Wasser gegeben. Nach Rühren für die Dauer von 14 h wird der Feststoff abfiltriert, in 2,2 1 l-molarer HCl für 1 h gerührt und wieder abfiltriert. Der Filterkuchen wird in 1000 ml Wasser suspendiert, 30 Min. gerührt und wieder filtriert. Diese Operation wird noch zweimal wiederholt. Der Filterkuchen wird für 24 h bei 80°C und einem Druck von 60 mmHg (8000 Pa) getrocknet. Es werden 170,5 g des Titelproduktes (b) erhalten der Formel 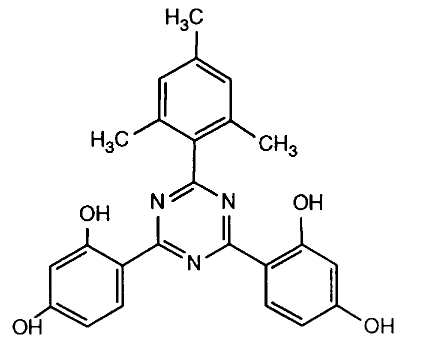 mit dem Smp. 230-234°C.
c) 2-Mesityl-4,6-bis(2-hydroxy-4-[3-n-butyloxy-2-hydroxy-propyloxy]-phenyl)- 1,3,5-triazin
   Eine Mischung von 20,0 g (0,048 Mol) 2-Mesityl-4,6-bis(2,4-dihydroxyphenyl)-1,3,5-triazin (b), 13,8 g (0,105 Mol) n-Butyl-glycidyl-ether (Reinheit 95%) und 1,8 g (4,8 mMol) Ethyl-triphenyl-phosphoniumbromid (Reinheit 97%) in 100 ml Mesitylen (Reinheit 99%) wird unter Stickstoff für 21 h bei 140°C gerührt. Dekantieren und Verdampfen des restlichen Lösungsmittels ergibt 41,2 g Rohprodukt. Dieses wird in 100 ml Ethylacetat gelöst und durch eine 10,5 cm hohe Schüttung Kieselgel 60 (230-400 mesh) vom Durchmesser 7,5 cm filtriert, eluiert wird mit 3 l Ethylacetat/Hexan (1:1-Mischung). Nach Entfernen des Lösungsmittels erhaltene 34,0 g Feststoff werden erneut in 25 ml Ethylacetat gelöst. Zu der dickflüssigen Lösung werden 250 ml Hexan gegeben. Nach zweistündigem Rühren bei 0°C wird abfiltriert; der Feststoff wird für 24 h bei 80°C und einem Druck von 60 mmHg (8000 Pa) getrocknet. Es werden 23,0 g des Titelproduktes (c) erhalten der Formel 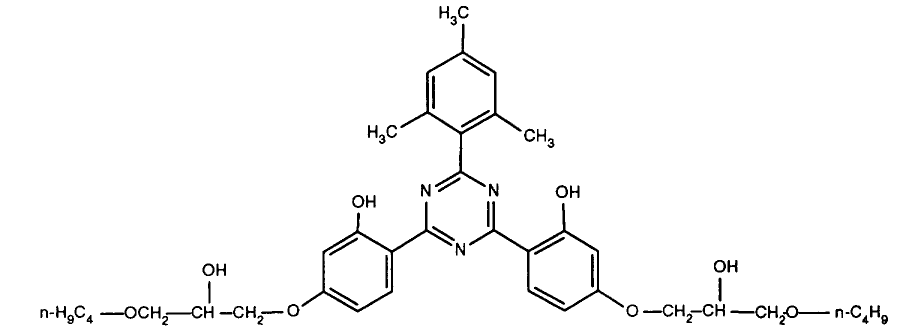 mit dem Smp. 125-131°C.

A24) 2-Mesityl-4,6-bis-(2,4-diacetoxyphenyl)-1,3,5-triazin Eine Mischung aus 20,8 g (0,050 Mol) 2-Mesityl-4,6-bis-(2,4-dihydroxyphenyl)-1,3,5-triazin (b), 0,5 g (0,004 Mol) 4-Dimethyl-aminopyridin (DMAP, Fluka, 98%) und 150 ml (162,0 g, 1,59 Mol) Acetanhydrid (Fluka, 99,5 %) wird unter Stickstoff für 16 h bei 110°C gerührt. Nach Abkühlen wird auf 1000 ml Wasser gegossen, bei 25°C für 1 h gerührt und der Feststoff abfiltriert. Das Rohprodukt wird mit 100 ml Wasser gewaschen und für 14 h bei 65°C/8 kPa getrocknet. Umkristallisieren aus einer Mischung aus 2 Volumenteilen Toluol und einem Volumenteil Hexan liefert das Produkt der Formel 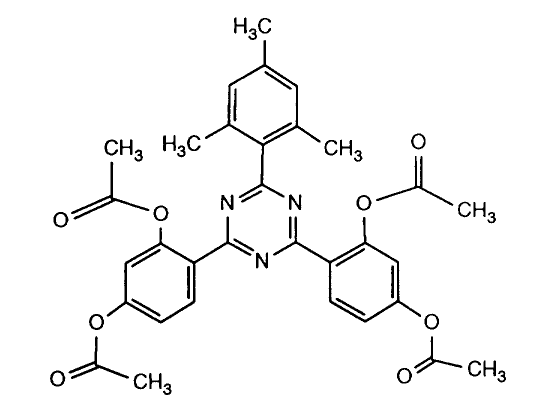 vom Schmelzpunkt 160-162°C.

A25) 2-Mesityl-4,6-bis-[2-acetoxy-4-(2-acetoxy-3-n-butoxypropoxy)-phenyl]-1,3,5-triazin Eine Mischung aus 30,4 g (0,045 Mol) 2-Mesityl-4,6-bis(2-hydroxy-4-[3-n-butyloxy-2-hydroxy-propyloxy]-phenyl)-1,3,5-triazin (c), 0,6 g (0,0045 Mol) 4-Dimethylaminopyridin (DMAP, Fluka, 98%) und 150 ml (162,0 g, 1,59 Mol) Acetanhydrid (Fluka, 99,5 %) wird unter Stickstoff für 17 h bei 110°C gerührt. Nach Abkühlen wird auf 1000 ml Wasser gegossen, bei 25°C für 3 h gerührt anschließend mit 500 ml Ethylacetat extrahiert. Die organische Phase wird mit 200 ml wässr. NaCl gewaschen und über MgSO₄ getrocknet. Es wird über eine Schüttung aus Kieselgel 60 (230-400 mesh; Höhe 4 cm; Durchmesser 6,5 cm) chromatographiert und mit Ethylacetat eluiert. Nach Abtrennen des Lösungsmittels und dreistündigem Trocknen bei 100°C/13 Pa erhält man das Titelprodukt der Formel 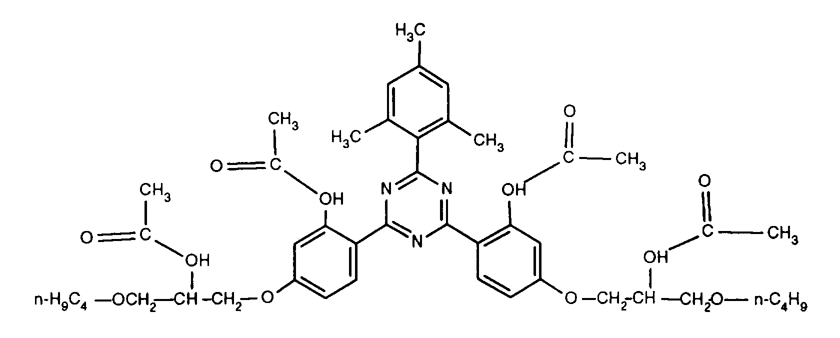 als gelbliches Harz. UV (Ethylacetat): ε(max: 302 nm) = 50400

A26) Verwendung von 2,4,6-Tris-(2-hydroxyphenyl)-1,3,5-triazin an Stelle von 2,4,6-Tris-(2,4-dihydroxyphenyl)-1,3,5-triazin in dem Herstellungsverfahren gemäß Beispiel A3) liefert ein Produkt vom Schmelzpunkt 147-149°C der Formel: 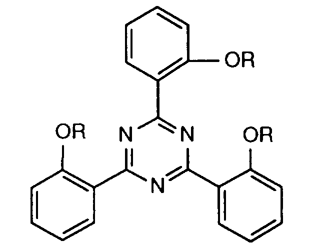 R = -CO-CH₃

### B) Anwendungsbeispiele

Biespiel B1 : Stabilisierung eines 2-Schicht Metalliclackes
Die zu prüfende Verbindung wird in 5-10 g Xylol eingearbeitet und einem Klarlack folgender Zusammensetzung geprüft:

| | |
|---|---|
| Synthacryl^{®} SC 303 ¹⁾ | 27,51 |
| Synthacryl^{®} SC 370 ²⁾ | 23,34 |
| Maprenal^{®} MF 650 ³⁾ | 27,29 |
| Butylacetat/Butanol (37/8) | 4,33 |
| Isobutanol | 4,87 |
| Solvesso^{®} 150 ⁴⁾ | 2,72 |
| Kristallöl K-30 ⁵⁾ | 8,74 |
| Verlaufshilfsmittel Baysilon^{®} MA ⁶⁾ | 1,20 |
| | 100,00 g |

| | |
|---|---|
| 1) Acrylatharz, Fa. Hoechst AG; 65 % Lösung in Xylol/Butanol 26:9 | |
| 2) Acrylatharz, Fa. Hoechst AG; 75 % Lösung in Solvesso^{®}100⁴⁾ | |
| 3) Melaminharz, Fa. Hoechst AG; 55 % Lösung in Isobutanol | |
| 4) aromat. Kohlenwasserstoffgemisch, Siedebereich 182-203°C (Solvesso^{®} 150) bzw. 161-178°C (Solvesso^{®} 100); Hersteller: Fa. ESSO | |
| 5) aliphat. Kohlenwasserstoffgemisch, Siedebereich 145-200°C; Hersteller: Fa. Shell | |
| 6) 1 % in Solvesso^{®} 150⁴⁾; Hersteller: Fa. Bayer AG | |

Dem Klarlack werden 2 % der zu prüfenden Verbindung zugesetzt, bezogen auf den Feststoffgehalt des Lackes. Einige weitere Lackproben werden hergestellt, die zusätzlich zu der erfindungsgemäßen Verbindung 0,7 % der Verbindung 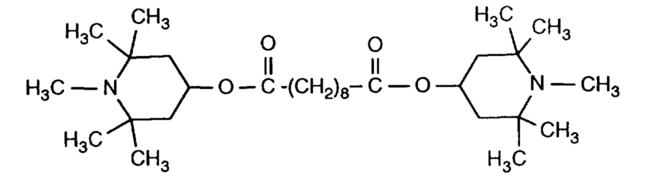 (Verbindung B) bezogen auf den Feststoffgehalt des Lackes enthalten. Als Vergleich dient ein Klarlack, der kein Lichtschutzmittel enthält.

Der Klarlack wird mit Solvesso^{®} 100 auf Spritzfähigkeit verdünnt und auf ein vorbereitetes Aluminiumblech (coil coat, Füller, hellgrün metallic Basislack) gespritzt und bei 130°C 30 Minuten eingebrannt. Es resultiert eine Trockenfilmdicke von 40-50 µm Klarlack. Die Proben werden dann in einem UVCON^{®}-Bewitterunbsgerät der Fa. Atlas Corp. (UVB-313 Lampen) bei einem Zyklus von 8 h UV-Bestrahlung bei 70°C und 4 h Kondensation bei 50°C bewittert.
Von den Proben wird in regelmäßigen Abständen der Oberflächenglanz (20°-Glanz gemäß DIN 67530) gemessen. Die Ergebnisse sind in nachfolgender Tabelle zusammengestellt.

**Tabelle:**

| | | | | | | | |
|---|---|---|---|---|---|---|---|
| 20°-Glanz gemäß DIN 67530 vor und nach Bewitterung | | | | | | | |

| Stabilisator (Verb. aus Beispiel) | 20°-Glanz nach | | | | | | |
|---|---|---|---|---|---|---|---|
| | 0 | 400 | 800 | 1200 | 1600 | 2000 | h Bewitterung |
| keiner | 87 | 84 | 46 | | | | |
| A5 | 89 | 91 | 84 | | | | |
| A6 | 89 | 91 | 89 | | | | |
| A7 | 88 | 91 | 88 | | | | |
| A8 | 87 | 91 | 89 | 78 | | | |
| A26 | 89 | 91 | 90 | 87 | | | |
| A1 | 88 | 91 | 90 | 77 | | | |
| A5 + B | 87 | 90 | 89 | 77 | 88 | 84 | |
| A6 + B | 88 | 91 | 90 | 74 | 87 | 69 | |
| A7 + B | 88 | 91 | 90 | 75 | 88 | 58 | |
| A8 + B | 88 | 85 | 89 | 83 | 87 | 65 | |
| A26 + B | 88 | 90 | 90 | 88 | 85 | 81 | |
| A1 + B | 88 | 91 | 90 | 89 | 86 | 75 | |

Die erfindungsgemäß stabiliserten Proben weisen eine bessere Bewitterungsstabilität (Glanzhaltung, Rißfestigkeit) auf als die unstabilisierte Vergleichsprobe.

## Patentansprüche

1. Zusammensetzung enthaltend
A) ein gegen Schädigung durch Licht, Sauerstoff und/oder Hitze empfindliches organisches Material und
B) als Stabilisator eine Verbindung der Formel I 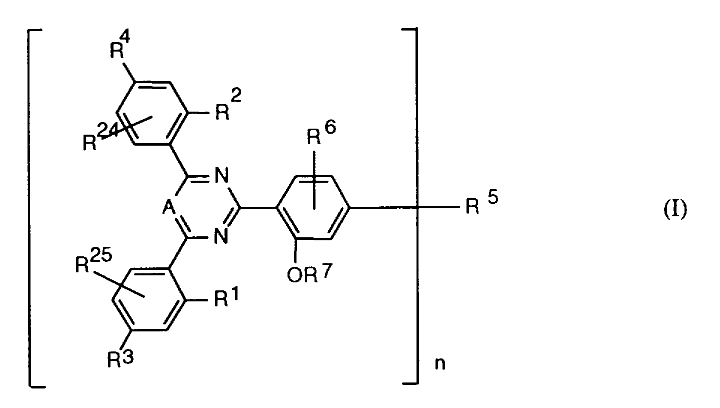
worin n 1 oder 2 ist;
A für CH oder ein Stickstoffatom steht;
R¹ und R, unabhängig voneinander, H; C₁-C₁₂-Alkyl; C₅-C₁₂-Cycloalkyl, Trifluormethyl oder OR⁷ bedeuten;
R³ und R⁴, unabhängig voneinander, H; C₁-C₁₂-Alkyl; C₅-C₁₂-Cycloalkyl, OR¹⁷, Phenyl, CN, Halogen oder OR⁷ bedeuten;
R⁵ im Fall n = 1 als monovalenter Rest C₆-C₁₈-Alkyl; C₁-C₁₈-Alkoxy; Halogen; -O-CO-R¹; -O-SO₂-R¹³; 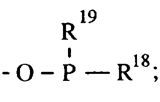 oder 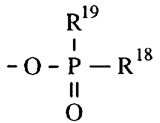 bedeutet; oder R⁵ im Fall n = 1 eine der für R¹⁷ angegebenen Bedeutungen hat oder -O-R¹⁷ bedeutet; und
R⁵ als monovalenter Rest im Fall, daß R¹ und R nicht OR⁷ sind, zusätzlich H und C₁-C₅-Alkyl umfaßt;
R⁵ im Fall n = 2 als divalenter Rest -O-G-O- bedeutet, wobei
G C₂-C₁₆-Alkylen; C₄-C₁₂-Alkenylene; Xylylen; oder C₃-C₂₀-Alkylen, das durch -O- unterbrochen und/oder durch OR⁷ substituiert ist, bedeutet; oder G für eine der Gruppen -CH₂CH(OR⁷)CH₂O-R⁰-OCH₂CH(OR⁷)CH₂-; -CO-R¹-CO-; -CO-NH-R-NH-CO-; -(CH₂)ₘ-COO-R³-OOC-(CH₂)ₘ-, worin m eine ganze Zahl aus dem Bereich 1 bis 3 ist; oder 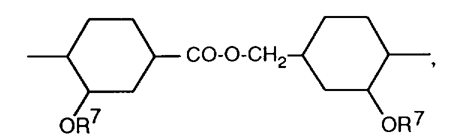 steht;
R⁶ H; C₂-C₁₈-Alkenyl; -X-Z³; unsubstituiertes oder am Phenylkern durch Methyl, Halogen, -CN oder Methoxy substituiertes Benzoyl; Halogen; -SR¹⁴; -SOR¹³; -SO₂R¹³; -C(Z³)=N-Z³; -CH(Z³)-NH-Z³; ein Rest der Formel 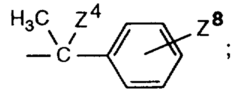 oder ein Rest der Formel 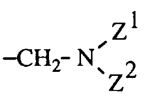 ist;
R⁷ eine der Bedeutungen -CO-R¹¹, -SO₂-R¹³, 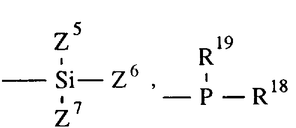 oder 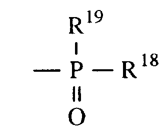 hat oder Allyl oder eine Gruppe der Formel 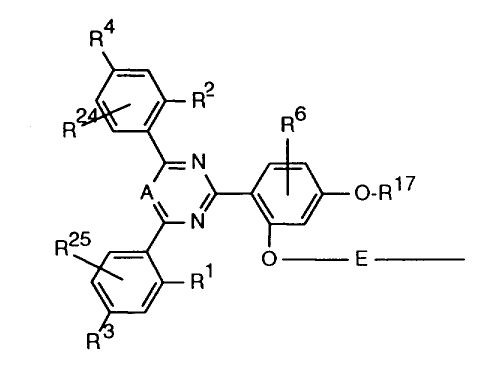 darstellt, worin E C₃-C₁₈-Alkylen oder C₄-C₁₈-Alkenylen ist;
R⁸ C₁-C₁₈-Alkyl; C₃-C₁₈-Alkenyl; durch O, N oder S unterbrochenes und/oder durch OR⁷ substituiertes C₃-C₂₀-Alkyl; durch -P(O)(OR¹⁴)₂, -N(R⁹)(R¹⁰) oder -OCOR¹¹ und/oder
OR⁷ substituiertes C₁-C₄-Alkyl; Glycidyl; C₅-C₁₂-Cycloalkyl oder C₇-C₁₁-Phenylalkyl bedeutet; oder eine Gruppe der Formel 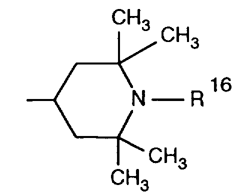 darstellt; R⁹ und R¹⁰, unabhängig voneinander, C₁-C₁₂-Alkyl; C₃-C₁₂-Alkoxyalkyl;
C₄-C₁₆-Dialkylaminoalkyl oder C₅-C₁₂-Cycloalkyl bedeuten oder
R⁹ und R¹⁰ zusammen C₃-C₉-Alkylen oder -Oxaalkylen oder -Azaalkylen bedeuten;
R¹¹ C₁-C₁₈-Alkyl; durch Halogen substituiertes C₁-C₁₈-Alkyl; C₅-C₁₂-Cycloalkyl;
C₂-C₁₈-Alkenyl; -CH₂-CO-CH₃; C₇-C₁₂-Aralkyl; C₁-C₁₂-Alkoxy; oder Phenyl bedeutet, welches unsubstituiert oder durch C₁-C₁₂-Alkyl,C₁-C₄-Alkoxy, Halogen und/oder Benzyl substituiert ist;
R¹ C₁-C₁₈-Alkyl; durch Halogen substituiertes C₁-C₁₈-Alkyl; C₅-C₁₂-Cycloalkyl;
C₂-C₁₈-Alkenyl; Phenyl; oder -R¹⁵-O-CO-R¹¹ bedeutet; oder eine Gruppe der Formel 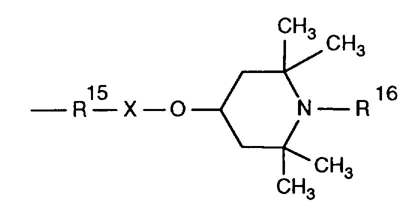 darstellt;
R¹³ C₁-C₁₂-Alkyl; C₆-C₁₀-Aryl; oder C₇-C₁₈-Alkylaryl bedeutet; und
R¹⁴ C₁-C₁₂-Alkyl oder Phenyl oder C₇-C₁₅-Phenylalkyl;
R¹⁵ C₁-C₁₈-Alkylen oder C₂-C₁₈-Alkenylen;
R¹⁶ Wasserstoff; Oxyl; Formyl; C₂-C₈-Alkanoyl; C₁-C₁₈-Alkyl; C₁-C₁₈-Alkoxy;
C₅-C₁₂-Cycloalkyl; C₅-C₁₂-Cycloalkoxy; C₇-C₁₁-Phenylalkyl; C₇-C₁₁-Phenylalkyl, welches am Phenylring durch 1 bis 3 Reste C₁-C₄-Alkyl oder C₁-C₈-Alkanoyl substituiert ist; oder C₇-C₁₁-Phenylalkoxy;
R¹⁷ C₅-C₁₈-Alkyloxycarbonyl; oder C₂-C₁₈-Alkenyl bedeutet; oder R¹⁷ C₁-C₁₈-Alkyl bedeutet, das substituiert ist durch OR⁷, C₁-C₁₈-Alkoxy, C₅-C₁₂-Cycloalkoxy,
C₂-C₁₈-Alkanoyl, C₂-C₈-Alkenyloxy, Halogen, -COOR⁸, -CONH₂, -CONHR⁹, -CON(R⁹)(R¹⁰), -NHR⁹, -N(R⁹)(R¹⁰), -NHCOR¹¹, -CN, -OCOR¹¹, eine Gruppe der Formel 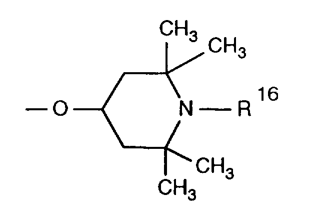 und/oder Phenoxy, welches unsubstituiert ist oder durch C₁-C₁₈-Alkyl, C₁-C₁₈-Alkoxy oder Halogen substituiert ist; oder R¹⁷ C₃-C₅₀-Alkyl bedeutet, welches durch O unterbrochen ist und durch OR⁷ substituiert sein kann; oder R¹⁷ Glycidyl;
C₅-C₁₂-Cycloalkyl; durch OR⁷, C₁-C₄-Alkyl oder -OCOR¹¹ substituiertes
C₅-C₁₂-Cycloalkyl; oder unsubstituiertes oder durch OR⁷, Cl oder CH₃ substituiertes C₇-C₁₁-Phenylalkyl bedeutet; R¹⁸ und R¹⁹, unabhängig voneinander, C₁-C₁₂-Alkoxy, Phenoxy, C₁-C₁₂-Alkyl, C₅-C₁₂-Cycloalkyl, Benzyl, Tolyl oder Phenyl darstellen;
R⁰ C₂-C₁₀-Alkylen; durch -O- unterbrochenes C₄-C₅₀-Alkylen; Phenylen; oder eine Gruppe -Phenylen-D-Phenylen- ist, worin D für -O-, -S-, -SO₂-, -CH₂- oder -C(CH₃)₂- steht;
R¹ C₂-C₁₀-Alkylen; durch O oder S unterbrochenes C₂-C₁₀-Alkylen; C₆-C₁₂-Arylen; oder C₂-C₆-Alkenylen;
R C₂-C₁₀-Alkylen; Phenylen; Tolylen; Diphenylenmethan; oder 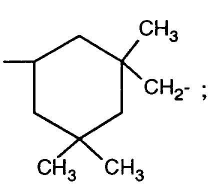 und
R³ C₂-C₁₀-Alkylen oder durch -O- unterbrochenes C₄-C₂₀-Alkylen ist;
R⁴ und R⁵, unabhängig voneinander, H, C₁-C₁₂-Alkyl; C₂-C₆-Alkenyl;
C₁-C₁₂-Alkoxy; C₃-C₁₈-Alkenyloxy; Halogen; Trifluormethyl; C₇-C₁₁-Phenylalkyl; Phenyl; durch C₁-C₁₈-Alkyl, C₁-C₁₈-Alkoxy oder Halogen substituiertes Phenyl; Phenyloxy; oder durch C₁-C₁₈-Alkyl, C₁-C₁₈-Alkoxy oder Halogen substituiertes Phenyloxy darstellen;
X eine direkte Bindung oder -CO- bedeutet;
Z¹ und Z, unabhängig voneinander, C₁-C₁₂-Alkyl sind oder gemeinsam C₄-C₁₀-Alkylen darstellen, das durch ein Sauerstoffatom unterbrochen sein kann;
Z³ C₁-C₂₀-Alkyl oder C₇-C₁₅-Phenylalkyl und
Z⁴ und Z⁸, unabhängig voneinander, Wasserstoff oder Methyl sind; und
Z⁵, Z⁶ und Z⁷, unabhängig voneinander, C₁-C₁₈-Alkyl, Cyclohexyl, Phenyl oder C₁-C₁₈-Alkoxy bedeuten.

2. Zusammensetzung gemäß Anspruch 1, worin in der Verbindung der Formel I
R¹ und R, unabhängig voneinander, H; C₁-C₁₂-Alkyl; Cyclohexyl, Trifluormethyl oder OR⁷ bedeuten;
R³ und R⁴, unabhängig voneinander, H; C₁-C₁₂-Alkyl; Cyclohexyl, C₁-C₁₈-Alkoxy, durch O unterbrochenes und/oder durch OR⁷ substituiertes C₃-C₁₈-Alkoxy, Halogen oder OR⁷ bedeuten;
R⁵ im Fall n = 1 als monovalenter Rest eine der für R¹⁷ angegebenen Bedeutungen hat oder C₆-C₁₈-Alkyl; C₁-C₁₈-Alkoxy; Halogen; -O-CO-R¹; -O-SO₂-R¹³ oder -O-R¹⁷ bedeutet; und im Fall, daß R¹ und R nicht OR⁷ sind, zusätzlich H und C₁-C₅-Alkyl umfaßt;
R⁵ im Fall n = 2 als divalenter Rest -O-G-O- bedeutet, wobei
G C₂-C₁₆-Alkylen; C₄-C₁₂-Alkenylene; Xylylen; C₃-C₂₀-Alkylen, das durch -O- unterbrochen ist, bedeutet; oder G für eine der Gruppen
-CH₂CH(OR⁷)CH₂O-R⁰-OCH₂CH(OR⁷)CH₂-; -CO-R¹-CO-; -CO-NH-R-NH-CO-;
-(CH₂)ₘ-COO-R³-OOC-(CH₂)ₘ-, worin m eine ganze Zahl aus dem Bereich 1 bis 3 ist; oder 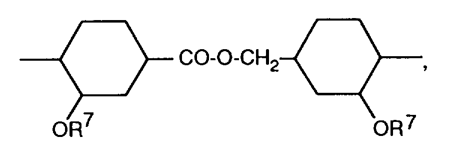 steht;
R⁶ in o-Position zu R⁵ und in p-Position zu OR⁷ steht;
R⁹ und R¹⁰, unabhängig voneinander, C₁-C₁₂-Alkyl; C₃-C₁₂-Alkoxyalkyl;
C₄-C₁₆-Dialkylaminoalkyl oder Cyclohexyl bedeuten oder
R⁹ und R¹⁰ zusammen C₃-C₉-Alkylen oder -Oxaalkylen oder -Azaalkylen bedeuten;
R¹¹ C₁-C₁₈-Alkyl; Trihalogenmethyl; C₂-C₁₈-Alkenyl; -CH₂-CO-CH₃; C₇-C₁₂-Aralkyl;
C₁-C₁₂-Alkoxy; oder Phenyl bedeutet, welches unsubstituiert oder durch C₁-C₁₂-Alkyl,
C₁-C₄-Alkoxy, Halogen und/oder Benzyl substituiert ist;
R¹⁷ C₅-C₁₈-Alkyloxycarbonyl; oder C₂-C₁₈-Alkenyl bedeutet; oder R¹⁷ C₁-C₁₈-Alkyl bedeutet, das substituiert ist durch OR⁷, C₁-C₁₈-Alkoxy, C₂-C₁₈-Alkanoyl,
C₂-C₈-Alkenyloxy, Halogen, -COOR⁸, -CONH₂, -CONHR⁹, -CON(R⁹)(R¹⁰), -NHR⁹, -N(R⁹)(R¹⁰), -NHCOR¹¹, -CN, -OCOR¹¹, eine Gruppe der Formel 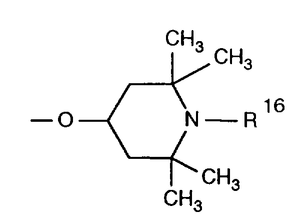 und/oder Phenoxy, welches unsubstituiert ist oder durch C₁-C₁₈-Alkyl, C₁-C₁₈-Alkoxy, oder Halogen substituiert ist; oder R¹⁷ C₄-C₂₀-Alkyl darstellt, welches durch O unterbrochen ist und durch OR⁷ substituiert sein kann; oder R¹⁷ Glycidyl; Cyclohexyl; durch OR⁷, C₁-C₄-Alkyl oder -OCOR¹¹ substituiertes Cyclohexyl; oder unsubstituiertes oder durch Cl oder CH₃ substituiertes C₇-C₁₁-Phenylalkyl bedeutet; und
R¹⁸ und R¹⁹, unabhängig voneinander, C₁-C₆-Alkoxy, C₁-C₄-Alkyl, Cyclohexyl, Tolyl oder Phenyl darstellen;
R⁴ und R⁵, unabhängig voneinander, H oder C₁-C₁₂-Alkyl sind; und
Z⁵, Z⁶ und Z⁷, unabhängig voneinander, C₁-C₄-Alkyl, oder C₁-C₄-Alkoxy bedeuten.

3. Zusammensetzung gemäß Anspruch 1, worin in der Verbindung der Formel I
R¹ und R, unabhängig voneinander, H; C₁-C₄-Alkyl oder OR⁷ bedeuten;
R³ und R⁴, unabhängig voneinander, H; C₁-C₄-Alkyl; C₁-C₈-Alkoxy, durch O unterbrochenes und/oder durch OR⁷ substituiertes C₃-C₁₂-Alkoxy, Halogen oder OR⁷ bedeuten;
R⁵ im Fall n = 1 als monovalenter Rest eine der für R¹⁷ angegebenen Bedeutungen hat oder Halogen; C₆-C₁₈-Alkyl; C₁-C₁₈-Alkoxy; -O-CO-R¹; -O-SO₂-R¹³ oder -O-R¹⁷ bedeutet; und im Fall, daß R¹ und R nicht OR⁷ sind, zusätzlich H und C₁-C₅-Alkyl umfaßt;
R⁵ im Fall n = 2 als divalenter Rest -O-G-O- bedeutet, wobei
G C₂-C₁₆-Alkylen; C₄-C₁₂-Alkenylene; oder Xylylen bedeutet; oder G für eine der Gruppen -CH₂CH(OR⁷)CH₂O-R⁰-OCH₂CH(OR⁷)CH₂-; -CO-R¹-CO-;
-CO-NH-R-NH-CO-; -(CH₂)ₘ-COO-R³-OOC-(CH₂)ₘ- steht, worin m eine ganze Zahl aus dem Bereich 1 bis 3 ist;
R⁶ H; Allyl; C₁-C₁ₒ-Alkyl; Acetyl oder Benzoyl ist;
R⁷ eine der Bedeutungen -CO-R¹¹, -SO₂-R¹³, 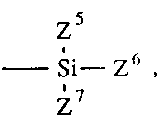 Allyl oder 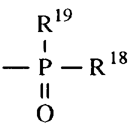 hat oder eine Gruppe der Formel 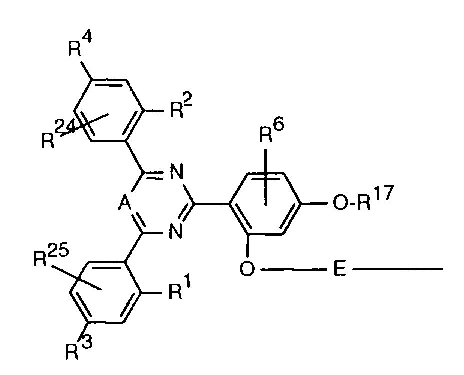 darstellt, worin E C₃-C₁₈-Alkylen oder C₄-C₁₈-Alkenylen ist;
R⁸ C₁-C₁₈-Alkyl; C₃-C₁₈-Alkenyl; durch O, N oder S unterbrochenes und/oder durch OR⁷ substituiertes C₃-C₂₀-Alkyl; Glycidyl; oder eine Gruppe der Formel 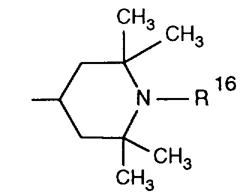 darstellt;
R⁹ und R¹⁰, unabhängig voneinander, C₁-C₁₂-Alkyl bedeuten oder zusammen C₄-C₉-Alkylen oder -Oxaalkylen oder -Azaalkylen bedeuten;
R¹¹ C₁-C₁₂-Alkyl; Trihalogenmethyl; C₁-C₁₂-Alkoxy; C₂-C₄-Alkenyl; Phenyl; Tolyl oder Xylyl bedeutet;
R¹ C₁-C₁₈-Alkyl; C₂-C₁₈-Alkenyl; Phenyl; -R¹⁵-O-CO-C(CH₃)=CH₂; oder -R¹⁵-O-CO-CH=CH₂ bedeutet; oder eine Gruppe der Formel 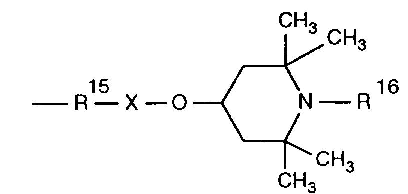 darstellt;
R¹³ Phenyl oder C₇-C₁₈-Alkylphenyl bedeutet; und
R¹⁵ C₂-C₁₂-Alkylen;
R¹⁶ Wasserstoff; Oxyl; C₂-C₈-Alkanoyl; C₁-C₁₂-Alkyl; C₁-C₁₈-Alkoxy; Cyclohexyl;
C₅-C₁₂-Cycloalkoxy; C₇-C₁₁-Phenylalkyl; oder C₇-C₁₁-Phenylalkoxy darstellt;
R¹⁷ C₃-C₁₈-Alkenyl bedeutet; oder R¹⁷ C₁-C₁₂-Alkyl bedeutet, das substituiert ist durch OR⁷, C₁-C₁₈-Alkoxy, -COOR⁸, -CONHR⁹, -CON(R⁹)(R¹⁰), -CCOR¹¹, und/oder eine Gruppe der Formel 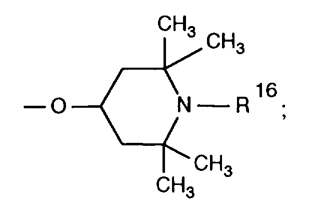 oder R¹⁷ durch 1 bis 6 O unterbrochenes C₄-C₂₀-Alkyl, welches durch OR⁷ substituiert sein kann; oder Glycidyl; C₅-C₁₂-Cycloalkyl; oder C₇-C₁₁-Phenylalkyl bedeutet;
R¹⁸ und R¹⁹, unabhängig voneinander, C₁-C₄-Alkoxy, Methyl oder Phenyl darstellen;
R⁰ C₂-C₁₀-Alkylen; Phenylen; oder eine Gruppe -Phenylen-D-Phenylen- ist, worin D für -O-, -SO₂- oder -C(CH₃)₂- steht;
R¹ C₂-C₁₀-Alkylen; Phenylen; oder C₂-C₆-Alkenylen;
R C₂-C₁₀-Alkylen; oder 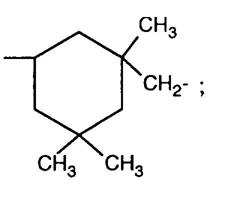
R³ C₂-C₁₀-Alkylen ist; und
R⁴ und R⁵, unabhängig voneinander, H oder Methyl sind.

4. Zusammensetzung gemäß Anspruch 3, worin in der Verbindung der Formel I
A für ein Stickstoffatom steht;
R⁶ in o-Position zu R⁵ und in p-Position zu OR⁷ steht; und
R⁷ C₂-C₈-Alkanoyl, Benzoyl, Phenylsulfonyl, -CO-CF₃, Allyl, 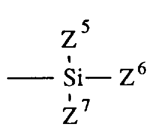 oder C₇-C₉-Alkylphenylsulfonyl bedeutet oder eine Gruppe der Formel 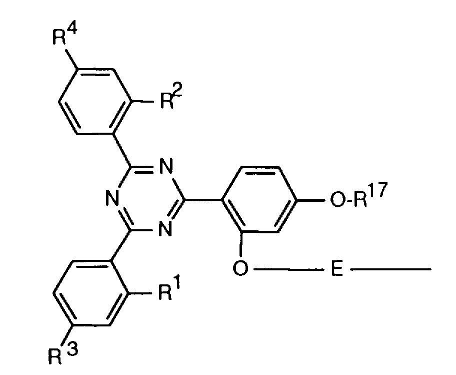 darstellt, worin E C₃-C₆-Alkylen oder C₄-C₆-Alkenylen ist; und
Z⁵, Z⁶ und Z⁷, unabhängig voneinander, C₁-C₄-Alkyl bedeuten.

5. Zusammensetzung gemäß Anspruch 3, worin in der Verbindung der Formel I
R¹ und R, unabhängig voneinander, H; C₁-C₄-Alkyl oder OR⁷ bedeuten;
R³ und R⁴, unabhängig voneinander, H; C₁-C₄-Alkyl; C₁-C₄-Alkoxy, durch O unterbrochenes und durch OR⁷ substituiertes C₃-C₁₂-Alkoxy, Cl oder OR⁷ bedeuten;
R⁵ im Fall n = 1 -O-CO-R¹ oder -O-R¹⁷ bedeutet und im Fall, daß R¹ und R nicht OR⁷ sind, zusätzlich H umfaßt; und R⁵ im Fall n = 2 -O-G-O- bedeutet, wobei G
C₂-C₁₆-Alkylen ist;
R⁶ H oder C₁-C₁₀-Alkyl ist;
R⁸ C₁-C₈-Alkyl darstellt;
R¹¹ C₁-C₃-Alkyl; Trifluormethyl; Trichlormethyl; C₁-C₄-Alkoxy; C₂-C₃-Alkenyl; Phenyl; Tolyl oder Xylyl bedeutet;
R¹ C₁-C₈-Alkyl; oder Phenyl bedeutet;
R¹³ Phenyl oder C₇-C₉-Alkylphenyl bedeutet;
R¹⁶ Wasserstoff; Oxyl; C₁-C₁₂-Alkyl; C₁-C₁₈-Alkoxy; Cyclohexyloxy; oder
C₇-C₁₁-Phenylalkyl darstellt;
R¹⁷ C₁-C₁₈-Alkyl; oder Allyl bedeutet; oder R¹⁷ C₁-C₁₂-Alkyl bedeutet, das substituiert ist durch OR⁷, C₁-C₁₈-Alkoxy, -COOR⁸ und/oder -OCOR¹¹.

6. Zusammensetzung gemäß Anspruch 1, enthaltend auf 100 Gew.-Teile der Komponente A 0,01 bis 15 Gew.-Teile der Komponente B.

7. Zusammensetzung gemäß Anspruch 1, enthaltend außer den Komponenten A und B einen oder mehrere andere Stabilisatoren oder sonstige Zusätze.

8. Zusammensetzung gemäß Anspruch 7, enthaltend neben den Komponenten A und B als Komponente C ein Lichtschutzmittel vom Typ der sterisch gehinderten Amine, der 2-(2-Hydroxyphenyl)-1,3,5-triazine und/oder der 2-Hydroxyphenyl-2H-benztriazole.

9. Zusammensetzung gemäß Anspruch 1, enthaltend als Komponente A ein synthetisches organisches Polymer.

10. Zusammensetzung gemäß Anspruch 1, enthaltend als Komponente A ein thermoplastisches Polymer, ein Bindemittel für Überzüge oder ein photographisches Material.

11. Verfahren zum Stabilisieren von organischem Material gegen Schädigung durch Licht, Sauerstoff und/oder Hitze, dadurch gekennzeichnet, daß man diesem als Stabilisator eine Verbindung der Formel I gemäß Anspruch 1 zusetzt.

12. Verwendung von Verbindungen der Formel I gemäß Anspruch 1 zum Stabilisieren von organischem Material gegen Schädigung durch Licht, Sauerstoff und/oder Hitze.

13. Verbindung der Formel Ia 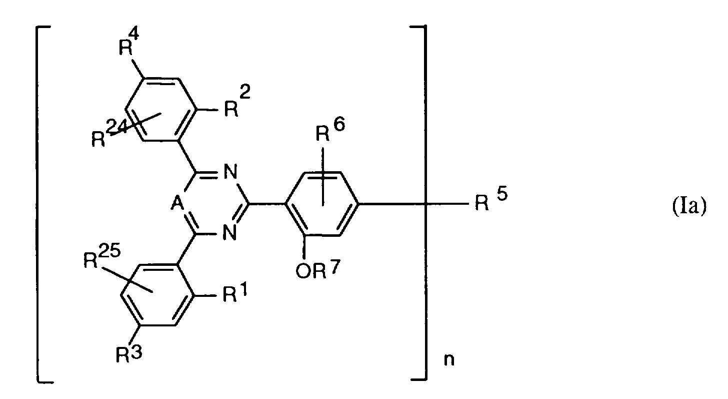 worin n 1 oder 2 ist;
A für CH oder ein Stickstoffatom steht;
R¹ und R, unabhängig voneinander, H; C₁-C₁₂-Alkyl; C₅-C₁₂-Cycloalkyl, Trifluormethyl oder OR⁷ bedeuten;
R³ und R⁴, unabhängig voneinander, H; C₁-C₁₂-Alkyl; C₅-C₁₂-Cycloalkyl, OR¹⁷, Halogen oder OR⁷ bedeuten;
R⁵ im Fall n = 1 als monovalenter Rest C₆-C₁₈-Alkyl; C₁-C₁₈-Alkoxy; Halogen; -O-SO₂-R¹³; 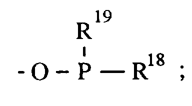 oder 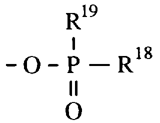 bedeutet; oder R⁵ im Fall n = 1 eine der für R¹⁷ angegebenen Bedeutungen hat oder -O-R¹⁷ bedeutet; und R⁵ als monovalenter Rest im Fall, daß R¹ und R nicht OR⁷ sind, zusätzlich H und C₁-C₅-Alkyl umfaßt;
R⁵ im Fall n = 2 als divalenter Rest -O-G-O- bedeutet, wobei
G C₂-C₁₆-Alkylen; C₄-C₁₂-Alkenylene; Xylylen; oder C₃-C₂₀-Alkylen, das durch -O- unterbrochen und/oder durch OR⁷ substituiert ist, bedeutet; oder G für eine der Gruppen -CH₂CH(OR⁷)CH₂O-R⁰-OCH₂CH(OR⁷)CH₂-; -CO-R¹-CO-; -CO-NH-R-NH-CO-;
-(CH₂)ₘ-COO-R³-OOC-(CH₂)ₘ-, worin m eine ganze Zahl aus dem Bereich 1 bis 3 ist; oder 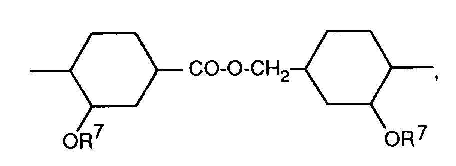 steht;
R⁶ H; C₂-C₁₈-Alkenyl; -X-Z³; unsubstituiertes oder am Phenylkern durch Methyl, Halogen, -CN oder Methoxy substituiertes Benzoyl; Halogen; -SR¹⁴; -SOR¹³; -SO₂R¹³; -C(Z³)=N-Z³; -CH(Z³)-NH-Z³; ein Rest der Formel 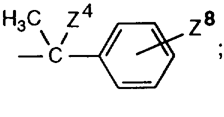 oder ein Rest der Formel 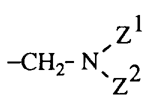 ist;
R⁷ eine der Bedeutungen -CO-R¹¹, -SO₂-R¹³, 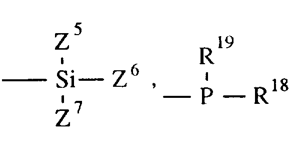 oder 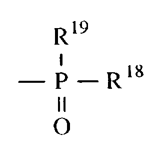 hat oder Allyl oder eine Gruppe der Formel 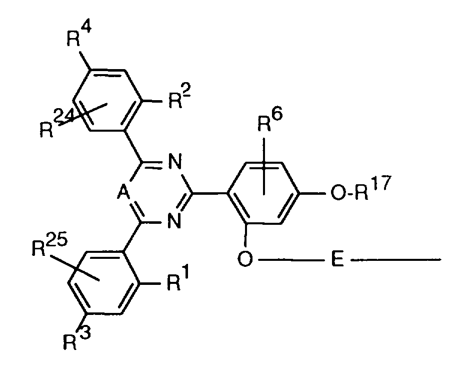 darstellt, worin E C₃-C₁₈-Alkylen oder C₄-C₁₈-Alkenylen ist;
R⁸ C₁-C₁₈-Alkyl; C₃-C₁₈-Alkenyl; durch O, N oder S unterbrochenes und/oder durch OR⁷ substituiertes C₃-C₂₀-Alkyl; durch -P(O)(OR¹⁴)₂, -N(R⁹)(R¹⁰) oder -OCOR¹¹ und/oder OR⁷ substituiertes C₁-C₄-Alkyl; Glycidyl; C₅-C₁₂-Cycloalkyl oder C₇-C₁₁-Phenylalkyl bedeutet; oder eine Gruppe der Formel 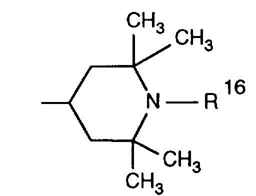 darstellt;
R⁹ und R¹⁰, unabhängig voneinander, C₁-C₁₂-Alkyl; C₃-C₁₂-Alkoxyalkyl;
C₄-C₁₆-Dialkylaminoalkyl oder C₅-C₁₂-Cycloalkyl bedeuten oder
R⁹ und R¹⁰ zusammen C₃-C₉-Alkylen oder -Oxaalkylen oder -Azaalkylen bedeuten;
R¹¹ C₁-C₁₈-Alkyl; C₅-C₁₂-Cycloalkyl; durch Halogen substituiertes C₁-C₁₈-Alkyl;
C₂-C₁₈-Alkenyl; -CH₂-CO-CH₃; C₇-C₁₂-Aralkyl; C₁-C₁₂-Alkoxy; oder Phenyl bedeutet, welches unsubstituiert oder durch C₁-C₁₂-Alkyl, C₁-C₄-Alkoxy, Halogen und/oder Benzyl substituiert ist;
R¹³ C₁-C₁₂-Alkyl; C₆-C₁₀-Aryl; oder C₇-C₁₈-Alkylaryl bedeutet; und
R¹⁴ C₁-C₁₂-Alkyl oder Phenyl oder C₇-C₁₅-Phenylalkyl;
R¹⁵ C₁-C₁₈-Alkylen oder C₂-C₁₈-Alkenylen;
R¹⁶ Wasserstoff; Oxyl; Formyl; C₂-C₈-Alkanoyl; C₁-C₁₈-Alkyl; C₁-C₁₈-Alkoxy;
C₅-C₁₂-Cycloalkyl; C₅-C₁₂-Cycloalkoxy; C₇-C₁₁-Phenylalkyl; C₇-C₁₁-Phenylalkyl, welches am Phenylring durch 1 bis 3 Reste C₁-C₄-Alkyl oder C₁-C₈-Alkanoyl substituiert ist; oder C₇-C₁₁-Phenylalkoxy;
R¹⁷ C₅-C₁₈-Alkyloxycarbonyl; oder C₂-C₁₈-Alkenyl bedeutet; oder R¹⁷ C₁-C₁₈-Alkyl bedeutet, das substituiert ist durch OR⁷, C₁-C₁₈-Alkoxy, C₅-C₁₂-Cycloalkoxy,
C₂-C₁₈-Alkanoyl, C₂-C₈-Alkenyloxy, Halogen, -COOR⁸, -CONH₂, -CONHR⁹, -CON(R⁹)(R¹⁰), -NHR⁹, -N(R⁹)(R¹⁰), -NHCOR¹¹, -CN, -OCOR¹¹, eine Gruppe der Formel 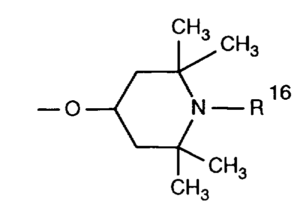 und/oder Phenoxy, welches unsubstituiert ist oder durch C₁-C₁₈-Alkyl, C₁-C₁₈-Alkoxy oder Halogen substituiert ist; oder R¹⁷ C₃-C₅₀-Alkyl bedeutet, welches durch O unterbrochen ist und durch OR⁷ substituiert sein kann; oder R¹⁷ Glycidyl;
C₅-C₁₂-Cycloalkyl; durch OR⁷, C₁-C₄-Alkyl oder -OCOR¹¹ substituiertes
C₅-C₁₂-Cycloalkyl; oder unsubstituiertes oder durch OR⁷, Cl oder CH₃ substituiertes
C₇-C₁₁-Phenylalkyl bedeutet;
R¹⁸ und R¹⁹, unabhängig voneinander, C₁-C₁₂-Alkoxy, Phenoxy, C₁-C₁₂-Alkyl,
C₅-C₁₂-Cycloalkyl, Benzyl, Tolyl oder Phenyl darstellen;
R⁰ C₂-C₁₀-Alkylen; durch -O- unterbrochenes C₄-C₅₀-Alkylen; Phenylen; oder eine Gruppe -Phenylen-D-Phenylen- ist, worin D für -O-, -S-, -SO₂-, -CH₂- oder -C(CH₃)₂- steht;
R¹ C₂-C₁₀-Alkylen; durch O oder S unterbrochenes C₂-C₁₀-Alkylen; C₆-C₁₂-Arylen; oder C₂-C₆-Alkenylen;
R C₂-C₁₀-Alkylen; Phenylen; Tolylen; Diphenylenmethan; oder 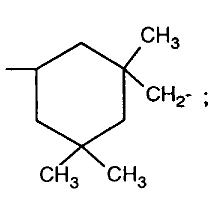 und
R³ C₂-C₁₀-Alkylen oder durch -O- unterbrochenes C₄-C₂₀-Alkylen ist;
R⁴ und R⁵, unabhängig voneinander, H, C₁-C₁₂-Alkyl; C₂-C₆-Alkenyl;
C₁-C₁₂-Alkoxy; C₃-C₁₈-Alkenyloxy; Halogen; Trifluormethyl; C₇-C₁₁-Phenylalkyl;
Phenyl; durch C₁-C₁₈-Alkyl, C₁-C₁₈-Alkoxy oder Halogen substituiertes Phenyl;
Phenyloxy; oder durch C₁-C₁₈-Alkyl, C₁-C₁₈-Alkoxy oder Halogen substituiertes Phenyloxy darstellen;
X eine direkte Bindung oder -CO- bedeutet;
Z¹ und Z, unabhängig voneinander, C₁-C₁₂-Alkyl sind oder gemeinsam C₄-C₁₀-Alkylen darstellen, das durch ein Sauerstoffatom unterbrochen sein kann;
Z³ C₁-C₂₀-Alkyl oder C₇-C₁₅-Phenylalkyl und
Z⁴ und Z⁸, unabhängig voneinander, Wasserstoff oder Methyl sind; und
Z⁵, Z⁶ und Z⁷, unabhängig voneinander, C₁-C₁₈-Alkyl, Cyclohexyl, Phenyl oder
C₁-C₁₈-Alkoxy bedeuten.

14. Verbindung der Formel Ia gemäß Anspruch 13, worin
R¹ und R, unabhängig voneinander, H; C₁-C₁₂-Alkyl; Cyclohexyl, Trifluormethyl oder OR⁷ bedeuten;
R³ und R⁴, unabhängig voneinander, H; C₁-C₁₂-Alkyl; Cyclohexyl, C₁-C₁₈-Alkoxy, durch O unterbrochenes und/oder durch OR⁷ substituiertes C₃-C₁₈-Alkoxy, Halogen oder OR⁷ bedeuten;
R⁵ im Fall n = 1 als monovalenter Rest eine der für R¹⁷ angegebenen Bedeutungen hat oder C₆-C₁₈-Alkyl; C₁-C₁₈-Alkoxy; Halogen; -O-SO₂-R¹³ oder -O-R¹⁷ bedeutet; und im Fall, daß R¹ und R nicht OR⁷ sind, zusätzlich H und C₁-C₅-Alkyl umfaßt;
R⁵ im Fall n = 2 als divalenter Rest -O-G-O- bedeutet, wobei
G C₂-C₁₆-Alkylen; C₄-C₁₂-Alkenylene; Xylylen; C₃-C₂₀-Alkylen, das durch -O- unterbrochen ist, bedeutet; oder G für eine der Gruppen
-CH₂CH(OR⁷)CH₂O-R⁰-OCH₂CH(OR⁷)CH₂-; -CO-R¹-CO-; -CO-NH-R-NH-CO-;
-(CH₂)ₘ-COO-R³-OOC-(CH₂)ₘ-, worin m eine ganze Zahl aus dem Bereich 1 bis 3 ist; oder 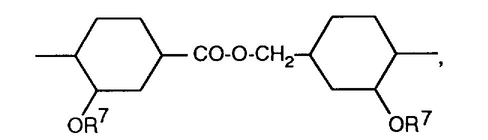 steht;
R⁶ in o-Position zu R⁵ und in p-Position zu OR⁷ steht;
R⁹ und R¹⁰, unabhängig voneinander, C₁-C₁₂-Alkyl; C₃-C₁₂-Alkuxyalkyl;
C₄-C₁₆-Dialkylaminoalkyl oder Cyclohexyl bedeuten oder
R⁹ und R¹⁰ zusammen C₃-C₉-Alkylen oder -Oxaalkylen oder -Azaalkylen bedeuten;
R¹¹ C₁-C₁₈-Alkyl; Trihalogenmethyl; C₂-C₁₈-Alkenyl; -CH₂-CO-CH₃; C₇-C₁₂-Aralkyl;
C₁-C₁₂-Alkoxy; oder Phenyl bedeutet, welches unsubstituiert oder durch C₁-C₁₂-Alkyl,
C₁-C₄-Alkoxy, Halogen und/oder Benzyl substituiert ist;
R¹⁷ C₅-C₁₈-Alkyloxycarbonyl; oder C₂-C₁₈-Alkenyl bedeutet; oder R¹⁷ C₁-C₁₈-Alkyl bedeutet, das substituiert ist durch OR⁷, C₁-C₁₈-Alkoxy, C₂-C₁₈-Alkanoyl, C₂-C₈-Alkenyloxy, Halogen, -COOR⁸, -CONH₂, -CONHR⁹, -CON(R⁹)(R¹⁰), -NHR⁹, -N(R⁹)(R¹⁰), -NHCOR¹¹, -CN, -OCOR¹¹, eine Gruppe der Formel 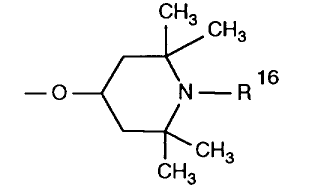 und/oder Phenoxy, welches unsubstituiert ist oder durch C₁-C₁₈-Alkyl, C₁-C₁₈-Alkoxy, oder Halogen substituiert ist; oder R¹⁷ C₄-C₂₀-Alkyl darstellt, welches durch O unterbrochen ist und durch OR⁷ substituiert sein kann; oder R¹⁷ Glycidyl; Cyclohexyl; durch OR⁷, C₁-C₄-Alkyl oder -OCOR¹¹ substituiertes Cyclohexyl; oder unsubstituiertes oder durch Cl oder CH₃ substituiertes C₇-C₁₁-Phenylalkyl bedeutet; und
R¹⁸ und R¹⁹, unabhängig voneinander, C₁-C₆-Alkoxy, C₁-C₄-Alkyl, Cyclohexyl, Tolyl oder Phenyl darstellen;
R⁴ und R⁵, unabhängig voneinander, H oder C₁-C₁₂-Alkyl sind; und
Z⁵, Z⁶ und Z⁷, unabhängig voneinander, C₁-C₄-Alkyl, oder C₁-C₄-Alkoxy bedeuten.

15. Verbindung der Formel Ia gemäß Anspruch 13, worin
R¹ und R, unabhängig voneinander, H; C₁-C₄-Alkyl oder OR⁷ bedeuten;
R³ und R⁴, unabhängig voneinander, H; C₁-C₄-Alkyl; C₁-C₈-Alkoxy, durch O unterbrochenes und/oder durch OR⁷ substituiertes C₃-C₁₂-Alkoxy, Halogen oder OR⁷ bedeuten;
R⁵ im Fall n = 1 als monovalenter Rest eine der für R¹⁷ angegebenen Bedeutungen hat oder Halogen; C₆-C₁₈-Alkyl; C₁-C₁₈-Alkoxy; -O-SO₂-R¹³ oder -O-R¹⁷ bedeutet; und im Fall, daß R¹ und R nicht OR⁷ sind, zusätzlich H und C₁-C₅-Alkyl umfaßt;
R⁵ im Fall n = 2 als divalenter Rest -O-G-O- bedeutet, wobei
G C₂-C₁₆-Alkylen; C₄-C₁₂-Alkenylene; oder Xylylen bedeutet; oder G für eine der Gruppen -CH₂CH(OR⁷)CH₂O-R⁰-OCH₂CH(OR⁷)CH₂-; -CO-R¹-CO-;
-CO-NH-R-NH-CO-; -(CH₂)ₘ-COO-R₂₃-OOC-(CH₂)ₘ- steht, worin m eine ganze Zahl aus dem Bereich 1 bis 3 ist;
R⁶ H; Allyl; C₁-C₁₀-Alkyl; Acetyl oder Benzoyl ist;
R⁷ eine der Bedeutungen -CO-R¹¹, -SO₂-R¹³, 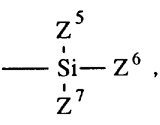 Allyl oder 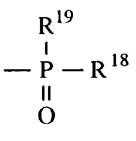 hat oder eine Gruppe der Formel 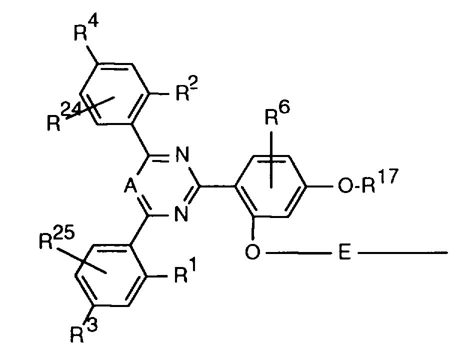 darstellt, worin E C₃-C₁₈-Alkylen oder C₄-C₁₈-Alkenylen ist;
R⁸ C₁-C₁₈-Alkyl; C₃-C₁₈-Alkenyl; durch O, N oder S unterbrochenes und/oder durch OR⁷ substituiertes C₃-C₂₀-Alkyl; Glycidyl; oder eine Gruppe der Formel 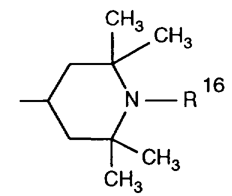 darstellt;
R⁹ und R¹⁰, unabhängig voneinander, C₁-C₁₂-Alkyl bedeuten oder zusammen C₄-C₉-Alkylen oder -Oxaalkylen oder -Azaalkylen bedeuten;
R¹¹ C₁-C₁₂-Alkyl; Trihalogenmethyl; C₂-C₄-Alkenyl; C₁-C₁₂-Alkoxy; Phenyl; Tolyl oder Xylyl bedeutet;
R¹³ Phenyl oder C₇-C₁₈-Alkylphenyl bedeutet; und
R¹⁵ C₂-C₁₂-Alkylen;
R¹⁶ Wasserstoff; Oxyl; C₂-C₈-Alkanoyl; C₁-C₁₂-Alkyl; C₁-C₁₈-Alkoxy; Cyclohexyl;
C₅-C₁₂-Cycloalkoxy; C₇-C₁₁-Phenylalkyl; oder C₇-C₁₁-Phenylalkoxy darstellt;
R¹⁷ C₃-C₁₈-Alkenyl bedeutet; oder R¹⁷ C₁-C₁₂-Alkyl bedeutet, das substituiert ist durch OR⁷, C₁-C₁₈-Alkoxy, -COOR⁸, -CONHR⁹, -CON(R⁹)(R¹⁰), -OCOR¹¹, und/oder eine Gruppe der Formel 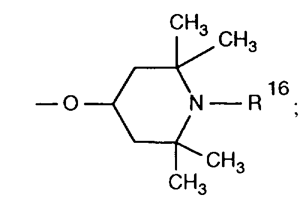 oder R¹⁷ durch 1 bis 6 O unterbrochenes C₄-C₂₀-Alkyl, welches durch OR⁷ substituiert sein kann; oder Glycidyl; C₅-C₁₂-Cycloalkyl; oder C₇-C₁₁-Phenylalkyl bedeutet;
R¹⁸ und R¹⁹, unabhängig voneinander, C₁-C₄-Alkoxy, Methyl oder Phenyl darstellen;
R⁰ C₂-C₁₀-Alkylen; Phenylen; oder eine Gruppe -Phenylen-D-Phenylen- ist, worin D für -O-, -SO₂- oder -C(CH₃)₂- steht;
R¹ C₂-C₁₀-Alkylen; Phenylen; oder C₂-C₆-Alkenylen;
R C₂-C₁₀-Alkylen; oder 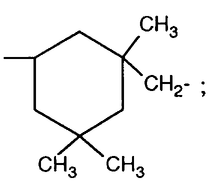
R³ C₂-C₁₀-Alkylen ist; und
R⁴ und R⁵, unabhängig voneinander, H oder Methyl sind.

16. Verbindung der Formel Ia gemäß Anspruch 14, worin
A für ein Stickstoffatom steht;
R⁶ in o-Position zu R⁵ und in p-Position zu OR⁷ steht; und
R⁷ C₂-C₈-Alkanoyl, Benzoyl, Phenylsulfonyl, Allyl, -CO-CF₃, 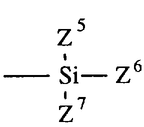 oder C₇-C₉-Alkylphenylsulfonyl bedeutet oder eine Gruppe der Formel 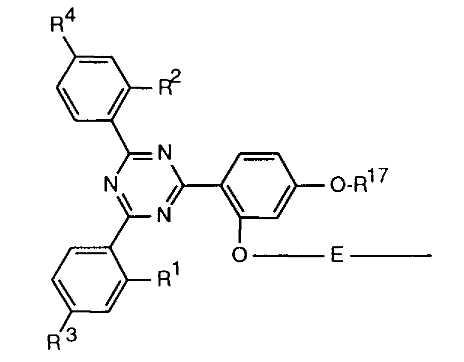 darstellt, worin E C₃-C₆-Alkylen oder C₄-C₆-Alkenylen ist; und
Z⁵, Z⁶ und Z⁷, unabhängig voneinander, C₁-C₄-Alkyl bedeuten.

17. Verbindung der Formel Ia gemäß Anspruch 14, worin
R¹ und R, unabhängig voneinander, H; C₁-C₄-Alkyl oder OR⁷ bedeuten;
R³ und R⁴, unabhängig voneinander, H; C₁-C₄-Alkyl; C₁-C₄-Alkoxy, durch O unterbrochenes und durch OR⁷ substituiertes C₃-C₁₂-Alkoxy, Cl oder OR⁷ bedeuten;
R⁵ im Fall n = 1 -O-R¹⁷ bedeutet und im Fall, daß R¹ und R nicht OR⁷ sind, zusätzlich H umfaßt; und im Fall n = 2 -O-G-O- bedeutet, wobei G C₂-C₁₆-Alkylen ist;
R⁶ H oder C₁-C₁₀-Alkyl ist;
R⁸ C₁-C₈-Alkyl darstellt;
R¹¹ C₁-C₃-Alkyl; Trifluormethyl; Trichlormethyl; C₁-C₄-Alkoxy; C₂-C₃-Alkenyl; Phenyl; Tolyl oder Xylyl bedeutet;
R¹³ Phenyl oder C₇-C₉-Alkylphenyl bedeutet;
R¹⁶ Wasserstoff; Oxyl; C₁-C₁₂-Alkyl; C₁-C₁₈-Alkoxy; Cyclohexyloxy; oder
C₇-C₁₁-Phenylalkyl darstellt;
R¹⁷ C₁-C₁₈-Alkyl; oder Allyl bedeutet; oder R¹⁷ C₁-C₁₂-Alkyl bedeutet, das substituiert ist durch OR⁷, C₁-C₁₈-Alkoxy, -COOR⁸ und/oder -OCOR¹¹; und
R⁴ und R⁵, unabhängig voneinander, H oder Methyl sind.
